# EUROPEAN PATENT APPLICATION

(11) **EP 1 285 963 A1**
(43) Date of publication of application: **26.02.2003**
(21) Application number: 02017626.9
(22) Date of filing: 06.08.2002
(51) Int. Cl.: C12N 9/14, A61K 38/00, G01N 33/68, C12N 15/10, A01K 67/027

(54) **Human ZZAP1 protein**

(30) Priority: 09.08.2001 US 311480 P
(71) Applicant: Aeomica, Inc., Sunnyvale, CA 94085 (US)
(72) Inventor: Shannon, Mark, Sunnyvale, CA 94085 (US)
(74) Representative: Rollins, Anthony John

(57) **Abstract**

The invention provides isolated nucleic acids that encode human ZZAP1, and fragments thereof, vectors for propagating and expressing human ZZAP1 nucleic acids, host cells comprising the nucleic acids and vectors of the present invention, proteins, protein fragments, and protein fusions of the novel human ZZAP1 isoforms, and antibodies thereto. The invention further provides transgenic cells and non-human organisms comprising human ZZAP1 nucleic acids, and transgenic cells and non-human organisms with targeted disruption of the endogenous orthologue of the human ZZAP1 gene. The invention further provides pharmaceutical formulations of the nucleic acids, proteins, and antibodies of the present invention, and diagnostic, investigational, and therapeutic methods based on the human ZZAP1 nucleic acids, proteins, and antibodies of the present invention.

<110> Shannon, Mark

<120> HUMAN ZZAP1 PROTEIN

<130> PB0152

<150> US 60/311,480
<151> 2001-08-09

<160> 152

<170> Aeomica Sequence Listing Engine

<210> 1
<211> 1849
<212> DNA
<213> Homo sapiens

<400> 1
gcttcacaat gctgtagcag aaatggaaca ggaaaagttt gatcttcaaa agcaacacac
60
tgaaaacatt caagaattgc ttgaggatac aaatgtgcgt ctgaataaaa tggagagtga
120
atatatggcg caaacacagt ccacaaacca catgatcaaa gaactggagg cccgtgtcca
180
gcagctgact ggtgaagcag agaacagtaa tttacagagg cagaaattaa ttcaagaaaa
240
agcagaactt gaaagatgtt accagataac gtgtagtgaa ttacaagaag taaaggcaag
300
gcgtaacaca ctgcataaag agaaggacca tcttgtaaat gattatgagc aaaacatgaa
360
actgttacaa accaaatatg atgctgatat aaaccttcta aaacaagaac atgctctttc
420
agcttctaag gcatctagta tgattgaaga attagagcag aatgtctgtc aattaaaaca
480
gcagttacag gaatcagaac ttcaaagaaa gcaacaacta agggatcaag aaaataagtt
540
tcagatggag aaaagtcatc taaaacacat ctatgaaaaa aaggctcatg acttgcagag
600
tgaacttgat aaaggaaagg aagatactca aaagaaaatt cataaatttg aggaagcttt
660
gaaggaaaaa gaggagcagc taactcgtgt gactgaagtt cagaggttgc aggcccagca
720
ggcagatgcc gctctggaag agtttaagcg gcaggtggaa ctgaactcag agaaagtcta
780
tgctgaaatg aaagagcaga tggaaaaagt ggaggcagat ctaactagat ccaaatctct
840
tcgtgagaaa caatcaaagg agtttttatg gcaactggag gacatcagac agcggtatga
900
acaacagata gtagagctga agctggagca tgaacaggag aagacgcacc tattacagca
960
gcataacgca gagaaggata gcctagtccg agaccatgaa cgggaaattg aaaacctgga

1020
aaagcagctt cgcgctgcca atatggagca cgaaaatcaa attcaggagt tcaagaaacg
1080
agatgcacag gttattgccg acatggaggc ccgggttcac aagttgagag aagaattgat
1140
caatgtgaac tcacagcgga aacagcagct ggtagagctt ggtcttcttc gtgaagagga
1200
aaagcaaagg gctacaaggg aacatgagat tgtcgtcaat aaactgaagg ctgaatcaga
1260
aaagatgaaa atagagctga aaaagactca tgctgctgag acagagatga cactggaaaa
1320
ggccaacagc aagctgaagc agattgagaa ggaatacact cagaagcttg ccaaatcttc
1380
acagatcata gcagaacttc agacaaccat ttcctctctg aaagaagaga acagccagca
1440
gcagcttgct gcagaaaggc ggctccagga tgttagacaa aagtttgaag atgagaagaa
1500
gcagctgatt agagataatg accaagcaat caaggtttta caagatgaat tagaaaaccg
1560
ttctaatcag gtgcgatgtg cagagaaaaa attacaacac aaagaattgg agtcacagga
1620
acagataact tacatacgac aagaatatga aacaaaattg aaaggattga tgccagcatc
1680
cctaagacaa gaacttgaag acaccatttc ctccctaaaa tcacaggtta atttctgca
1740
aaagagagct tccatccttc aggaagaact gactacatat caaggcagaa ggtaactgca
1800
cgagagaatg caacggatgc aatttccagg ctgtgctgtg gacttcttc
1849

<210> 2
<211> 1773
<212> DNA
<213> Homo sapiens

<400> 2
   atggaacagg aaaagtttga tcttcaaaag caacacactg aaaacattca agaattgctt
   60
   gaggatacaa atgtgcgtct gaataaaatg gagagtgaat atatggcgca aacacagtcc
   120
   acaaaccaca tgatcaaaga actggaggcc cgtgtccagc agctgactgg tgaagcagag
   180
   aacagtaatt tacagaggca gaaattaatt caagaaaaag cagaacttga aagatgttac
   240
   cagataacgt gtagtgaatt acaagaagta aaggcaaggc gtaacacact gcataaagag
   300
   aaggaccatc ttgtaaatga ttatgagcaa aacatgaaac tgttacaaac caaatatgat
   360
   gctgatataa accttctaaa acaagaacat gctctttcag cttctaaggc atctagtatg
   420
   attgaagaat tagagcagaa tgtctgtcaa ttaaaacagc agttacagga atcagaactt
   480
   caaagaaagc aacaactaag ggatcaagaa aataagtttc agatggagaa aagtcatcta
    540
   aaacacatct atgaaaaaaa ggctcatgac ttgcagagtg aacttgataa aggaaaggaa
    600
   gatactcaaa agaaaattca taaatttgag gaagctttga aggaaaaaga ggagcagcta
    660
   actcgtgtga ctgaagttca gaggttgcag gcccagcagg cagatgccgc tctggaagag
    720
   tttaagcggc aggtggaact gaactcagag aaagtctatg ctgaaatgaa agagcagatg
    780
   gaaaaagtgg aggcagatct aactagatcc aaatctcttc gtgagaaaca atcaaaggag
    840
   tttttatggc aactggagga catcagacag cggtatgaac aacagatagt agagctgaag
    900
   ctggagcatg aacaggagaa gacgcaccta ttacagcagc ataacgcaga gaaggatagc
    960
   ctagtccgag accatgaacg ggaaattgaa aacctggaaa agcagcttcg cgctgccaat
    1020
   atggagcacg aaaatcaaat tcaggagttc aagaaacgag atgcacaggt tattgccgac
    1080
   atggaggccc gggttcacaa gttgagagaa gaattgatca atgtgaactc acagcggaaa
    1140
   cagcagctgg tagagcttgg tcttcttcgt gaagaggaaa agcaaagggc tacaagggaa
    1200
   catgagattg tcgtcaataa actgaaggct gaatcagaaa agatgaaaat agagctgaaa
    1260
   aagactcatg ctgctgagac agagatgaca ctggaaaagg ccaacagcaa gctgaagcag
    1320
   attgagaagg aatacactca gaagcttgcc aaatcttcac agatcatagc agaacttcag
    1380
   acaaccattt cctctctgaa agaagagaac agccagcagc agcttgctgc agaaaggcgg
    1440
   ctccaggatg ttagacaaaa gtttgaagat gagaagaagc agctgattag agataatgac
    1500
   caagcaatca aggttttaca agatgaatta gaaaaccgtt ctaatcaggt gcgatgtgca
    1560
   gagaaaaaat tacaacacaa agaattggag tcacaggaac agataactta catacgacaa
    1620
   gaatatgaaa caaaattgaa aggattgatg ccagcatccc taagacaaga acttgaagac
    1680
   accatttcct ccctaaaatc acaggttaat tttctgcaaa agagagcttc catccttcag
    1740
   gaagaactga ctacatatca aggcagaagg taa
    1773

<210> 3
<211> 590
<212> PRT
<213> Homo sapiens

<400> 3
Met Glu Gln Glu Lys Phe Asp Leu Gln Lys Gln His Thr Glu Asn Ile

1               5                   10                  15

Gln Glu Leu Leu Glu Asp Thr Asn Val Arg Leu Asn Lys Met Glu Ser
            20                  25                  30

Glu Tyr Met Ala Gln Thr Gln Ser Thr Asn His Met Ile Lys Glu Leu
        35                  40                  45

Glu Ala Arg Val Gln Gln Leu Thr Gly Glu Ala Glu Asn Ser Asn Leu
    50                  55                  60

Gln Arg Gln Lys Leu Ile Gln Glu Lys Ala Glu Leu Glu Arg Cys Tyr
65                  70                  75                  80

Gln Ile Thr Cys Ser Glu Leu Gln Glu Val Lys Ala Arg Arg Asn Thr
                85                  90                  95

Leu His Lys Glu Lys Asp His Leu Val Asn Asp Tyr Glu Gln Asn Met
            100                 105                 110

Lys Leu Leu Gln Thr Lys Tyr Asp Ala Asp Ile Asn Leu Leu Lys Gln
        115                 120                 125

Glu His Ala Leu Ser Ala Ser Lys Ala Ser Ser Met Ile Glu Glu Leu
    130                 135                 140

Glu Gln Asn Val Cys Gln Leu Lys Gln Gln Leu Gln Glu Ser Glu Leu
145                 150                 155                 160

Gln Arg Lys Gln Gln Leu Arg Asp Gln Glu Asn Lys Phe Gln Met Glu
                165                 170                 175

Lys Ser His Leu Lys His Ile Tyr Glu Lys Lys Ala His Asp Leu Gln
            180                 185                 190

Ser Glu Leu Asp Lys Gly Lys Glu Asp Thr Gln Lys Lys Ile His Lys
        195                 200                 205

Phe Glu Glu Ala Leu Lys Glu Lys Glu Glu Gln Leu Thr Arg Val Thr
    210                 215                 220

Glu Val Gln Arg Leu Gln Ala Gln Gln Ala Asp Ala Ala Leu Glu Glu
225                 230                 235                 240

Phe Lys Arg Gln Val Glu Leu Asn Ser Glu Lys Val Tyr Ala Glu Met
                245                 250                 255

Lys Glu Gln Met Glu Lys Val Glu Ala Asp Leu Thr Arg Ser Lys Ser
            260                 265                 270

Leu Arg Glu Lys Gln Ser Lys Glu Phe Leu Trp Gln Leu Glu Asp Ile
        275                 280                 285

Arg Gln Arg Tyr Glu Gln Gln Ile Val Glu Leu Lys Leu Glu His Glu
    290                 295                 300

Gln Glu Lys Thr His Leu Leu Gln Gln His Asn Ala Glu Lys Asp Ser
305                 310                 315                 320

Leu Val Arg Asp His Glu Arg Glu Ile Glu Asn Leu Glu Lys Gln Leu
                325                 330                 335

Arg Ala Ala Asn Met Glu His Glu Asn Gln Ile Gln Glu Phe Lys Lys
            340                 345                 350

Arg Asp Ala Gln Val Ile Ala Asp Met Glu Ala Arg Val His Lys Leu
        355                 360                 365

Arg Glu Glu Leu Ile Asn Val Asn Ser Gln Arg Lys Gln Gln Leu Val
    370                 375                 380

Glu Leu Gly Leu Leu Arg Glu Glu Glu Lys Gln Arg Ala Thr Arg Glu
385                 390                 395                 400

His Glu Ile Val Val Asn Lys Leu Lys Ala Glu Ser Glu Lys Met Lys
                405                 410                 415

Ile Glu Leu Lys Lys Thr His Ala Ala Glu Thr Glu Met Thr Leu Glu
            420                 425                 430

Lys Ala Asn Ser Lys Leu Lys Gln Ile Glu Lys Glu Tyr Thr Gln Lys
        435                 440                 445

Leu Ala Lys Ser Ser Gln Ile Ile Ala Glu Leu Gln Thr Thr Ile Ser
    450                 455                 460

Ser Leu Lys Glu Glu Asn Ser Gln Gln Gln Leu Ala Ala Glu Arg Arg
465                 470                 475                 480

Leu Gln Asp Val Arg Gln Lys Phe Glu Asp Glu Lys Lys Gln Leu Ile
                485                 490                 495

Arg Asp Asn Asp Gln Ala Ile Lys Val Leu Gln Asp Glu Leu Glu Asn
            500                 505                 510

Arg Ser Asn Gln Val Arg Cys Ala Glu Lys Lys Leu Gln His Lys Glu
        515                 520                 525

Leu Glu Ser Gln Glu Gln Ile Thr Tyr Ile Arg Gln Glu Tyr Glu Thr
    530                 535                 540

Lys Leu Lys Gly Leu Met Pro Ala Ser Leu Arg Gln Glu Leu Glu Asp
545                 550                 555                 560

Thr Ile Ser Ser Leu Lys Ser Gln Val Asn Phe Leu Gln Lys Arg Ala
                565                 570                 575

Ser Ile Leu Gln Glu Glu Leu Thr Thr Tyr Gln Gly Arg Arg
            580                 585                 590

<210> 4
<211> 28
<212> DNA
<213> Homo sapiens

<400> 4
gtgtagtgaa ttacaagaag taaaggca
 28

<210> 5
<211> 9
<212> PRT
<213> Homo sapiens

<400> 5
Cys Ser Glu Leu Gln Glu Val Lys Ala
1               5

<210> 6
<211> 67
<212> DNA
<213> Homo sapiens

<400> 6
gagcagatgg aaaaagtgga ggcagatcta actagatcca aatctcttcg tgagaaacaa
 60
tcaaagg
 67

<210> 7
<211> 22
<212> PRT
<213> Homo sapiens

<400> 7
Glu Gln Met Glu Lys Val Glu Ala Asp Leu Thr Arg Ser Lys Ser Leu
1               5                   10                  15

Arg Glu Lys Gln Ser Lys
            20

<210> 8
<211> 145
<212> DNA
<213> Homo sapiens

<400> 8
gcttcacaat gctgtagcag aaatggaaca ggaaaagttt gatcttcaaa agcaacacac
 60
tgaaaacatt caagaattgc ttgaggatac aaatgtgcgt ctgaataaaa tggagagtga
 120
atatatggcg caaacacagt ccaca
 145

<210> 9
<211> 155
<212> DNA
<213> Homo sapiens

<400> 9
aaccacatga tcaaagaact ggaggcccgt gtccagcagc tgactggtga agcagagaac
 60
agtaatttac agaggcagaa attaattcaa gaaaaagcag aacttgaaag atgttaccag
 120
ataacgtgta gtgaattaca agaagtaaag gcaag
 155

<210> 10
<211> 130
<212> DNA
<213> Homo sapiens

<400> 10
gcgtaacaca ctgcataaag agaaggacca tcttgtaaat gattatgagc aaaacatgaa
 60
actgttacaa accaaatatg atgctgatat aaaccttcta aaacaagaac atgctctttc
 120
agcttctaag
 130

<210> 11
<211> 93
<212> DNA
<213> Homo sapiens

<400> 11
gcatctagta tgattgaaga attagagcag aatgtctgtc aattaaaaca gcagttacag
 60
gaatcagaac ttcaaagaaa gcaacaacta agg
 93

<210> 12
<211> 60
<212> DNA
<213> Homo sapiens

<400> 12
gatcaagaaa ataagtttca gatggagaaa agtcatctaa aacacatcta tgaaaaaaag

60

<210> 13
<211> 80
<212> DNA
<213> Homo sapiens

<400> 13
gctcatgact tgcagagtga acttgataaa ggaaaggaag atactcaaaa gaaaattcat
 60
aaatttgagg aagctttgaa
 80

<210> 14
<211> 136
<212> DNA
<213> Homo sapiens

<400> 14
ggaaaaagag gagcagctaa ctcgtgtgac tgaagttcag aggttgcagg cccagcaggc
 60
agatgccgct ctggaagagt ttaagcggca ggtggaactg aactcagaga aagtctatgc
 120
tgaaatgaaa gagcag
 136

<210> 15
<211> 108
<212> DNA
<213> Homo sapiens

<400> 15
atggaaaaag tggaggcaga tctaactaga tccaaatctc ttcgtgagaa acaatcaaag
 60
gagtttttat ggcaactgga ggacatcaga cagcggtatg aacaacag
 108

<210> 16
<211> 183
<212> DNA
<213> Homo sapiens

<400> 16
atagtagagc tgaagctgga gcatgaacag gagaagacgc acctattaca gcagcataac
 60
gcagagaagg atagcctagt ccgagaccat gaacgggaaa ttgaaaacct ggaaaagcag
 120
cttcgcgctg ccaatatgga gcacgaaaat caaattcagg agttcaagaa acgagatgca
 180
cag
 183

<210> 17
<211> 231
<212> DNA
<213> Homo sapiens

<400> 17
gttattgccg acatggaggc ccgggttcac aagttgagag aagaattgat caatgtgaac
 60
tcacagcgga aacagcagct ggtagagctt ggtcttcttc gtgaagagga aaagcaaagg
 120
gctacaaggg aacatgagat tgtcgtcaat aaactgaagg ctgaatcaga aaagatgaaa
 180
atagagctga aaaagactca tgctgctgag acagagatga cactggaaaa g
 231

<210> 18
<211> 63
<212> DNA
<213> Homo sapiens

<400> 18
gccaacagca agctgaagca gattgagaag gaatacactc agaagcttgc caaatcttca
 60
cag
 63

<210> 19
<211> 150
<212> DNA
<213> Homo sapiens

<400> 19
atcatagcag aacttcagac aaccatttcc tctctgaaag aagagaacag ccagcagcag
 60
cttgctgcag aaaggcggct ccaggatgtt agacaaaagt ttgaagatga gaagaagcag
 120
ctgattagag ataatgacca agcaatcaag
 150

<210> 20
<211> 90
<212> DNA
<213> Homo sapiens

<400> 20
gttttacaag atgaattaga aaaccgttct aatcaggtgc gatgtgcaga gaaaaaatta
 60
caacacaaag aattggagtc acaggaacag
 90

<210> 21
<211> 102

<212> DNA
<213> Homo sapiens

<400> 21
ataacttaca tacgacaaga atatgaaaca aaattgaaag gattgatgcc agcatcccta
 60
agacaagaac ttgaagacac catttcctcc ctaaaatcac ag
 102

<210> 22
<211> 65
<212> DNA
<213> Homo sapiens

<400> 22
gttaattttc tgcaaaagag agcttccatc cttcaggaag aactgactac atatcaaggc
 60
agaag
 65

<210> 23
<211> 58
<212> DNA
<213> Homo sapiens

<400> 23
gtaactgcac gagagaatgc aacggatgca atttccaggc tgtgctgtgg acttcttc
 58

<210> 24
<211> 500
<212> DNA
<213> Homo sapiens

<400> 24
gatcctgctt tttatatgat atctttacct tgaatgttta ctggtggttt tttgtatctt
 60
ttggtgttca ggtcatcata tatataaata gaggaatata gaaaccattt tttccgctga
 120
attacaaaat agtcaattcc atagtagtaa aagagtcaac tcttgatttc ctgatgtagc
 180
ttcacaatgc tgtagcagaa atggaacagg aaaagtttga tcttcaaaag caacacactg
 240
aaaacattca agaattgctt gaggatacaa atgtgcgtct gaataaaatg gagagtgaat
 300
acatggcgca aacacagtcc acagtgagtc tttaaacctg ttcttgacat cttagaacct
 360
gtcttctcaa tttccatgtc ctcatgtttc cctgatgtgc acagtgtaga aagaagtgtg
 420
ttacagaatc aaaagaaaaa ctttaccttc ctactccata cagatttatg tgtggccatg
 480
gacagaccct tgaagtctgt

500

<210> 25
<211> 500
<212> DNA
<213> Homo sapiens

<400> 25
tttttttaaa gacttggtta agttcaaggt tgatttaatt tctactacca tggtttatga
 60
tacatttgtt ttatttattc ttatagatta tacgtgttcc tcaagcttct tacagataca
 120
aaagtgttac aactgagtca gagactttct taaccatgac ctgttctttt agaaccacat
 180
gatcaaagaa ctggaggccc gtgtccagca gctgactgga gaagcagaga acagtaattt
 240
acagaggcag aaattaattc aagaaaaagc agaacttgaa agatgttacc agataacgtg
 300
tagtgaatta caagaagtaa aggcaaggta ttgtctgaaa tcagatattt atcaggtgta
 360
gctataaagt actgatatta actgtatcag ttatctgcca aaattaatta caccttgtgc
 420
gtttagcctt gggaagtttc taatctaaat actgctgttt gtttgtttgt ttgtttgttt
 480
gtttgtttat ttagagacag
 500

<210> 26
<211> 500
<212> DNA
<213> Homo sapiens

<400> 26
gcatgagtca ctgtacccaa cctgttttta acataagcag aatgtttaga agcagttgac
 60
aaaattgtgg atacttattt aatatattga atggaaatgt atttggattt agctgattaa
 120
aaaatttcat tttagagatt ggttttaaat acattgaaag tctattttat tctcttgttt
 180
ttaggcgtaa cacactgcat aaagagaagg accatcttgt aaatgattat gagcaaaaca
 240
tgaaactgtt acaaaccaaa tatgatgctg atataaacct tctaaaacaa gaacatgctc
 300
tttcagcttc taaggtatta tttatgttaa tagataatcc acatgaagta tttattcact
 360
aagcctagag ccatagatag ccaagtagaa attatttggc atttgaaatt aatctctctt
 420
tcactttatt cttaaattaa caggaacttc aggaattttt actggtattt aaaatgttta
 480
aaattcgaaa tgtctactat
 500

<210> 27
<211> 500
<212> DNA
<213> Homo sapiens

<400> 27
ctatgctgca tttaatttat atttttactt taaaagatta ttttttctag tatcttttaa
 60
aaacattttt cattcaaatt tctagataat tttaattctg attggacttt caggggtaca
 120
aaaggcatat tttttcaatc agaaagtata atatggtaca aaaatcaata ttctgctttt
 180
tattcttctt aaaatccttg taggcatcta gtatgattga agaattagag cagaatgtct
 240
gtcaattaaa acagcagtta caggaatcag aacttcaaag aaagcaacaa ctaagggtga
 300
gtcttgtatt tctacacaga agaacaatgg tcaaacacag ttttcttgat tcagatgtat
 360
cattgagagt caattaaaaa aataaaactc agaaatgcag gtcatctttt ctgattcttt
 420
tatcgcattg tataatttta cattttattt atcttaatag agattgatgc tttgagatgt
 480
agagaataaa gtacaataat
 500

<210> 28
<211> 500
<212> DNA
<213> Homo sapiens

<400> 28
aagtgctttt gagaatagta atatcttcag gtgactaaga ttttatgcct cgtaatacaa
 60
agctactcca atttctacct gtagggctta cttaatcata tgcaaaactc tgaactgaag
 120
tttgacatta agtagattga ttgatttaat taggttgcta attttattga ctctagtata
 180
atttaaagta taataaatat aaacatttat cattcacagg atcaagaaaa taagtttcag
 240
atggagaaaa gtcatttaaa acacatctat gaaaaaaagg taatatgttt tatggaagct
 300
ataaaatatt taaaatagca aatgaggcat taatgcagac tgatttttat gaaaccatac
 360
attctcacct ttaaagaaca acttgtttct ttgtcttttt ttttagacag agtcttgctc
 420
tgtcacccag gctggagtgc agtggcacga tctcggctca ctgtagcttc tgcctcctag
 480
gttcaaggga ttctcatgcc
 500

<210> 29
<211> 500

<212> DNA
<213> Homo sapiens

<400> 29
agactcaaga aattcatttt gaacagaaaa taggtatttg agtatagtat tgaaatcatt
 60
ctatggtaga ccaagtttct atcttgaaat atctaaatct aattttatat taaaaatgga
 120
tttgtgatca ctgtttcatt gtttgtatgt ctctttgaag taaactttac tcgtcttcca
 180
ataaattttc catgttcttg tcatggcagg ctcatgactt gcagagtgaa cttgataaag
 240
gaaaagaaga tactcaaaag aaaattcata aatttgagga agctttgaag tgagtaaaat
 300
tgtaatgcat ttgatttaaa actacccttt attaaactgt gagccaaatt aagtaactgg
 360
gacctaaaca ctatattttt ataagagctt tgctatggat tcgtttgcat cagtttccca
 420
tcacaaatct ctatcttcaa gattttatta ctagccagta atttttttta aaagaaaaat
 480
cagttcagct gtttttaagt
 500

<210> 30
<211> 500
<212> DNA
<213> Homo sapiens

<400> 30
tatacccttt ttctcttgag acaaattagt ttattgtcaa atttctaaag ggaagttttg
 60
taggatttgg atcaaaaaaa ttaaaatctg gattttattt tagagggggt taaatattgt
 120
tctttcattc aggcctagct cttttatatt ttaactctga agcttctctg aacctttcta
 180
gggaaaaaga ggagcagcta actcgtgtga ctgaagttca gaggttgcag gcccagcagg
 240
cagatgccgc tctggaagag tttaagcggc aggtggaact gaactcagag aaagtctatg
 300
ctgaaatgaa agagcaggta aggaggctgc cacaccatcc cttttgaaag tcactctctc
 360
agtaggaagg tacattctca tcttaaatat gacagaaaca tttcactttt taaaagagct
 420
gtatcaaaat tagccataca atcgggtcag agattcagaa tgagaaagag atgttaccac
 480
ttgattcatc cttcaagcaa
 500

<210> 31
<211> 500
<212> DNA
<213> Homo sapiens

<400> 31
ttttaatttc cctccttaaa taattaaaat atattaaaat gaattgataa atgtctcatt
 60
attttaatac atttaaattc atcattatta ttggtgaggt gcttaaaatc tgtcatttaa
 120
acaaatggac aaattacaaa aacatggaac acaattgttt gtttaaaaaa ttaatatttg
 180
atttttaaat tttatagatg gaaaaagtgg aggcagatct aactagatcc aaatctcttc
 240
gtgagaaaca atcaaaggag tttttatggc aactggagga catcagacag cggtatgaac
 300
aacaggttgg tctttcattt tgatgctgcc attaaatttt taaaatacat atatgattta
 360
tcttttaaaa atcactatca caaaatatct gcataatgaa aatattacta agagctgttg
 420
ttaccaatat atttaaaata atgtttgttt caataaaatc tataaacaga attttaacac
 480
tccactgaag tttattctga
 500

<210> 32
<211> 500
<212> DNA
<213> Homo sapiens

<400> 32
ccaccccaac aaatgagagt atttcgaggg tgaggtaata atgatgtaaa aataaaaatt
 60
ttcaacttgg acaaattaga gaaaaattaa gctgtacatg agttgtcatg acgacaagga
 120
tgttctgttg atgaactgtc ctcctcattg tccaatcaga tagtagagct gaagctggag
 180
catgaacagg agaagacgca cctattacag cagcataacg cagagaagga tagcctagtc
 240
cgagaccatg aacgggaaat tgaaaacctg gaaaaacagc ttcgcgctgc caatatggag
 300
cacgaaaatc aaattcagga gttcaagaaa cgagatgcac aggtaattat caataatatt
 360
tgataaaacg ggtatctgtt tttaaaaagt catcagcgtt taatgttttc ttattattct
 420
tattgatgca cgctgtttga tcatttgcaa aactgacata taaatgtgat tttttttttg
 480
tttttctccc ccccaccccc
 500

<210> 33
<211> 500
<212> DNA
<213> Homo sapiens

<400> 33

aggaaggatg gagtgcaatc taagctttct attccatatg ttcatatttc atttattggc
60
cttttgccca ttggttcttc ttttgtgact tccctattga tatcttctgc ccatttttcc
120
atttttaaaa acaaggttat tgccgacatg gaggcccagg ttcacaagtt gagagaagaa
180
ttgatcaatg tgaactcaca gcggaaacag cagctggtag agcttggtct tcttcgtgaa
240
gaggaaaagc aaagggctac aagggaacat gagattgtcg tcaataaact gaaggctgaa
300
tcagaaaaga tgaaaataga gctgaaaaag actcatgctg ctgagacaga gatgacactg
360
gaaaaggtaa gatatttaac tagtttttta ataggcagtt gaaatccatg tttaaaatcc
420
attgccacca cccttatatt taatctccaa aaggtagtag acatggcatt actctttaaa
480
tgtataaact cttcagtcga
500

<210> 34
<211> 500
<212> DNA
<213> Homo sapiens

<400> 34
atgtttgtct aataattggc agaagagatc tttttttttt ttttgctttt taagtgatga
60
atagttggcg taacatcaaa gacaagccct gtggtttctt ggctgcccca gaagggcggt
120
gcagctggca ggcatcccag ctgcctggtg ataggcatgt ggaagagata ccaagctcac
180
aggtcactgt gtatgtgttc atgtacatgt gtttttcagg ccaacagcaa gctgaagcag
240
attgagaagg aatacactca gaagcttgcc aaatcttcac aggtaagcaa cattaaaaga
300
cacaaaacag ggtaaaacca aaacaaaaat gaaacctcca cataaaagcc ttggcacttt
360
caagttaaac ttcattcttt tcagtttttt tttgtggaat ggtatcatat ttttatgggt
420
atacaaataa acaatatggt tttaatattt cagtcattaa aaatcaagaa aaattaggca
480
tatttaacca tttgaaacaa
500

<210> 35
<211> 500
<212> DNA
<213> Homo sapiens

<400> 35
aaagtctttt attgtgtggt atttcatgca ttatagtgtg tgatatgtta attgaaaata
60

tttaatttgt ggaatagagt tttgtcaaat ccattctttt gttaagcatc aatacatata
 120
aaatagatgc ctctcccaga agaatttgaa gttatagcat gctgttttca ttttagatca
 180
tagcagaact tcagacaacc atttcctctc tgaaagaaga gaacagccag cagcagcttg
 240
ctgcagaaag gcggctccag gatgttagac aaaagtttga agatgagaag aagcagctga
 300
ttagagataa tgaccaagca atcaaggtaa tctgaagact tcagtaacca gtggtaccag
 360
ctgctttaat gtaattaaaa ggagggctga aatcccagca gtgggcagat gaatgcgggt
 420
atctggcttg ttcacacagc ctgcagtaat tccattcact ggactctacc tttgatgtat
 480
ccagtgactc aggctgactt
 500

<210> 36
<211> 500
<212> DNA
<213> Homo sapiens

<400> 36
ctttggaatt ttttatacta ggtattctta aagcagaaaa catcttggcc tgacacacaa
 60
aagtctggta ttgtttgcct gaatgctttg gaacaagagt aataaactag agatctgagg
 120
cctttttagt gccagttcat ggtgtagaac tatttttcca agtgtgctaa ttattaatga
 180
tatctttatt ttataccgtt tcaggtttta caagatgaat tagaaaaccg ttctaatcag
 240
gtgcgatgtg cagagaaaaa attacaacac aaagaattgg agtcacagga acaggtaccc
 300
tctcctttac tatttgactc tcttgtttac tttctctagg tcaagaagtg tgtgcagcca
 360
ggtgcagtgg ctcacacctg taatctcagc actttgggag gccgaggtgg gtggatcacc
 420
tgaagttagg agttcgagac tagcctggcc aacatggtga aaccctgtct ctactaaaaa
 480
tacaaaaatt aactgggtgt
 500

<210> 37
<211> 500
<212> DNA
<213> Homo sapiens

<400> 37
tctctcagag tcacagggtc agactgggta gccttttcag tttttaactc tttcttctta
 60
tttcatcttt attgtctggt ttctgaccgc gtgtaacaaa cctttcattg tgatgaacag
 120

atctgttctg ggacttctta aatccaaaac aatcatttat gtggtaaaaa gataactctt
 180
aatttttttc caatttagat aacttacata cgacaagaat atgaaacaaa attgaaagga
 240
ttgatgccag catccctaag acaagaactt gaagacacca tttcctccct aaaatcacag
 300
gtaattacta gaatcaaggc attttccaag gatttagggg atactctgaa aagaaacttg
 360
caaataaatc tgattccaaa ttgttgccag aaatcaatgt gatgtcattg cataacctac
 420
tactcaggat aaattttcat tttgttttaa atttttttct tacttgttta ataactcact
 480
gtctcaataa gaatttcttt
 500

<210> 38
<211> 500
<212> DNA
<213> Homo sapiens

<400> 38
gcaatgagga gtttgaactt tattctgtcg taactgatgg attttcaata aggatgggat
 60
gtgaccaaat gtaaaaaaca caacattgac attcaacaaa tcctctgagt tttatctagg
 120
gaattgtctg aatttaaata aaatcttacc atactatttg cacaatattg acatgtaagc
 180
cacgtccacc tcttctcaca caaggtgtct tttctaggtt aattttctgc aaaagagagc
 240
ttccatcctt caggaagaac tgactacata tcaaggcaga aggtacagcc cataagcagg
 300
tgtcttgtct cctgattagt ttgtgttgtg tcacctgtga gcctccttgt ctggcctttg
 360
tctttattaa aatgcttgga tataatacaa gttttgaaaa agacttgttt ttagtaagta
 420
gcatgtcttt aagaagtcac cccccttctc atcccagtgc ttagtctgta ctatcaaaac
 480
aagatgagag aatctgccgg
 500

<210> 39
<211> 500
<212> DNA
<213> Homo sapiens

<400> 39
aattaaaagg aaagatcttg tgaatcacac atttttcgaa agattctgtt tgctcggtgt
 60
aggtaagatt tgatccctta tagataagca taaattatga cattttgata tcaaatagcc
 120
ttttcaaccc ctatcaactt ctccagaatt gaagcttatt ttttgattga catacctgtt
 180

aaacctagag aaagtcgtga ctgcgatttt tggtttacag gtaactgcac gagagaatgc
 240
aacggatgca atttccaggc tgtgctgtgg acttcttcca gcaggtttga agatttggat
 300
attgtaaact gtgagatcag tggcattttt taaatcctta aatgtaatta agatcataaa
 360
aacatgcatc attcatacac tagtggggtg gttttatttg tgtctgccta agttattttt
 420
ttaacattcc tttatttccg attttcatgt ttgtgtgcat ttgagtatgt gctgagaact
 480
gcttatattg ggcaaagtga
 500

<210> 40
<211> 1000
<212> DNA
<213> Homo sapiens

<400> 40
ggaggctgag gcaggagaat cacgtgaacc tgggaggcgg aggttgcact gagccgagat
 60
tgcaccactg cactccagcc tgggtgacag agtgagactc catctcaaaa caaaaacaaa
 120
aacaaaaagc caaatatatc atttggactg tttggactcc tggtctcttc tcctattggg
 180
taggtgtggg gttggatgca gggaatggaa gttagactcg gagttgtacc cccattaaga
 240
taagtgaagg aatgcccaca gggctgggag cacagatttt caaccccttt tggaagatgt
 300
ccattgaagc ttgtctggac ctcctgtatt tgcctttgtt ttccttacct tagtttgtgt
 360
agaaatttta aaagctcatc catttaaaca gtatatacat aatgattttt ataaataaag
 420
acctctgtac atgtagtata caattctgct tagtcatcct acccatattg tttgactgat
 480
aaatgtgatc tttttgtctg tttttgtata gattagggaa tgactagaag taatttcttg
 540
tttgaggtgc aggtagggga gaaagcagtt tttaacatta ctggtgtaca aaattccgtg
 600
caaataggat gaataatggg gacaatggga caatattcct aattagttac aatattataa
 660
tgtttctgtt ttctcatttc agactaatac tagtaatgtt cacttgtatt atctgcctga
 720
tttatttacc atttctacct gaggcagata attcctttta atatttgttc atccattaaa
 780
attttcaatt caggaaagta aaggaaaaat taagtgtttc ttgatcctgc tttttatatg
 840
atatctttac cttgaatgtt tactggtggt tttttgtatc ttttggtgtt caggtcatca
 900
tatatataaa tagaggaata tagaaaccat tttttccgct gaattacaaa atagtcaatt
 960
ccatagtagt aaaagagtca actcttgatt tcctgatgta

1000

<210> 41
<211> 17
<212> DNA
<213> Homo sapiens

<400> 41
gtgtagtgaa ttacaag
 17

<210> 42
<211> 17
<212> DNA
<213> Homo sapiens

<400> 42
tgtagtgaat tacaaga
 17

<210> 43
<211> 17
<212> DNA
<213> Homo sapiens

<400> 43
gtagtgaatt acaagaa
 17

<210> 44
<211> 17
<212> DNA
<213> Homo sapiens

<400> 44
tagtgaatta caagaag
 17

<210> 45
<211> 17
<212> DNA
<213> Homo sapiens

<400> 45
agtgaattac aagaagt
 17

<210> 46
<211> 17
<212> DNA
<213> Homo sapiens

<400> 46
gtgaattaca agaagta
 17

<210> 47
<211> 17
<212> DNA
<213> Homo sapiens

<400> 47
tgaattacaa gaagtaa
 17

<210> 48
<211> 17
<212> DNA
<213> Homo sapiens

<400> 48
gaattacaag aagtaaa
 17

<210> 49
<211> 17
<212> DNA
<213> Homo sapiens

<400> 49
aattacaaga agtaaag
 17

<210> 50
<211> 17
<212> DNA
<213> Homo sapiens

<400> 50
attacaagaa gtaaagg
 17

<210> 51
<211> 17
<212> DNA
<213> Homo sapiens

<400> 51
ttacaagaag taaaggc
 17

<210> 52
<211> 17
<212> DNA

<213> Homo sapiens

<400> 52
tacaagaagt aaaggca
 17

<210> 53
<211> 25
<212> DNA
<213> Homo sapiens

<400> 53
gtgtagtgaa ttacaagaag taaag
 25

<210> 54
<211> 25
<212> DNA
<213> Homo sapiens

<400> 54
tgtagtgaat tacaagaagt aaagg
 25

<210> 55
<211> 25
<212> DNA
<213> Homo sapiens

<400> 55
gtagtgaatt acaagaagta aaggc
 25

<210> 56
<211> 25
<212> DNA
<213> Homo sapiens

<400> 56
tagtgaatta caagaagtaa aggca
 25

<210> 57
<211> 17
<212> DNA
<213> Homo sapiens

<400> 57
gagcagatgg aaaaagt
 17

<210> 58

<211> 17
<212> DNA
<213> Homo sapiens

<400> 58
agcagatgga aaaagtg
 17

<210> 59
<211> 17
<212> DNA
<213> Homo sapiens

<400> 59
gcagatggaa aaagtgg
 17

<210> 60
<211> 17
<212> DNA
<213> Homo sapiens

<400> 60
cagatggaaa aagtgga
 17

<210> 61
<211> 17
<212> DNA
<213> Homo sapiens

<400> 61
agatggaaaa agtggag
 17

<210> 62
<211> 17
<212> DNA
<213> Homo sapiens

<400> 62
gatggaaaaa gtggagg
 17

<210> 63
<211> 17
<212> DNA
<213> Homo sapiens

<400> 63
atggaaaaag tggaggc
 17

<210> 64
<211> 17
<212> DNA
<213> Homo sapiens

<400> 64
   tggaaaaagt ggaggca
    17

<210> 65
<211> 17
<212> DNA
<213> Homo sapiens

<400> 65
   ggaaaaagtg gaggcag
    17

<210> 66
<211> 17
<212> DNA
<213> Homo sapiens

<400> 66
   gaaaaagtgg aggcaga
    17

<210> 67
<211> 17
<212> DNA
<213> Homo sapiens

<400> 67
   aaaaagtgga ggcagat
    17

<210> 68
<211> 17
<212> DNA
<213> Homo sapiens

<400> 68
   aaaagtggag gcagatc
    17

<210> 69
<211> 17
<212> DNA
<213> Homo sapiens

<400> 69

aaagtggagg cagatct
 17

<210> 70
<211> 17
<212> DNA
<213> Homo sapiens

<400> 70
aagtggaggc agatcta
 17

<210> 71
<211> 17
<212> DNA
<213> Homo sapiens

<400> 71
agtggaggca gatctaa
 17

<210> 72
<211> 17
<212> DNA
<213> Homo sapiens

<400> 72
gtggaggcag atctaac
 17

<210> 73
<211> 17
<212> DNA
<213> Homo sapiens

<400> 73
tggaggcaga tctaact
 17

<210> 74
<211> 17
<212> DNA
<213> Homo sapiens

<400> 74
ggaggcagat ctaacta
 17

<210> 75
<211> 17
<212> DNA
<213> Homo sapiens

<400> 75
gaggcagatc taactag
 17

<210> 76
<211> 17
<212> DNA
<213> Homo sapiens

<400> 76
aggcagatct aactaga
 17

<210> 77
<211> 17
<212> DNA
<213> Homo sapiens

<400> 77
ggcagatcta actagat
 17

<210> 78
<211> 17
<212> DNA
<213> Homo sapiens

<400> 78
gcagatctaa ctagatc
 17

<210> 79
<211> 17
<212> DNA
<213> Homo sapiens

<400> 79
cagatctaac tagatcc
 17

<210> 80
<211> 17
<212> DNA
<213> Homo sapiens

<400> 80
agatctaact agatcca
 17

<210> 81
<211> 17

<212> DNA
<213> Homo sapiens

<400> 81
gatctaacta gatccaa
 17

<210> 82
<211> 17
<212> DNA
<213> Homo sapiens

<400> 82
atctaactag atccaaa
 17

<210> 83
<211> 17
<212> DNA
<213> Homo sapiens

<400> 83
tctaactaga tccaaat
 17

<210> 84
<211> 17
<212> DNA
<213> Homo sapiens

<400> 84
ctaactagat ccaaatc
 17

<210> 85
<211> 17
<212> DNA
<213> Homo sapiens

<400> 85
taactagatc caaatct
 17

<210> 86
<211> 17
<212> DNA
<213> Homo sapiens

<400> 86
aactagatcc aaatctc
 17

<210> 87
<211> 17
<212> DNA
<213> Homo sapiens

<400> 87
actagatcca aatctct
 17

<210> 88
<211> 17
<212> DNA
<213> Homo sapiens

<400> 88
ctagatccaa atctctt
 17

<210> 89
<211> 17
<212> DNA
<213> Homo sapiens

<400> 89
tagatccaaa tctcttc
 17

<210> 90
<211> 17
<212> DNA
<213> Homo sapiens

<400> 90
agatccaaat ctcttcg
 17

<210> 91
<211> 17
<212> DNA
<213> Homo sapiens

<400> 91
gatccaaatc tcttcgt
 17

<210> 92
<211> 17
<212> DNA
<213> Homo sapiens

<400> 92
atccaaatct cttcgtg

17

<210> 93
<211> 17
<212> DNA
<213> Homo sapiens

<400> 93
tccaaatctc ttcgtga
17

<210> 94
<211> 17
<212> DNA
<213> Homo sapiens

<400> 94
ccaaatctct tcgtgag
17

<210> 95
<211> 17
<212> DNA
<213> Homo sapiens

<400> 95
caaatctctt cgtgaga
17

<210> 96
<211> 17
<212> DNA
<213> Homo sapiens

<400> 96
aaatctcttc gtgagaa
17

<210> 97
<211> 17
<212> DNA
<213> Homo sapiens

<400> 97
aatctcttcg tgagaaa
17

<210> 98
<211> 17
<212> DNA
<213> Homo sapiens

<400> 98
atctcttcgt gagaaac
 17

<210> 99
<211> 17
<212> DNA
<213> Homo sapiens

<400> 99
tctcttcgtg agaaaca
 17

<210> 100
<211> 17
<212> DNA
<213> Homo sapiens

<400> 100
ctcttcgtga gaaacaa
 17

<210> 101
<211> 17
<212> DNA
<213> Homo sapiens

<400> 101
tcttcgtgag aaacaat
 17

<210> 102
<211> 17
<212> DNA
<213> Homo sapiens

<400> 102
cttcgtgaga aacaatc
 17

<210> 103
<211> 17
<212> DNA
<213> Homo sapiens

<400> 103
ttcgtgagaa acaatca
 17

<210> 104
<211> 17
<212> DNA

<213> Homo sapiens

<400> 104
tcgtgagaaa caatcaa
 17

<210> 105
<211> 17
<212> DNA
<213> Homo sapiens

<400> 105
cgtgagaaac aatcaaa
 17

<210> 106
<211> 17
<212> DNA
<213> Homo sapiens

<400> 106
gtgagaaaca atcaaag
 17

<210> 107
<211> 17
<212> DNA
<213> Homo sapiens

<400> 107
tgagaaacaa tcaaagg
 17

<210> 108
<211> 25
<212> DNA
<213> Homo sapiens

<400> 108
gagcagatgg aaaaagtgga ggcag
 25

<210> 109
<211> 25
<212> DNA
<213> Homo sapiens

<400> 109
agcagatgga aaaagtggag gcaga
 25

<210> 110

<211> 25
<212> DNA
<213> Homo sapiens

<400> 110
gcagatggaa aaagtggagg cagat
 25

<210> 111
<211> 25
<212> DNA
<213> Homo sapiens

<400> 111
cagatggaaa aagtggaggc agatc
 25

<210> 112
<211> 25
<212> DNA
<213> Homo sapiens

<400> 112
agatggaaaa agtggaggca gatct
 25

<210> 113
<211> 25
<212> DNA
<213> Homo sapiens

<400> 113
gatggaaaaa gtggaggcag atcta
 25

<210> 114
<211> 25
<212> DNA
<213> Homo sapiens

<400> 114
atggaaaaag tggaggcaga tctaa
 25

<210> 115
<211> 25
<212> DNA
<213> Homo sapiens

<400> 115
tggaaaaagt ggaggcagat ctaac
 25

<210> 116
<211> 25
<212> DNA
<213> Homo sapiens

<400> 116
   ggaaaaagtg gaggcagatc taact
    25

<210> 117
<211> 25
<212> DNA
<213> Homo sapiens

<400> 117
   gaaaaagtgg aggcagatct aacta
    25

<210> 118
<211> 25
<212> DNA
<213> Homo sapiens

<400> 118
   aaaaagtgga ggcagatcta actag
    25

<210> 119
<211> 25
<212> DNA
<213> Homo sapiens

<400> 119
aaaagtggag gcagatctaa ctaga
 25

<210> 120
<211> 25
<212> DNA
<213> Homo sapiens

<400> 120
aaagtggagg cagatctaac tagat
 25

<210> 121
<211> 25
<212> DNA
<213> Homo sapiens

<400> 121

aagtggaggc agatctaact agatc
25

<210> 122
<211> 25
<212> DNA
<213> Homo sapiens

<400> 122
agtggaggca gatctaacta gatcc
25

<210> 123
<211> 25
<212> DNA
<213> Homo sapiens

<400> 123
gtggaggcag atctaactag atcca
25

<210> 124
<211> 25
<212> DNA
<213> Homo sapiens

<400> 124
tggaggcaga tctaactaga tccaa
25

<210> 125
<211> 25
<212> DNA
<213> Homo sapiens

<400> 125
ggaggcagat ctaactagat ccaaa
25

<210> 126
<211> 25
<212> DNA
<213> Homo sapiens

<400> 126
gaggcagatc taactagatc caaat
25

<210> 127
<211> 25
<212> DNA
<213> Homo sapiens

<400> 127
aggcagatct aactagatcc aaatc
 25

<210> 128
<211> 25
<212> DNA
<213> Homo sapiens

<400> 128
ggcagatcta actagatcca aatct
 25

<210> 129
<211> 25
<212> DNA
<213> Homo sapiens

<400> 129
gcagatctaa ctagatccaa atctc
 25

<210> 130
<211> 25
<212> DNA
<213> Homo sapiens

<400> 130
cagatctaac tagatccaaa tctct
 25

<210> 131
<211> 25
<212> DNA
<213> Homo sapiens

<400> 131
agatctaact agatccaaat ctctt
 25

<210> 132
<211> 25
<212> DNA
<213> Homo sapiens

<400> 132
gatctaacta gatccaaatc tcttc
 25

<210> 133
<211> 25

<212> DNA
<213> Homo sapiens

<400> 133
atctaactag atccaaatct cttcg
 25

<210> 134
<211> 25
<212> DNA
<213> Homo sapiens

<400> 134
tctaactaga tccaaatctc ttcgt
 25

<210> 135
<211> 25
<212> DNA
<213> Homo sapiens

<400> 135
ctaactagat ccaaatctct tcgtg
 25

<210> 136
<211> 25
<212> DNA
<213> Homo sapiens

<400> 136
taactagatc caaatctctt cgtga
 25

<210> 137
<211> 25
<212> DNA
<213> Homo sapiens

<400> 137
aactagatcc aaatctcttc gtgag
 25

<210> 138
<211> 25
<212> DNA
<213> Homo sapiens

<400> 138
actagatcca aatctcttcg tgaga
 25

<210> 139
<211> 25
<212> DNA
<213> Homo sapiens

<400> 139
ctagatccaa atctcttcgt gagaa
 25

<210> 140
<211> 25
<212> DNA
<213> Homo sapiens

<400> 140
tagatccaaa tctcttcgtg agaaa
 25

<210> 141
<211> 25
<212> DNA
<213> Homo sapiens

<400> 141
agatccaaat ctcttcgtga gaaac
 25

<210> 142
<211> 25
<212> DNA
<213> Homo sapiens

<400> 142
gatccaaatc tcttcgtgag aaaca
 25

<210> 143
<211> 25
<212> DNA
<213> Homo sapiens

<400> 143
atccaaatct cttcgtgaga aacaa
 25

<210> 144
<211> 25
<212> DNA
<213> Homo sapiens

<400> 144
tccaaatctc ttcgtgagaa acaat

25

<210> 145
<211> 25
<212> DNA
<213> Homo sapiens

<400> 145
ccaaatctct tcgtgagaaa caatc
25

<210> 146
<211> 25
<212> DNA
<213> Homo sapiens

<400> 146
caaatctctt cgtgagaaac aatca
25

<210> 147
<211> 25
<212> DNA
<213> Homo sapiens

<400> 147
aaatctcttc gtgagaaaca atcaa
25

<210> 148
<211> 25
<212> DNA
<213> Homo sapiens

<400> 148
aatctcttcg tgagaaacaa tcaaa
25

<210> 149
<211> 25
<212> DNA
<213> Homo sapiens

<400> 149
atctcttcgt gagaaacaat caaag
25

<210> 150
<211> 25
<212> DNA
<213> Homo sapiens

<400> 150
tctcttcgtg agaaacaatc aaagg
 25

<210> 151
<211> 25
<212> DNA
<213> Homo sapiens

<400> 151
gcttcacaat gctgtagcag aaatg
 25

<210> 152
<211> 23
<212> DNA
<213> Homo sapiens

<400> 152
ctgttcctgt gactccaatt ctt
 23

## Description

### REFERENCE TO SEQUENCE LISTING SUBMITTED ON COMPACT DISC

The present application includes a Sequence Listing filed on one floppy disc containing a single file named pto_PB0152.txt, having 41 kilobytes, last modified on July 18, 2002 and recorded July 18, 2002. The Sequence Listing contained in said file is shown in Appendix 1.

### FIELD OF THE INVENTION

The present invention relates to novel human ZZAP1 protein. More specifically, the invention provides isolated nucleic acid molecules encoding human ZZAP1 protein, fragments thereof, vectors and host cells comprising isolated nucleic acid molecules encoding human ZZAP1 protein, human ZZAP1 polypeptides, antibodies, transgenic cells and non-human organisms, and diagnostic, therapeutic, and investigational methods of using the same.

### BACKGROUND OF THE INVENTION

Members of the vacuolar-type family of ATPases (V-ATPase) play central roles in energy-requiring cellular processes that involve the acidification of intracellular compartments in eukaryotic cells and are highly conserved (reviewed by Forgac M., *J Biol*. *Chem*. 274: 12951-12954 (1999); Briggs M.W. *et al, Virology* 280: 169-175 (2001)). This class of ATP hydrolyzing molecules, found coupled to a proton pump, primarily affects endocytotic activities but can also be involved in the acidification of other intracellular compartments, and in special cases, certain extracellular compartments (Forgac M., *J Biol*. *Chem.* 274: 12951-12954 (1999)).

Like other members of the larger ATPase family, V-ATPases are multi-subunit proteins with separate functional domains. In all ATPases, one subunit includes an ATP hydrolysis domain that converts ATP to ADP and an inorganic phosphate. The energy released from this hydrolysis reaction can drive a number of energy-requiring cellular activities. As a result, ATPase domains are found in an assortment of proteins which serve a variety of energy-requiring functions ranging from microtubule severing localized in mitotic spindle poles (McNally K.P. et *al, J*. *Cell Sci.* 113: 1623-1633 (2000)) to DNA repair (Richmond E. and Peterson C.L., *Nucleic Acids Res.* 24: 3685-3692 (1996)).

More specifically, members of the V-ATPase family of ATPases have two multi-subunit functional domains, V1 and V0. The V1 domain is related in sequence similarity and overall structure to an ATP synthase involved in ATP synthesis in mitochondria, chloroplasts and bacteria. Despite this homology to the ATP synthase, the V1 domain is known to function as an ATPase. The other domain, V0, does not normally function as a passive proton channel, but is involved in protein translocation when coupled with the energy released by the reactions of the V1 functional domain (reviewed by Forgac M., *FEBS Letters* 440: 258-263 (1998)).

V-ATPases functioning in active proton transport brings about the acidification of cellular compartments, which is mainly associated with the creation of a favorable environment for a range of cellular processes. For instance, proteins that contain V-ATPases that acidify intracellular compartments play an important role in receptor-mediated endocytosis by serving as a signal that activates release of internalized ligands from their receptors. Similarly, intracellular targeting of lysosomal enzymes occurs in response to the low pH created by V-ATPase acidification of a late recycling compartment which causes enzyme dissociation while allowing receptors to recycle to the trans-Golgi and the enzymes to be targeted to lysosomes (reviewed by Forgac M., *J. Biol*. *Chem.* 274: 12951-12954 (1999)). Along with these and other cellular processes, the V-ATPase domain has also been found to play a central role in osteoclast acidification of an extracellular compartment in bone required for the dissolution of inorganic bone material. It is therefore not surprising that a mislocalization of the V-ATPase domain has been associated with osteosclerosis (Scimeca J.C. *et al*, *Bone* 26: 207-213 (2000)) and a mutation in specifically the a-subunit of V-ATPases has been identified as a cause of human infantile malignant osteopetrosis (Kornak U. *et al*, *Hum. Mol*. *Gen.* 9: 2059-2063 (2000)).

Recently, V-ATPases have been the focus of a number of studies related to the diagnosis and treatment of cancer because V-ATPases have been shown to be expressed on the plasma membrane of cancer cells and an overexpression of V-ATPases has been implicated in the process of cell invasion (Kubota S. and Seyama *Y., Biochem*. *and Biophys*. *Res*. *Comm*. 278: 390-394 (2000)). Furthermore, studies have also shown that V-ATPases are involved in the development of drug resistance to breast cancer treatments (Martinez-Zaguilan R. *et al*, *Biochem*. *Pharm.* 57: 1037-1046 (1999)). Another recent study reported that because proteins containing V-ATPase domains influence cell growth and differentiation, the overexpression of some V-ATPases may play a crucial role in tumor progression as it is characteristic of invasive pancreatic tumors (Ohta T. *et al*, *British Journal of Cancer* 73: 1511-1517 (1996). As a result, V-ATPases have been identified as a treatment target for human pancreatic cancer (Ohta T. *et al*, *Japanese Journal of Cancer and Chemotherapy* 23: 1660-1664 (1996)).

Other recent studies have implicated the endocytotic role of V-ATPase domains in the development of acquired immunodeficiency syndrome (AIDS). Specifically, the catalytic subunit H of V-ATPase is believed to facilitate internalization of the accessory protein negative factor (Nef) from human immunodeficiency virus (HIV), a key step in the progression from HIV to AIDS, and required for optimal viral infectivity (Lu X. *et al*, *Immunity* 8: 647-656 (1998); Mandic R. *et al*, *Mol*. *Biol*. *of the Cell* 12: 463-473 (2001)).

Given the importance of V-ATPases in cellular processes and disease diagnosis and treatment, there is a need to identify novel genes that encode proteins with this structural motif.

### SUMMARY OF THE INVENTION

The present invention solves these and other needs in the art by providing isolated nucleic acids that encode ZZAP1 (so named because it contains two bZIP transcription factor motifs and similarity to V-ATPases), and fragments thereof.

In other aspects, the invention provides vectors for propagating and expressing the nucleic acids of the present invention, host cells comprising the nucleic acids and vectors of the present invention, proteins, protein fragments, and protein fusions of the human ZZAP1, and antibodies thereto.

The invention further provides pharmaceutical formulations of the nucleic acids, proteins, and antibodies of the present invention.

In other aspects, the invention provides transgenic cells and non-human organisms comprising human ZZAP1 nucleic acids, and transgenic cells and non-human organisms with targeted disruption of the endogenous orthologue of the human ZZAP1.

The invention additionally provides diagnostic, investigational, and therapeutic methods based on the human ZZAP1 nucleic acids, proteins, and antibodies of the present invention.

### BRIEF DESCRIPTION OF THE DRAWINGS

The above and other objects and advantages of the present invention will be apparent upon consideration of the following detailed description taken in conjunction with the accompanying drawings, in which like characters refer to like parts throughout, and in which:
FIG. 1 (A) schematizes the protein domain structure of human ZZAP1, and FIG. 1 (B) schematizes the protein domain structure of the earlier-described putative macaque homologue.
FIG. 2 is a map showing the genomic structure of human ZZAP1 encoded at chromosome 17q23.3.
FIG. 3 presents the nucleotide and predicted amino acid sequences of human ZZAP1; and
FIG. 4 presents the expression profile of human ZZAP1 by RT-PCR.

### DETAILED DESCRIPTION OF THE INVENTION

Mining the sequence of the human genome for novel human genes, the present inventors have identified ZZAP1, a novel molecule that functions in protein-protein interactions, aberrant expression of which is likely associated with the development of certain types of cancer or other diseases (such as osteosclerosis or AIDS).

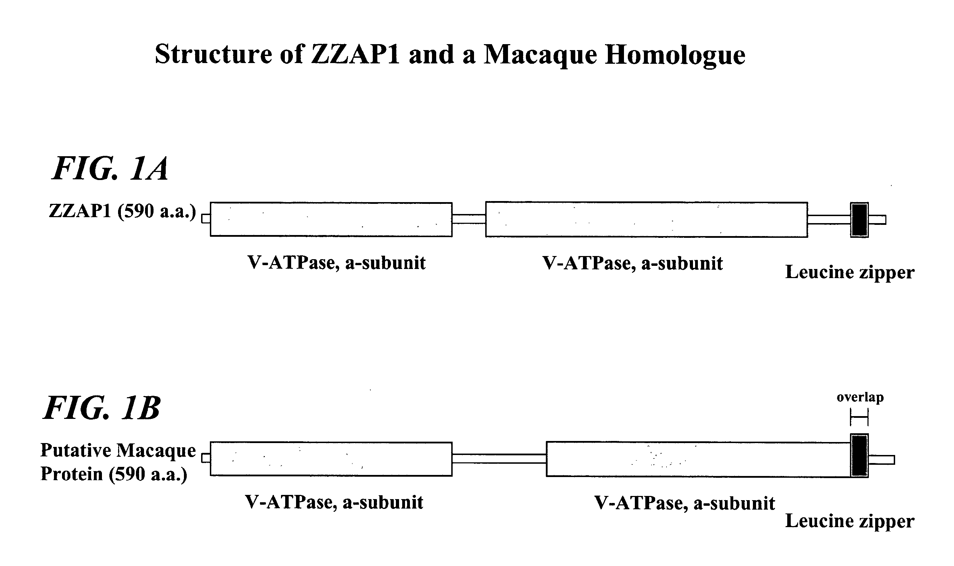

As schematized in FIG. 1, the newly isolated ZZAP1 shares certain protein domains and an overall structural organization with a putative macaque transcript (GenBank accession number BAB21900). The shared structural features strongly imply that human ZZAP1 plays a role similar to that of other proteins containing V-ATPase motifs in having protein-protein interaction activity, aberrant expression of some of these proteins leads to cancer, osteosclerosis or AIDS.

Like other proteins with ATP hydrolysis activity, ZZAP1 has two V-ATPase domains occuring at amino acids 2-222 and again at 271-544 (http://pfam.wustl.edu/). These domains bear particular homology to the membrane-spanning α-subunit of V-ATPases. V-ATPase domains are involved in hydrolyzing ATP to ADP and an inorganic phosphate, and operating as ATP-dependent proton pumps. These domains are present in a number of different proteins and are involved in the acidification of intra- and extra-cellular compartments.

In common with the putative macaque transcript (GenBank accession number BAB21900), ZZAP1 also has a leucine zipper motif at amino acids 558-579. Leucine zippers are found in a number of gene regulatory proteins and consist of a periodic repetition of leucine residues at every seventh turn forming an alpha-helical conformation able to interact with other polypeptides to facilitate dimerization. As a result, it is believed that leucine zipper motifs play a key role in eukaryotic gene regulation through its role in protein-protein interaction activity and protein-DNA binding.

Other signatures of the newly isolated ZZAP1 proteins were identified by searching the PROSITE database (http://www.expasy.ch/tools/scnpsitl.html). These include one N-glycosylation site (512-515), two cAMP- and cGMP-dependent protein kinase phosphorylation sites (93-96 and 574-577), seven protein kinase C phosphorylation sites (202-204, 446-448, 249-251, 412-414, 272-274, 553-555, 377-379), six Casein kinase II phosphorylation sites (83-86, 117-120, 139-142, 193-196, 272-275, and 465-468), two Tyrosine kinase phosphorylation sites (73-80, 439-445), a single *N*-myristoylation site (548-553), and one amidation site (587-590).

Additional signatures of the newly isolated ZZAP1 proteins were identified by searching the PFAM database (http://pfam.wostl.edu/cgi-bin/nph-hmmsearch). These include two bZIP transcription factor motifs (45-75 and 141-157), one Ribosomal protein S21 motif (224-277), one Myosin tail pattern (326-361), a single DNA gyrase/topoisomerase IV, subunit a domain (358-380), and an Hr1 repeat motif (479-562).

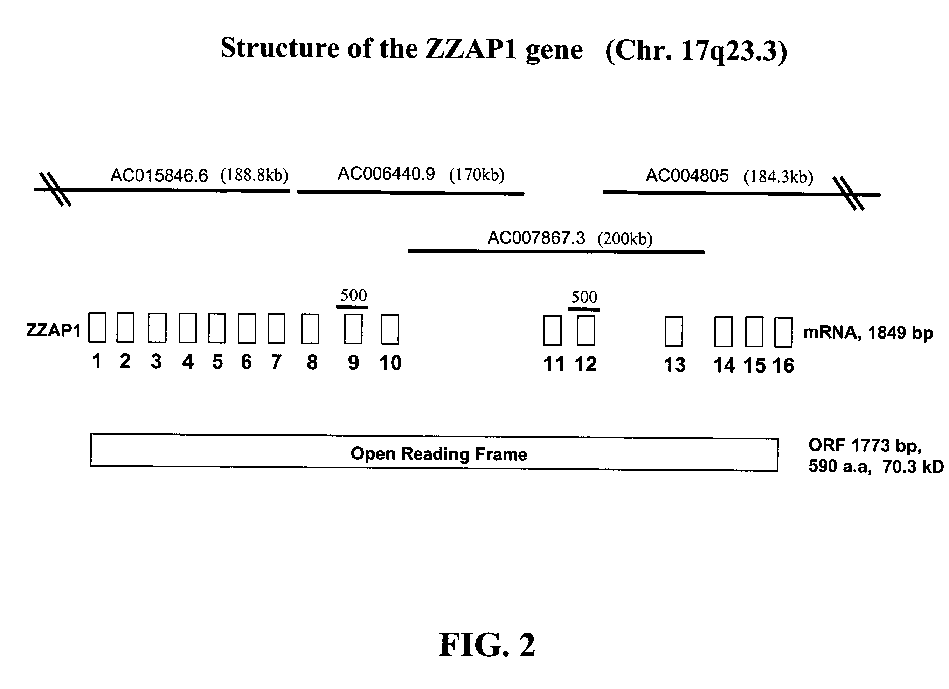

FIG. 2 shows the genomic organization of ZZAP1.

At the top is shown the four bacterial artificial chromosomes (BACs), with GenBank accession numbers, that span the ZZAP1 locus. The genome-derived single-exon probes first used to demonstrate expression from this locus are shown below the BACs and labeled "500". The 500 bp probes includes sequence drawn from exons 9 and 12, with additional sequence from adjacent introns.

As shown in FIG. 2, ZZAP1 is comprised of exons 1-16 and encodes a protein of 590 amino acids, with a predicted molecular weight, prior to any post-translational modification, of 70.4kD.

As further discussed in the examples herein, expression of ZZAP1 was assessed using hybridization to genome-derived single exon microarrays and RT-PCR. Microarray analysis of exons nine and twelve showed expression in adult liver, brain, fetal liver, lung, and placenta as well as cell lines, hela and bt474. RT-PCR further provided expression data for testis, skeletal muscle, heart and bone marrow. This was confirmed by quantitative RT-PCR.

As more fully described below, the present invention provides isolated nucleic acids that encode ZZAP1 and fragments thereof. The invention further provides vectors for propagation and expression of the nucleic acids of the present invention, host cells comprising the nucleic acids and vectors of the present invention, proteins, protein fragments, and protein fusions of the present invention, and antibodies specific for all or any one of the isoforms. The invention provides pharmaceutical formulations of the nucleic acids, proteins, and antibodies of the present invention. The invention further provides transgenic cells and non-human organisms comprising human ZZAP1 nucleic acids, and transgenic cells and non-human organisms with targeted disruption of the endogenous orthologue of the human ZZAP1. The invention additionally provides diagnostic, investigational, and therapeutic methods based on the ZZAP1 nucleic acids, proteins, and antibodies of the present invention.

### DEFINITIONS

Unless defined otherwise, all technical and scientific terms used herein have the meaning commonly understood by one of ordinary skill in the art to which this invention belongs.

As used herein, **"nucleic acid"** (synonymously, **"polynucleotide")** includes polynucleotides having natural nucleotides in native 5'-3' phosphodiester linkage - *e.g.,* DNA or RNA - as well as polynucleotides that have nonnatural nucleotide analogues, nonnative internucleoside bonds, or both, so long as the nonnatural polynucleotide is capable of sequence-discriminating basepairing under experimentally desired conditions. Unless otherwise specified, the term "nucleic acid" includes any topological conformation; the term thus explicitly comprehends single-stranded, double-stranded, partially duplexed, triplexed, hairpinned, circular, and padlocked conformations.

As used herein, an **"isolated nucleic acid"** is a nucleic acid molecule that exists in a physical form that is nonidentical to any nucleic acid molecule of identical sequence as found in nature; "isolated" does not require, although it does not prohibit, that the nucleic acid so described has itself been physically removed from its native environment.

For example, a nucleic acid can be said to be "isolated" when it includes nucleotides and/or internucleoside bonds not found in nature. When instead composed of natural nucleosides in phosphodiester linkage, a nucleic acid can be said to be "isolated" when it exists at a purity not found in nature, where purity can be adjudged with respect to the presence of nucleic acids of other sequence, with respect to the presence of proteins, with respect to the presence of lipids, or with respect the presence of any other component of a biological cell, or when the nucleic acid lacks sequence that flanks an otherwise identical sequence in an organism's genome, or when the nucleic acid possesses sequence not identically present in nature.

As so defined, "isolated nucleic acid" includes nucleic acids integrated into a host cell chromosome at a heterologous site, recombinant fusions of a native fragment to a heterologous sequence, recombinant vectors present as episomes or as integrated into a host cell chromosome.

As used herein, an isolated nucleic acid "encodes" a reference polypeptide when at least a portion of the nucleic acid, or its complement, can be directly translated to provide the amino acid sequence of the reference polypeptide, or when the isolated nucleic acid can be used, alone or as part of an expression vector, to express the reference polypeptide *in vitro,* in a prokaryotic host cell, or in a eukaryotic host cell.

As used herein, the term "**exon**" refers to a nucleic acid sequence found in genomic DNA that is bioinformatically predicted and/or experimentally confirmed to contribute contiguous sequence to a mature mRNA transcript.

As used herein, the phrase **"open reading frame"** and the equivalent acronym **"ORF"** refer to that portion of a transcript-derived nucleic acid that can be translated in its entirety into a sequence of contiguous amino acids. As so defined, an ORF has length, measured in nucleotides, exactly divisible by 3. As so defined, an ORF need not encode the entirety of a natural protein.

As used herein, the phrase **"ORF-encoded peptide"** refers to the predicted or actual translation of an ORF.

As used herein, the phrase **"degenerate variant"** of a reference nucleic acid sequence intends all nucleic acid sequences that can be directly translated, using the standard genetic code, to provide an amino acid sequence identical to that translated from the reference nucleic acid sequence.

As used herein, the term **"microarray"** and the equivalent phrase **"nucleic acid microarray"** refer to a substrate-bound collection of plural nucleic acids, hybridization to each of the plurality of bound nucleic acids being separately detectable. The substrate can be solid or porous, planar or non-planar, unitary or distributed.

As so defined, the term "microarray" and phrase "nucleic acid microarray" include all the devices so called in Schena (ed.), DNA Microarrays: A Practical Approach (Practical Approach Series), Oxford University Press (1999) (ISBN: 0199637768); *Nature Genet.* 21(1)(suppl):1 - 60 (1999); and Schena (ed.), Microarray Biochip: Tools and Technology, Eaton Publishing Company/BioTechniques Books Division (2000) (ISBN: 1881299376), the disclosures of which are incorporated herein by reference in their entireties.

As so defined, the term "microarray" and phrase "nucleic acid microarray" also include substrate-bound collections of plural nucleic acids in which the plurality of nucleic acids are distributably disposed on a plurality of beads, rather than on a unitary planar substrate, as is described, *inter alia,* in Brenner *et al.*, *Proc. Natl*. *Acad*. *Sci*. *USA* 97(4):166501670 (2000), the disclosure of which is incorporated herein by reference in its entirety; in such case, the term "microarray" and phrase "nucleic acid microarray" refer to the plurality of beads in aggregate.

As used herein with respect to solution phase hybridization, the term **"probe",** or equivalently, **"nucleic acid probe"** or **"hybridization probe",** refers to an isolated nucleic acid of known sequence that is, or is intended to be, detectably labeled. As used herein with respect to a nucleic acid microarray, the term "probe" (or equivalently "nucleic acid probe" or "hybridization probe") refers to the isolated nucleic acid that is, or is intended to be, bound to the substrate. In either such context, the term "target" refers to nucleic acid intended to be bound to probe by sequence complementarity.

As used herein, the expression **"probe comprising SEQ ID NO:X",** and variants thereof, intends a nucleic acid probe, at least a portion of which probe has either (i) the sequence directly as given in the referenced SEQ ID NO:X, or (ii) a sequence complementary to the sequence as given in the referenced SEQ ID NO:X, the choice as between sequence directly as given and complement thereof dictated by the requirement that the probe be complementary to the desired target.

As used herein, the phrases **"expression of a probe"** and **"expression of an isolated nucleic acid"** and their linguistic equivalents intend that the probe or, (respectively, the isolated nucleic acid), or a probe (or, respectively, isolated nucleic acid) complementary in sequence thereto,can hybridize detectably under high stringency conditions to a sample of nucleic acids that derive from mRNA transcripts from a given source. For example, and by way of illustration only, expression of a probe in "liver" means that the probe can hybridize detectably under high stringency conditions to a sample of nucleic acids that derive from mRNA obtained from liver.

As used herein, **"a single exon probe"** comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and that consist of one or more exons that are found adjacent to the reference exon in the genome.

For purposes herein, **"high stringency conditions"** are defined for solution phase hybridization as aqueous hybridization (i.e., free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. **"Moderate stringency conditions"** are defined for solution phase hybridization as aqueous hybridization (i.e., free of formamide) in 6X SSC, 1% SDS at 65°C for at least 8 hours, followed by one or more washes in 2x SSC, 0.1% SDS at room temperature.

For microarray-based hybridization, standard "high stringency conditions" are defined as hybridization in 50% formamide, 5X SSC, 0.2 µg/µl poly(dA), 0.2 µg/µl human cot1 DNA, and 0.5% SDS, in a humid oven at 42°C overnight, followed by successive washes of the microarray in 1X SSC, 0.2% SDS at 55°C for 5 minutes, and then 0.1X SSC, 0.2% SDS, at 55°C for 20 minutes. For microarray-based hybridization, "moderate stringency conditions", suitable for cross-hybridization to mRNA encoding structurally- and functionally-related proteins, are defined to be the same as those for high stringency conditions but with reduction in temperature for hybridization and washing to room temperature (approximately 25°C).

As used herein, the terms **"protein", "polypeptide",** and **"peptide"** are used interchangeably to refer to a naturally-occurring or synthetic polymer of amino acid monomers (residues), irrespective of length, where amino acid monomer here includes naturally-occurring amino acids, naturally-occurring amino acid structural variants, and synthetic non-naturally occurring analogs that are capable of participating in peptide bonds. The terms "protein", "polypeptide", and "peptide" explicitly permits of post-translational and post-synthetic modifications, such as glycosylation.

The term "**oligopeptide**" herein denotes a protein, polypeptide, or peptide having 25 or fewer monomeric subunits.

The phrases **"isolated protein", "isolated polypeptide", "isolated peptide"** and **"isolated oligopeptide"** refer to a protein (or respectively to a polypeptide, peptide, or oligopeptide) that is nonidentical to any protein molecule of identical amino acid sequence as found in nature; "isolated" does not require, although it does not prohibit, that the protein so described has itself been physically removed from its native environment.

For example, a protein can be said to be "isolated" when it includes amino acid analogues or derivatives not found in nature, or includes linkages other than standard peptide bonds.

When instead composed entirely of natural amino acids linked by peptide bonds, a protein can be said to be "isolated" when it exists at a purity not found in nature - where purity can be adjudged with respect to the presence of proteins of other sequence, with respect to the presence of non-protein compounds, such as nucleic acids, lipids, or other components of a biological cell, or when it exists in a composition not found in nature, such as in a host cell that does not naturally express that protein.

A "**purified protein**" (equally, a purified polypeptide, peptide, or oligopeptide) is an isolated protein, as above described, present at a concentration of at least 95%, as measured on a weight basis with respect to total protein in a composition. A **"substantially purified protein"** (equally, a substantially purified polypeptide, peptide, or oligopeptide) is an isolated protein, as above described, present at a concentration of at least 70%, as measured on a weight basis with respect to total protein in a composition.

As used herein, the phrase **"protein isoforms"** refers to a plurality of proteins having nonidentical primary amino acid sequence but that share amino acid sequence encoded by at least one common exon.

As used herein, the phrase **"alternative splicing"** and its linguistic equivalents includes all types of RNA processing that lead to expression of plural protein isoforms from a single gene; accordingly, the phrase **"splice variant(s)"** and its linguistic equivalents embraces mRNAs transcribed from a given gene that, however processed, collectively encode plural protein isoforms. For example, and by way of illustration only, splice variants can include exon insertions, exon extensions, exon truncations, exon deletions, alternatives in the 5' untranslated region ("5' UT") and alternatives in the 3' untranslated region ("3' UT"). Such 3' alternatives include, for example, differences in the site of RNA transcript cleavage and site of poly(A) addition. See, *e.g.,* Gautheret *et al*., *Genome Res.* 8:524-530 (1998).

As used herein, **"orthologues"** are separate occurrences of the same gene in multiple species. The separate occurrences have similar, albeit nonidentical, amino acid sequences, the degree of sequence similarity depending, in part, upon the evolutionary distance of the species from a common ancestor having the same gene.

As used herein, the term **"paralogues"** indicates separate occurrences of a gene in one species. The separate occurrences have similar, albeit nonidentical, amino acid sequences, the degree of sequence similarity depending, in part, upon the evolutionary distance from the gene duplication event giving rise to the separate occurrences.

As used herein, the term "**homologues**" is generic to "orthologues" and "paralogues".

As used herein, the term "**antibody**" refers to a polypeptide, at least a portion of which is encoded by at least one immunoglobulin gene, or fragment thereof, and that can bind specifically to a desired target molecule. The term includes naturally-occurring forms, as well as fragments and derivatives.

Fragments within the scope of the term "antibody" include those produced by digestion with various proteases, those produced by chemical cleavage and/or chemical dissociation, and those produced recombinantly, so long as the fragment remains capable of specific binding to a target molecule. Among such fragments are Fab, Fab', Fv, F(ab)'₂, and single chain Fv (scFv) fragments.

Derivatives within the scope of the term include antibodies (or fragments thereof) that have been modified in sequence, but remain capable of specific binding to a target molecule, including: interspecies chimeric and humanized antibodies; antibody fusions; heteromeric antibody complexes and antibody fusions, such as diabodies (bispecific antibodies), single-chain diabodies, and intrabodies (see, *e.g.*, Marasco (ed.), IntracellularAntibodies: Research and Disease Applications, Springer-Verlag New York, Inc. (1998) (ISBN: 3540641513), the disclosure of which is incorporated herein by reference in its entirety).

As used herein, antibodies can be produced by any known technique, including harvest from cell culture of native B lymphocytes, harvest from culture of hybridomas, recombinant expression systems, and phage display.

As used herein, "**antigen**" refers to a ligand that can be bound by an antibody; an antigen need not itself be immunogenic. The portions of the antigen that make contact with the antibody are denominated **"epitopes"**.

"**Specific binding**" refers to the ability of two molecular species concurrently present in a heterogeneous (inhomogeneous) sample to bind to one another in preference to binding to other molecular species in the sample. Typically, a specific binding interaction will discriminate over adventitious binding interactions in the reaction by at least two-fold, more typically by at least 10-fold, often at least 100-fold; when used to detect analyte, specific binding is sufficiently discriminatory when determinative of the presence of the analyte in a heterogeneous (inhomogeneous) sample. Typically, the affinity or avidity of a specific binding reaction is least about 10⁻⁷ M, with specific binding reactions of greater specificity typically having affinity or avidity of at least 10⁻⁸ M to at least about 10⁻⁹ M.

As used herein, **"molecular binding partners"** - and equivalently, **"specific binding partners"** - refer to pairs of molecules, typically pairs of biomolecules, that exhibit specific binding. Nonlimiting examples are receptor and ligand, antibody and antigen, and biotin to any of avidin, streptavidin, neutrAvidin and captAvidin.

The term "**antisense**", as used herein, refers to a nucleic acid molecule sufficiently complementary in sequence, and sufficiently long in that complementary sequence, as to hybridize under intracellular conditions to (i) a target mRNA transcript or (ii) the genomic DNA strand complementary to that transcribed to produce the target mRNA transcript.

The term "**portion**", as used with respect to nucleic acids, proteins, and antibodies, is synonymous with "fragment".

### NUCLEIC ACID MOLECULES

In a first aspect, the invention provides isolated nucleic acids that encode ZZAP1, variants having at least 65% sequence identity thereto, degenerate variants thereof, variants that encode ZZAP1 proteins having conservative or moderately conservative substitutions, cross-hybridizing nucleic acids, and fragments thereof.

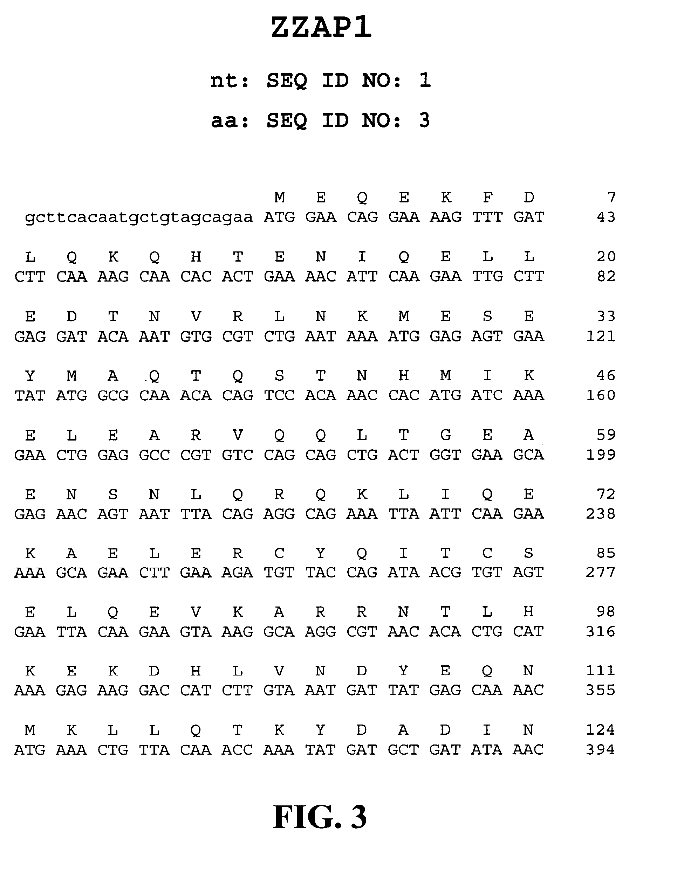

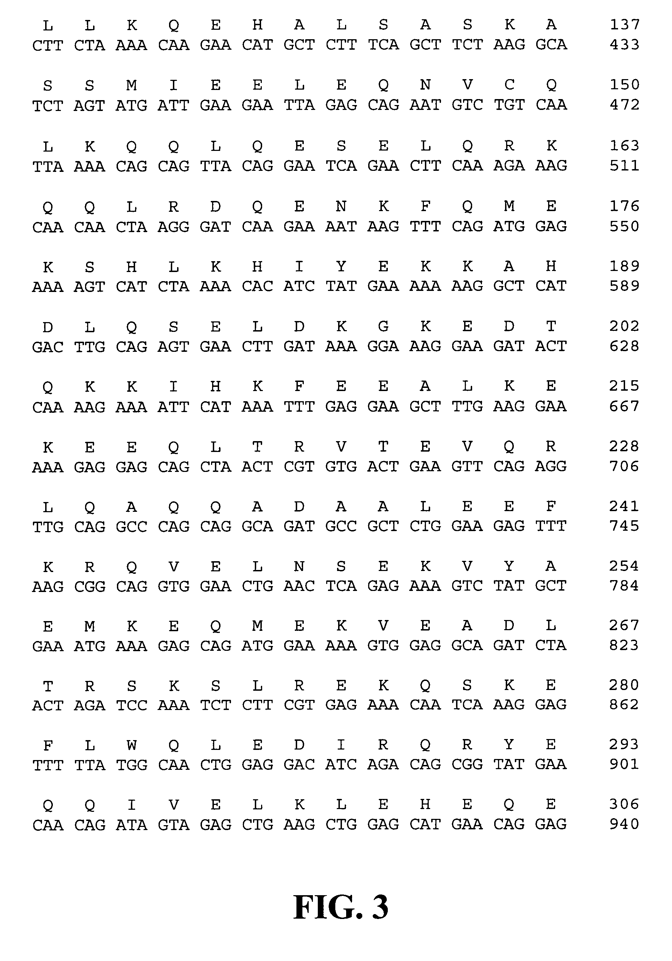

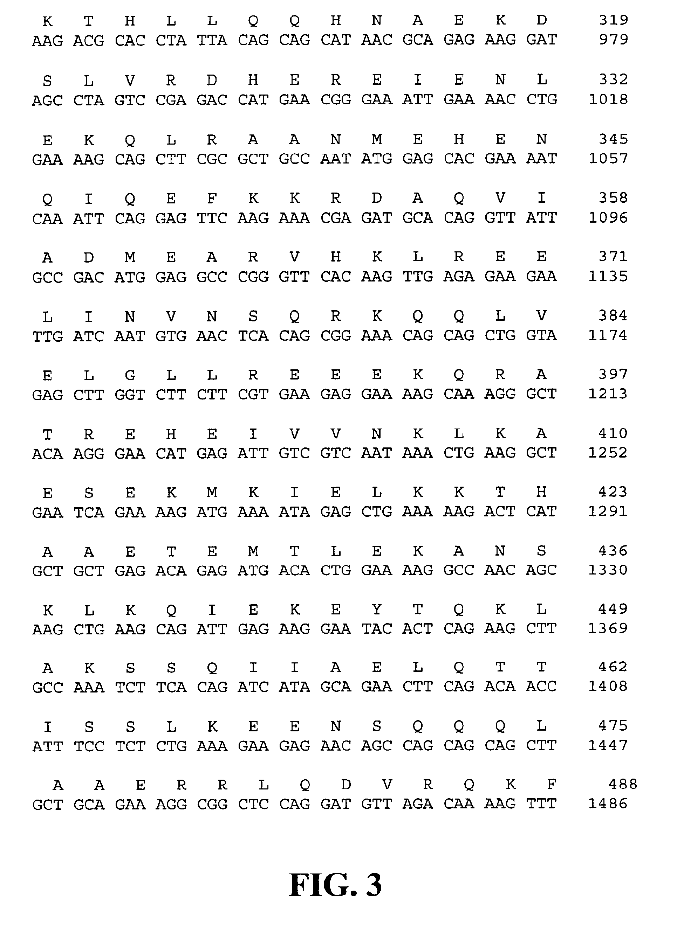

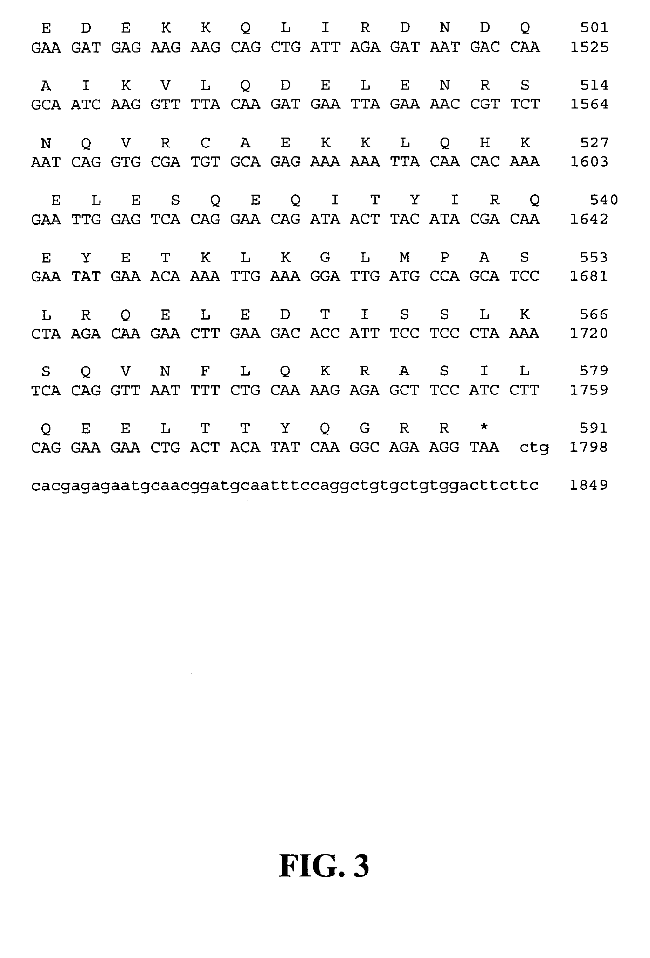

FIG. 3 presents the nucleotide sequence of the ZZAP1 cDNA clone, with predicted amino acid translation; the sequences are further presented in the Sequence Listing, incorporated herein by reference in its entirety, as follows: SEQ ID NOs: 1 (full length nucleotide sequence of ZZAP1 cDNA) and 3 (full length amino acid coding sequence of ZZAP1).

Unless otherwise indicated, each nucleotide sequence is set forth herein as a sequence of deoxyribonucleotides. It is intended, however, that the given sequence be interpreted as would be appropriate to the polynucleotide composition: for example, if the isolated nucleic acid is composed of RNA, the given sequence intends ribonucleotides, with uridine substituted for thymidine.

Unless otherwise indicated, nucleotide sequences of the isolated nucleic acids of the present invention were determined by sequencing a DNA molecule that had resulted, directly or indirectly, from at least one enzymatic polymerization reaction (*e.g*., reverse transcription and/or polymerase chain reaction) using an automated sequencer (such as the MegaBACE™ 1000, Amersham Biosciences, Sunnyvale, CA, USA), or by reliance upon such sequence or upon genomic sequence prior-accessioned into a public database. Unless otherwise indicated, all amino acid sequences of the polypeptides of the present invention were predicted by translation from the nucleic acid sequences so determined.

As a consequence, any nucleic acid sequence presented herein may contain errors introduced by erroneous incorporation of nucleotides during polymerization, by erroneous base calling by the automated sequencer (although such sequencing errors have been minimized for the nucleic acids directly determined herein, unless otherwise indicated, by the sequencing of each of the complementary strands of a duplex DNA), or by similar errors accessioned into the public database.

Accordingly, the ZZAP1 cDNA clone described herein has been deposited in a public repository (American Type Culture Collection, Manassas, Virginia, USA) on August 2, 2001 under accession number PTA-3582. Any errors in sequence reported herein can be determined and corrected by sequencing nucleic acids propagated from the deposited clones using standard techniques.

Single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Additionally, small deletions and insertions, rather than single nucleotide polymorphisms, are not uncommon in the general population, and often do not alter the function of the protein.

Accordingly, it is an aspect of the present invention to provide nucleic acids not only identical in sequence to those described with particularity herein, but also to provide isolated nucleic acids at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention, as by random or directed mutagenesis.

For purposes herein, percent identity of two nucleic acid sequences is determined using the procedure of Tatiana *et* al., "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/b12seq/b12.html.
To assess percent identity of nucleic acids, the BLASTN module of BLAST 2 SEQUENCES is used with default values of (i) reward for a match: 1; (ii) penalty for a mismatch: -2; (iii) open gap 5 and extension gap 2 penalties; (iv) gap X_dropoff 50 expect 10 word size 11 filter, and both sequences are entered in their entireties.

As is well known, the genetic code is degenerate, with each amino acid except methionine translated from a plurality of codons, thus permitting a plurality of nucleic acids of disparate sequence to encode the identical protein. As is also well known, codon choice for optimal expression varies from species to species. The isolated nucleic acids of the present invention being useful for expression of ZZAP1 proteins and protein fragments, it is, therefore, another aspect of the present invention to provide isolated nucleic acids that encode ZZAP1 proteins and portions thereof not only identical in sequence to those described with particularity herein, but degenerate variants thereof as well.

As is also well known, amino acid substitutions occur frequently among natural allelic variants, with conservative substitutions often occasioning only *de minimis* change in protein function.

Accordingly, it is an aspect of the present invention to provide nucleic acids not only identical in sequence to those described with particularity herein, but also to provide isolated nucleic acids that encode ZZAP1, and portions thereof, having conservative amino acid substitutions, and also to provide isolated nucleic acids that encode ZZAP1, and portions thereof, having moderately conservative amino acid substitutions.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al., Science* 256(5062):1443-5 (1992)): 
| A | R | N | D | C | Q | E | G | H | I | L | K | M | F | P | S | T | W | Y | V
A | 2 | -1 | 0 | 0 | 0 | 0 | 0 | 0 | -1 | -1 | -1 | 0 | -1 | -2 | 0 | 1 | 1 | -4 | -2 | 0
R | -1 | 5 | 0 | 0 | -2 | 2 | 0 | -1 | 1 | -2 | -2 | 3 | -2 | -3 | -1 | 0 | 0 | -2 | -2 | -2
N | 0 | 0 | 4 | 2 | -2 | 1 | 1 | 0 | 1 | -3 | -3 | 1 | -2 | -3 | -1 | 1 | 0 | -4 | -1 | -2
D | 0 | 0 | 2 | 5 | -3 | 1 | 3 | 0 | 0 | -4 | -4 | 0 | -3 | -4 | -1 | 0 | 0 | -5 | -3 | -3
C | 0 | -2 | -2 | -3 | 12 | -2 | -3 | -2 | -1 | -1 | -2 | -3 | -1 | -1 | -3 | 0 | 0 | -1 | 0 | 0
Q | 0 | 2 | 1 | 1 | -2 | 3 | 2 | -1 | 1 | -2 | -2 | 2 | -1 | -3 | 0 | 0 | 0 | -3 | -2 | -2
 
E 0 0 1 3 -3 2 4 -1 0 -3 -3 1 -2 -4 0 0 0 -4 -3 -2
G 0 -1 0 0 -2 -1 -1 7 -1 -4 -4 -1 -4 -5 -2 0 -1 -4 -4 -3
H -1 1 1 0 -1 1 0 -1 6 -2 -2 1 -1 0 -1 0 0 -1 2 -2
I -1 -2 -3 -4 -1 -2 -3 -4 -2 4 3 -2 2 1 -3 -2 -1 -2 -1 3
L -1 -2 -3 -4 -2 -2 -3 -4 -2 3 4 -2 3 2 -2 -2 -1 -1 0 2
K 0 3 1 0 -3 2 1 -1 1 -2 -2 3 -1 -3 -1 0 0 -4 -2 -2
M -1 -2 -2 -3 -1 -1 -2 -4 -1 2 3 -1 4 2 -2 -1 -1 -1 0 2
F -2 -3 -3 -4 -1 -3 -4 -5 0 1 2 -3 2 7 -4 -3 -2 4 5 0
P 0 -1 -1 -1 -3 0 0 -2 -1 -3 -2 -1 -2 -4 8 0 0 -5 -3 -2
S 1 0 1 0 0 0 0 0 0 -2 -2 0 -1 -3 0 2 2 -3 -2 -1
T 1 0 0 0 0 0 0 -1 0 -1 -1 0 -1 -2 0 2 2 -4 -2 0
W -4 -2 -4 -5 -1 -3 -4 -4 -1 -2 -1 -4 -1 4 -5 -3 -4 14 4 -3
Y -2 -2 -1 -3 0 -2 -3 -4 2 -1 0 -2 0 5 -3 -2 -2 4 8 -1
V 0 -2 -2 -3 0 -2 -2 -3 -2 3 2 -2 2 0 -2 -1 0 -3 -1 3
 For purposes herein, a "moderately conservative" replacement is any change having a nonnegative value in the PAM250 log-likelihood matrix reproduced herein above.

As is also well known in the art, relatedness of nucleic acids can also be characterized using a functional test, the ability of the two nucleic acids to base-pair to one another at defined hybridization stringencies.

It is, therefore, another aspect of the invention to provide isolated nucleic acids not only identical in sequence to those described with particularity herein, but also to provide isolated nucleic acids ("cross-hybridizing nucleic acids") that hybridize under high stringency conditions (as defined herein below) to all or to a portion of various of the isolated ZZAP1 nucleic acids of the present invention ("reference nucleic acids"), as well as cross-hybridizing nucleic acids that hybridize under moderate stringency conditions to all or to a portion of various of the isolated ZZAP1 nucleic acids of the present invention.

Such cross-hybridizing nucleic acids are useful, *inter alia,* as probes for, and to drive expression of, proteins related to the proteins of the present invention as alternative isoforms, homologues, paralogues, and orthologues. Particularly useful orthologues are those from other primate species, such as chimpanzee, rhesus macaque, monkey, baboon, orangutan, and gorilla; from rodents, such as rats, mice, guinea pigs; from lagomorphs, such as rabbits; and from domestic livestock, such as cow, pig, sheep, horse, goat and chicken.

For purposes herein, high stringency conditions are defined as aqueous hybridization *(i.e.,* free of formamide) in 6X SSC (where 20X SSC contains 3.0 M NaCl and 0.3 M sodium citrate), 1% SDS at 65°C for at least 8 hours, followed by one or more washes in 0.2X SSC, 0.1% SDS at 65°C. For purposes herein, moderate stringency conditions are defined as aqueous hybridization *(i.e.,* free of formamide) in 6X SSC, 1% SDS at 65°C for at least 8 hours, followed by one or more washes in 2x SSC, 0.1% SDS at room temperature.

The hybridizing portion of the reference nucleic acid is typically at least 15 nucleotides in length, often at least 17 nucleotides in length. Often, however, the hybridizing portion of the reference nucleic acid is at least 20 nucleotides in length, 25 nucleotides in length, and even 30 nucleotides, 35 nucleotides, 40 nucleotides, and 50 nucleotides in length. Of course, cross-hybridizing nucleic acids that hybridize to a larger portion of the reference nucleic acid - for example, to a portion of at least 50 nt, at least 100 nt, at least 150 nt, 200 nt, 250 nt, 300 nt, 350 nt, 400 nt, 450 nt, or 500 nt or more - or even to the entire length of the reference nucleic acid, are also useful.

The hybridizing portion of the cross-hybridizing nucleic acid is at least 75% identical in sequence to at least a portion of the reference nucleic acid. Typically, the hybridizing portion of the cross-hybridizing nucleic acid is at least 80%, often at least 85%, 86%, 87%, 88%, 89% or even at least 90% identical in sequence to at least a portion of the reference nucleic acid. Often, the hybridizing portion of the cross-hybridizing nucleic acid will be at least 91%, 92%, 93%, 94%, 95%, 96%, 97%, 98%, or 99% identical in sequence to at least a portion of the reference nucleic acid sequence. At times, the hybridizing portion of the cross-hybridizing nucleic acid will be at least 99.5% identical in sequence to at least a portion of the reference nucleic acid.

The invention also provides fragments of various of the isolated nucleic acids of the present invention.

By "fragments" of a reference nucleic acid is here intended isolated nucleic acids, however obtained, that have a nucleotide sequence identical to a portion of the reference nucleic acid sequence, which portion is at least 17 nucleotides and less than the entirety of the reference nucleic acid. As so defined, "fragments" need not be obtained by physical fragmentation of the reference nucleic acid, although such provenance is not thereby precluded.

In theory, an oligonucleotide of 17 nucleotides is of sufficient length as to occur at random less frequently than once in the three gigabase human genome, and thus to provide a nucleic acid probe that can uniquely identify the reference sequence in a nucleic acid mixture of genomic complexity. As is well known, further specificity can be obtained by probing nucleic acid samples of subgenomic complexity, and/or by using plural fragments as short as 17 nucleotides in length collectively to prime amplification of nucleic acids, as, *e.g*., by polymerase chain reaction (PCR).

As further described herein below, nucleic acid fragments that encode at least 6 contiguous amino acids (*i.e.*, fragments of 18 nucleotides or more) are useful in directing the expression or the synthesis of peptides that have utility in mapping the epitopes of the protein encoded by the reference nucleic acid. *See, e.g.,* Geysen *et al.,* "Use of peptide synthesis to probe viral antigens for epitopes to a resolution of a single amino acid," *Proc*. *Natl*. *Acad*. *Sci*. *USA* 81:3998-4002 (1984); and U.S. Pat. Nos. 4,708,871 and 5,595,915, the disclosures of which are incorporated herein by reference in their entireties.

As further described herein below, fragments that encode at least 8 contiguous amino acids (*i.e.*, fragments of 24 nucleotides or more) are useful in directing the expression or the synthesis of peptides that have utility as immunogens. *See, e.g.*, Lerner, "Tapping the immunological repertoire to produce antibodies of predetermined specificity," Nature 299:592-596 (1982); Shinnick *et al.,* "Synthetic peptide immunogens as vaccines," *Annu*. *Rev*. *Microbiol*. 37:425-46 (1983); Sutcliffe et *al*., "Antibodies that react with predetermined sites on proteins," *Science* 219:660-6 (1983), the disclosures of which are incorporated herein by reference in their entireties.

The nucleic acid fragment of the present invention is thus at least 17 nucleotides in length, typically at least 18 nucleotides in length, and often at least 24 nucleotides in length. Often, the nucleic acid of the present invention is at least 25 nucleotides in length, and even 30 nucleotides, 35 nucleotides, 40 nucleotides, or 45 nucleotides in length. Of course, larger fragments having at least 50 nt, at least 100 nt, at least 150 nt, 200 nt, 250 nt, 300 nt, 350 nt, 400 nt, 450 nt, or 500 nt or more are also useful, and at times preferred.

Having been based upon the mining of genomic sequence, rather than upon surveillance of expressed message, the present invention further provides isolated genome-derived nucleic acids that include portions of the ZZAP1 gene.

The invention particularly provides genome-derived single exon probes.

As further described in commonly owned and copending U.S. patent application serial nos. 09/864,761, filed May 23, 2001; 09/774,203, filed January 29, 2001; and 09/632,366, filed August 3, 2000, the disclosures of which are incorporated herein by reference in their entireties, "a single exon probe" comprises at least part of an exon ("reference exon") and can hybridize detectably under high stringency conditions to transcript-derived nucleic acids that include the reference exon. The single exon probe will not, however, hybridize detectably under high stringency conditions to nucleic acids that lack the reference exon and instead consist of one or more exons that are found adjacent to the reference exon in the genome.

Genome-derived single exon probes typically further comprise, contiguous to a first end of the exon portion, a first intronic and/or intergenic sequence that is identically contiguous to the exon in the genome. Often, the genome-derived single exon probe further comprises, contiguous to a second end of the exonic portion, a second intronic and/or intergenic sequence that is identically contiguous to the exon in the genome.

The minimum length of genome-derived single exon probes is defined by the requirement that the exonic portion be of sufficient length to hybridize under high stringency conditions to transcript-derived nucleic acids. Accordingly, the exon portion is at least 17 nucleotides, typically at least 18 nucleotides, 20 nucleotides, 24 nucleotides, 25 nucleotides or even 30, 35, 40, 45, or 50 nucleotides in length, and can usefully include the entirety of the exon, up to 100 nt, 150 nt, 200 nt, 250 nt, 300 nt, 350 nt, 400 nt or even 500 nt or more in length.

The maximum length of genome-derived single exon probes is defined by the requirement that the probes contain portions of no more than one exon, that is, be unable to hybridize detectably under high stringency conditions to nucleic acids that lack the reference exon but include one or more exons that are found adjacent to the reference exon the genome.

Given variable spacing of exons through eukaryotic genomes, the maximum length of single exon probes of the present invention is typically no more than 25 kb, often no more than 20 kb, 15 kb, 10 kb or 7.5 kb, or even no more than 5 kb, 4 kb, 3 kb, or even no more than about 2.5 kb in length.

The genome-derived single exon probes of the present invention can usefully include at least a first terminal priming sequence not found in contiguity with the rest of the probe sequence in the genome, and often will contain a second terminal priming sequence not found in contiguity with the rest of the probe sequence in the genome.

The present invention also provides isolated genome-derived nucleic acids that include nucleic acid sequence elements that control transcription of the ZZAP1 gene.

With a complete draft of the human genome now available, genomic sequences that are within the vicinity of the ZZAP1 coding region (and that are additional to those described with particularity herein) can readily be obtained by PCR amplification.

The isolated nucleic acids of the present invention can be composed of natural nucleotides in native 5'-3' phosphodiester internucleoside linkage - e.g., DNA or RNA - or can contain any or all of nonnatural nucleotide analogues, nonnative internucleoside bonds, or post-synthesis modifications, either throughout the length of the nucleic acid or localized to one or more portions thereof.

As is well known in the art, when the isolated nucleic acid is used as a hybridization probe, the range of such nonnatural analogues, nonnative internucleoside bonds, or post-synthesis modifications will be limited to those that permit sequence-discriminating basepairing of the resulting nucleic acid. When used to direct expression or RNA or protein *in vitro* or *in vivo,* the range of such nonnatural analogues, nonnative internucleoside bonds, or post-synthesis modifications will be limited to those that permit the nucleic acid to function properly as a polymerization substrate. When the isolated nucleic acid is used as a therapeutic agent, the range of such changes will be limited to those that do not confer toxicity upon the isolated nucleic acid.

For example, when desired to be used as probes, the isolated nucleic acids of the present invention can usefully include nucleotide analogues that incorporate labels that are directly detectable, such as radiolabels or fluorophores, or nucleotide analogues that incorporate labels that can be visualized in a subsequent reaction, such as biotin or various haptens.

Common radiolabeled analogues include those labeled with ³³P, ³²P, and ³⁵S, such as α-³²P-dATP, α-³²P-dCTP, α-³²P-dGTP, α-³²P-dTTP, α-³²P-3'dATP, α-³²P-ATP, α-³²P-CTP, α-³²P-GTP, α-³²P-UTP, α-³⁵S-dATP, γ-³⁵S-GTP, γ-³³P-dATP, and the like.

Commercially available fluorescent nucleotide analogues readily incorporated into the nucleic acids of the present invention include Cy3-dCTP, Cy3-dUTP, Cy5-dCTP, Cy3-dUTP (Amersham Pharmacia Biotech, Piscataway, New Jersey, USA), fluorescein-12-dUTP, tetramethylrhodamine-6-dUTP, Texas Red®-5-dUTP, Cascade Blue®-7-dUTP, BODIPY® FL-14-dUTP, BODIPY® TMR-14-dUTP, BODIPY® TR-14-dUTP, Rhodamine Green™-5-dUTP, Oregon Green® 488-5-dUTP, Texas Red®-12-dUTP, BODIPY® 630/650-14-dUTP, BODIPY® 650/665-14-dUTP, Alexa Fluor® 488-5-dUTP, Alexa Fluor® 532-5-dUTP, Alexa Fluor® 568-5-dUTP, Alexa Fluor® 594-5-dUTP, Alexa Fluor® 546-14-dUTP, fluorescein-12-UTP, tetramethylrhodamine-6-UTP, Texas Red®-5-UTP, Cascade Blue®-7-UTP, BODIPY® FL-14-UTP, BODIPY® TMR-14-UTP, BODIPY® TR-14-UTP, Rhodamine Green™-5-UTP, Alexa Fluor® 488-5-UTP, Alexa Fluor® 546-14-UTP (Molecular Probes, Inc. Eugene, OR, USA).

Protocols are available for custom synthesis of nucleotides having other fluorophores. Henegariu *et al.,* "Custom Fluorescent-Nucleotide Synthesis as an Alternative Method for Nucleic Acid Labeling," *Nature Biotechnol*. 18:345 - 348 (2000), the disclosure of which is incorporated herein by reference in its entirety.

Haptens that are commonly conjugated to nucleotides for subsequent labeling include biotin (biotin-11-dUTP, Molecular Probes, Inc., Eugene, OR, USA; biotin-21-UTP, biotin-21-dUTP, Clontech Laboratories, Inc., Palo Alto, CA, USA), digoxigenin (DIG-11-dUTP, alkali labile, DIG-11-UTP, Roche Diagnostics Corp., Indianapolis, IN, USA), and dinitrophenyl (dinitrophenyl-11-dUTP, Molecular Probes, Inc., Eugene, OR, USA).

As another example, when desired to be used for antisense inhibition of transcription or translation, the isolated nucleic acids of the present invention can usefully include altered, often nuclease-resistant, internucleoside bonds. *See* Hartmann *et al.* (eds.), Manual of Antisense Methodology (Perspectives in Antisense Science), Kluwer Law International (1999) (ISBN:079238539X); Stein et *al.* (eds.), Applied Antisense Oligonucleotide Technology, Wiley-Liss (cover (1998) (ISBN: 0471172790); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties. Such altered internucloside bonds are often desired also when the isolated nucleic acid of the present invention is to be used for or for targeted gene correction, Gamper *et al*., *Nucl*. *Acids Res.* 28(21):4332-4339 (2000), the disclosures of which are incorporated herein by reference in its entirety.

Modified oligonucleotide backbones often preferred when the nucleic acid is to be used for antisense purposes are, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Representative U.S. patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, the disclosures of which are incorporated herein by reference in their entireties.

Preferred modified oligonucleotide backbones for antisense use that do not include a phosphorus atom have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative U.S. patents that teach the preparation of the above backbones include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, the disclosures of which are incorporated herein by reference in their entireties.

In other preferred oligonucleotide mimetics, both the sugar and the internucleoside linkage are replaced with novel groups, such as peptide nucleic acids (PNA).

In PNA compounds, the phosphodiester backbone of the nucleic acid is replaced with an amide-containing backbone, in particular by repeating N-(2-aminoethyl) glycine units linked by amide bonds. Nucleobases are bound directly or indirectly to aza nitrogen atoms of the amide portion of the backbone, typically by methylene carbonyl linkages.

The uncharged nature of the PNA backbone provides PNA/DNA and PNA/RNA duplexes with a higher thermal stability than is found in DNA/DNA and DNA/RNA duplexes, resulting from the lack of charge repulsion between the PNA and DNA or RNA strand. In general, the Tm of a PNA/DNA or PNA/RNA duplex is 1°C higher per base pair than the Tm of the corresponding DNA/DNA or DNA/RNA duplex (in 100 mM NaCl).

The neutral backbone also allows PNA to form stable DNA duplexes largely independent of salt concentration. At low ionic strength, PNA can be hybridized to a target sequence at temperatures that make DNA hybridization problematic or impossible. And unlike DNA/DNA duplex formation, PNA hybridization is possible in the absence of magnesium. Adjusting the ionic strength, therefore, is useful if competing DNA or RNA is present in the sample, or if the nucleic acid being probed contains a high level of secondary structure.

PNA also demonstrates greater specificity in binding to complementary DNA. A PNA/DNA mismatch is more destabilizing than DNA/DNA mismatch. A single mismatch in mixed a PNA/DNA 15-mer lowers the Tm by 8-20°C (15°C on average). In the corresponding DNA/DNA duplexes, a single mismatch lowers the Tm by 4-16°C (11°C on average). Because PNA probes can be significantly shorter than DNA probes, their specificity is greater.

Additionally, nucleases and proteases do not recognize the PNA polyamide backbone with nucleobase sidechains. As a result, PNA oligomers are resistant to degradation by enzymes, and the lifetime of these compounds is extended both *in vivo* and *in vitro.* In addition, PNA is stable over a wide pH range.

Because its backbone is formed from amide bonds, PNA can be synthesized using a modified peptide synthesis protocol. PNA oligomers can be synthesized by both Fmoc and tBoc methods. Representative U.S. patents that teach the preparation of PNA compounds include, but are not limited to, U.S. Pat. Nos. 5,539,082; 5,714,331; and 5,719,262, each of which is herein incorporated by reference; automated PNA synthesis is readily achievable on commercial synthesizers *(see, e.g.,* "PNA User's Guide," Rev. 2, February 1998, Perseptive Biosystems Part No. 60138, Applied Biosystems, Inc., Foster City, CA).

PNA chemistry and applications are reviewed, *inter alia,* in Ray *et al*., *FASEB J.* 14(9):1041-60 (2000); Nielsen *et al*., *Pharmacol Toxicol*. 86(1):3-7 (2000); Larsen *et al*., *Biochim Biophys Acta.* 1489(1):159-66 (1999); Nielsen, *Curr*. *Opin*. *Struct. Biol.* 9(3):353-7 (1999), and Nielsen, *Curr*. *Opin*. *Biotechnol*. 10(1):71-5 (1999), the disclosures of which are incorporated herein by reference in their entireties.

Differences from nucleic acid compositions found in nature - *e.g.,* nonnative bases, altered internucleoside linkages, post-synthesis modification - can be present throughout the length of the nucleic acid or can, instead, usefully be localized to discrete portions thereof. As an example of the latter, chimeric nucleic acids can be synthesized that have discrete DNA and RNA domains and demonstrated utility for targeted gene repair, as further described in U.S. Pat. Nos. 5,760,012 and 5,731,181, the disclosures of which are incorporated herein by reference in their entireties. As another example, chimeric nucleic acids comprising both DNA and PNA have been demonstrated to have utility in modified PCR reactions. *See* Misra *et al., Biochem*. 37: 1917-1925 (1998); *see also* Finn *et al*., *Nucl*. *Acids Res*. 24: 3357-3363 (1996), incorporated herein by reference.

Unless otherwise specified, nucleic acids of the present invention can include any topological conformation appropriate to the desired use; the term thus explicitly comprehends, among others, single-stranded, double-stranded, triplexed, quadruplexed, partially double-stranded, partially-triplexed, partially-quadruplexed, branched, hairpinned, circular, and padlocked conformations. Padlock conformations and their utilities are further described in Banér *et al*., *Curr. Opin*. *Biotechnol*. 12:11-15 (2001); Escude et *al.,* Proc. Natl. Acad. Sci. USA 14;96(19):10603-7 (1999); Nilsson *et al*., *Science* 265(5181):2085-8 (1994), the disclosures of which are incorporated herein by reference in their entireties. Triplex and quadruplex conformations, and their utilities, are reviewed in Praseuth *et al*., *Biochim*. *Biophys*. *Acta*. 1489(1):181-206 (1999); Fox, *Curr*. *Med*. *Chem.* 7(1):17-37 (2000); Kochetkova *et al*., *Methods Mol*. *Biol*. 130:189-201 (2000); Chan *et al*., *J*. *Mol. Med.* 75(4):267-82 (1997), the disclosures of which are incorporated herein by reference in their entireties.

The nucleic acids of the present invention can be detectably labeled.

Commonly-used labels include radionuclides, such as ³²P, ³³P, ³⁵S, ³H (and for NMR detection, ¹³C and ¹⁵N), haptens that can be detected by specific antibody or high affinity binding partner (such as avidin), and fluorophores.

As noted above, detectable labels can be incorporated by inclusion of labeled nucleotide analogues in the nucleic acid.

Such analogues can be incorporated by enzymatic polymerization, such as by nick translation, random priming, polymerase chain reaction (PCR), terminal transferase tailing, and end-filling of overhangs, for DNA molecules, and *in vitro* transcription driven, *e.g.*, from phage promoters, such as T7, T3, and SP6, for RNA molecules. Commercial kits are readily available for each such labeling approach.

Analogues can also be incorporated during automated solid phase chemical synthesis.

As is well known, labels can also be incorporated after nucleic acid synthesis, with the 5' phosphate and 3' hydroxyl providing convenient sites for post-synthetic covalent attachment of detectable labels.

Various other post-synthetic approaches permit internal labeling of nucleic acids.

For example, fluorophores can be attached using a cisplatin reagent that reacts with the N7 of guanine residues (and, to a lesser extent, adenine bases) in DNA, RNA, and PNA to provide a stable coordination complex between the nucleic acid and fluorophore label (Universal Linkage System) (available from Molecular Probes, Inc., Eugene, OR, USA and Amersham Pharmacia Biotech, Piscataway, NJ, USA); *see Alers et al*., *Genes, Chromosomes & Cancer*, Vol. 25, pp. 301 - 305 (1999); Jelsma *et al*., *J*. *NIH Res.* 5:82 (1994); Van Belkum *et al*., *BioTechniques* 16:148-153 (1994), incorporated herein by reference. As another example, nucleic acids can be labeled using a disulfide-containing linker (FastTag™ Reagent, Vector Laboratories, Inc., Burlingame, CA, USA) that is photo- or thermally coupled to the target nucleic acid using aryl azide chemistry; after reduction, a free thiol is available for coupling to a hapten, fluorophore, sugar, affinity ligand, or other marker.

Multiple independent or interacting labels can be incorporated into the nucleic acids of the present invention.

For example, both a fluorophore and a moiety that in proximity thereto acts to quench fluorescence can be included to report specific hybridization through release of fluorescence quenching, Tyagi *et al*., *Nature Biotechnol*. 14: 303-308 (1996); Tyagi *et al., Nature Biotechnol*. 16, 49-53 (1998); Sokol *et al., Proc*. *Natl*. *Acad*. *Sci*. *USA* 95: 11538-11543 (1998); Kostrikis *et al*., *Science* 279:1228-1229 (1998); Marras *et al*., *Genet. Anal.* 14: 151-156 (1999); U.S. Pat. Nos. 5,846,726, 5,925,517, 5925517, or to report exonucleotidic excision, U.S. Pat. No. 5,538,848; Holland *et al*., *Proc*. *Natl*. *Acad*. *Sci*. *USA* 88:7276-7280 (1991); Heid *et al*., *Genome Res.* 6(10):986-94 (1996); Kuimelis *et al., Nucleic Acids Symp Ser.* (37):255-6 (1997); U.S. Patent No. 5,723,591, the disclosures of which are incorporated herein by reference in their entireties.

So labeled, the isolated nucleic acids of the present invention can be used as probes, as further described below.

Nucleic acids of the present invention can also usefully be bound to a substrate. The substrate can porous or solid, planar or non-planar, unitary or distributed; the bond can be covalent or noncovalent. Bound to a substrate, nucleic acids of the present invention can be used as probes in their unlabeled state.

For example, the nucleic acids of the present invention can usefully be bound to a porous substrate, commonly a membrane, typically comprising nitrocellulose, nylon, or positively-charged derivatized nylon; so attached, the nucleic acids of the present invention can be used to detect ZZAP1 nucleic acids present within a labeled nucleic acid sample, either a sample of genomic nucleic acids or a sample of transcript-derived nucleic acids, *e*.*g*. by reverse dot blot.

The nucleic acids of the present invention can also usefully be bound to a solid substrate, such as glass, although other solid materials, such as amorphous silicon, crystalline silicon, or plastics, can also be used. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof.

Typically, the solid substrate will be rectangular, although other shapes, particularly disks and even spheres, present certain advantages. Particularly advantageous alternatives to glass slides as support substrates for array of nucleic acids are optical discs, as described in Demers, "Spatially Addressable Combinatorial Chemical Arrays in CD-ROM Format," international patent publication WO 98/12559, incorporated herein by reference in its entirety.

The nucleic acids of the present invention can be attached covalently to a surface of the support substrate or applied to a derivatized surface in a chaotropic agent that facilitates denaturation and adherence by presumed noncovalent interactions, or some combination thereof.

The nucleic acids of the present invention can be bound to a substrate to which a plurality of other nucleic acids are concurrently bound, hybridization to each of the plurality of bound nucleic acids being separately detectable. At low density, *e.g.* on a porous membrane, these substrate-bound collections are typically denominated macroarrays; at higher density, typically on a solid support, such as glass, these substrate bound collections of plural nucleic acids are colloquially termed microarrays. As used herein, the term microarray includes arrays of all densities. It is, therefore, another aspect of the invention to provide microarrays that include the nucleic acids of the present invention.

The isolated nucleic acids of the present invention can be used as hybridization probes to detect, characterize, and quantify ZZAP1 nucleic acids in, and isolate ZZAP1 nucleic acids from, both genomic and transcript-derived nucleic acid samples. When free in solution, such probes are typically, but not invariably, detectably labeled; bound to a substrate, as in a microarray, such probes are typically, but not invariably unlabeled.

For example, the isolated nucleic acids of the present invention can be used as probes to detect and characterize gross alterations in the ZZAP1 genomic locus, such as deletions, insertions, translocations, and duplications of the ZZAP1 genomic locus through fluorescence *in situ* hybridization (FISH) to chromosome spreads. *See, e.g.,* Andreeff *et al.* (eds.), Introduction to Fluorescence *In Situ* Hybridization: Principles and Clinical Applications, John Wiley & Sons (1999) (ISBN: 0471013455), the disclosure of which is incorporated herein by reference in its entirety. The isolated nucleic acids of the present invention can be used as probes to assess smaller genomic alterations using, *e.g.,* Southern blot detection of restriction fragment length polymorphisms. The isolated nucleic acids of the present invention can be used as probes to isolate genomic clones that include the nucleic acids of the present invention, which thereafter can be restriction mapped and sequenced to identify deletions, insertions, translocations, and substitutions (single nucleotide polymorphisms, SNPs) at the sequence level.

The isolated nucleic acids of the present invention can also be used as probes to detect, characterize, and quantify ZZAP1 nucleic acids in, and isolate ZZAP1 nucleic acids from, transcript-derived nucleic acid samples.

For example, the isolated nucleic acids of the present invention can be used as hybridization probes to detect, characterize by length, and quantify ZZAP1 mRNA by northern blot of total or poly-A⁺- selected RNA samples. For example, the isolated nucleic acids of the present invention can be used as hybridization probes to detect, characterize by location, and quantify ZZAP1 message by *in situ* hybridization to tissue sections (see, *e.g.,* Schwarchzacher *et al.,* In Situ Hybridization, Springer-Verlag New York (2000) (ISBN: 0387915966), the disclosure of which is incorporated herein by reference in its entirety). For example, the isolated nucleic acids of the present invention can be used as hybridization probes to measure the representation of ZZAP1 clones in a cDNA library. For example, the isolated nucleic acids of the present invention can be used as hybridization probes to isolate ZZAP1 nucleic acids from cDNA libraries, permitting sequence level characterization of ZZAP1 messages, including identification of deletions, insertions, truncations - including deletions, insertions, and truncations of exons in alternatively spliced forms - and single nucleotide polymorphisms.

All of the aforementioned probe techniques are well within the skill in the art, and are described at greater length in standard texts such as Sambrook *et al.,* Molecular Cloning: A Laboratory Manual (3^{rd} ed.), Cold Spring Harbor Laboratory Press (2001) (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X); and Walker *et al.* (eds.), The Nucleic Acids Protocols Handbook, Humana Press (2000) (ISBN: 0896034593), the disclosures of which are incorporated herein by reference in their entirety.

As described in the Examples herein below, the nucleic acids of the present invention can also be used to detect and quantify ZZAP1 nucleic acids in transcript-derived samples - that is, to measure expression of the ZZAP1 gene - when included in a microarray. Measurement of ZZAP1 expression has particular utility, as ZZAP1 is potentially involved in protein-protein interaction, and aberrant expression of ZZAP1 is likely associated with the development of certain types of cancer (and other diseases, such as osteosclerosis or AIDS), as further described in the Examples herein below.

As would be readily apparent to one of skill in the art, each ZZAP1 nucleic acid probe - whether labeled, substrate-bound, or both - is thus currently available for use as a tool for measuring the level of ZZAP1 expression in each of the tissues in which expression has already been confirmed, notably fetal liver, placenta, brain, adult liver, lung, testis, skeletal muscle, heart, and bone marrow. The utility is specific to the probe: under high stringency conditions, the probe reports the level of expression of message specifically containing that portion of the ZZAP1 gene included within the probe.

Measuring tools are well known in many arts, not just in molecular biology, and are known to possess credible, specific, and substantial utility. For example, U.S. Patent No. 6,016,191 describes and claims a tool for measuring characteristics of fluid flow in a hydrocarbon well; U.S. Patent No. 6,042,549 describes and claims a device for measuring exercise intensity; U.S. Patent No. 5,889,351 describes and claims a device for measuring viscosity and for measuring characteristics of a fluid; U.S. Patent No. 5,570,694 describes and claims a device for measuring blood pressure; U.S. Patent No. 5,930,143 describes and claims a device for measuring the dimensions of machine tools; U.S. Patent No. 5,279,044 describes and claims a measuring device for determining an absolute position of a movable element; U.S. Patent No. 5,186,042 describes and claims a device for measuring action force of a wheel; and U.S. Patent No. 4,246,774 describes and claims a device for measuring the draft of smoking articles such as cigarettes.

As for tissues not yet demonstrated to express ZZAP1, the ZZAP1 nucleic acid probes of the present invention are currently available as tools for surveying such tissues to detect the presence of ZZAP1 nucleic acids.

Survey tools - *i*.*e*., tools for determining the presence and/or location of a desired object by search of an area - are well known in many arts, not just in molecular biology, and are known to possess credible, specific, and substantial utility. For example, U.S. Patent No. 6,046,800 describes and claims a device for surveying an area for objects that move; U.S. Patent No. 6,025,201 describes and claims an apparatus for locating and discriminating platelets from non-platelet particles or cells on a cell-by-cell basis in a whole blood sample; U.S. Patent No. 5,990,689 describes and claims a device for detecting and locating anomalies in the electromagnetic protection of a system; U.S. Patent No. 5,984,175 describes and claims a device for detecting and identifying wearable user identification units; U.S. Patent No. 3,980,986 ("Oil well survey tool"), describes and claims a tool for finding the position of a drill bit working at the bottom of a borehole.

As noted above, the nucleic acid probes of the present invention are useful in constructing microarrays; the microarrays, in turn, are products of manufacture that are useful for measuring and for surveying gene expression.

When included on a microarray, each ZZAP1 nucleic acid probe makes the microarray specifically useful for detecting that portion of the ZZAP1 gene included within the probe, thus imparting upon the microarray device the ability to detect a signal where, absent such probe, it would have reported no signal. This utility makes each individual probe on such microarray akin to an antenna, circuit, firmware or software element included in an electronic apparatus, where the antenna, circuit, firmware or software element imparts upon the apparatus the ability newly and additionally to detect signal in a portion of the radio-frequency spectrum where previously it could not; such devices are known to have specific, substantial, and credible utility.

Changes in the level of expression need not be observed for the measurement of expression to have utility.

For example, where gene expression analysis is used to assess toxicity of chemical agents on cells, the failure of the agent to change a gene's expression level is evidence that the drug likely does not affect the pathway of which the gene's expressed protein is a part. Analogously, where gene expression analysis is used to assess side effects of pharmacologic agents - whether in lead compound discovery or in subsequent screening of lead compound derivatives - the inability of the agent to alter a gene's expression level is evidence that the drug does not affect the pathway of which the gene's expressed protein is a part.

WO 99/58720, incorporated herein by reference in its entirety, provides methods for quantifying the relatedness of a first and second gene expression profile and for ordering the relatedness of a plurality of gene expression profiles, without regard to the identity or function of the genes whose expression is used in the calculation.

Gene expression analysis, including gene expression analysis by microarray hybridization, is, of course, principally a laboratory-based art. Devices and apparatus used principally in laboratories to facilitate laboratory research are well-established to possess specific, substantial, and credible utility. For example, U.S. Patent No. 6,001,233 describes and claims a gel electrophoresis apparatus having a cam-activated clamp; for example, U.S. Patent No. 6,051,831 describes and claims a high mass detector for use in time-of-flight mass spectrometers; for example, U.S. Patent NO. 5,824,269 describes and claims a flow cytometer-as is well known, few gel electrophoresis apparatuses, TOF-MS devices, or flow cytometers are sold for consumer use.

Indeed, and in particular, nucleic acid microarrays, as devices intended for laboratory use in measuring gene expression, are well-established to have specific, substantial and credible utility. Thus, the microarrays of the present invention have at least the specific, substantial and credible utilities of the microarrays claimed as devices and articles of manufacture in the following U.S. patents, the disclosures of each of which is incorporated herein by reference: U.S. Patent Nos. 5,445,934 ("Array of oligonucleotides on a solid substrate"); 5,744,305 ("Arrays of materials attached to a substrate"); and 6,004,752 ("Solid support with attached molecules").

Genome-derived single exon probes and genome-derived single exon probe microarrays have the additional utility, *inter alia*, of permitting high-throughput detection of splice variants of the nucleic acids of the present invention, as further described in copending and commonly owned U.S. Patent application no. 09/632,366, filed August 3, 2000, the disclosure of which is incorporated herein by reference in its entirety.

The isolated nucleic acids of the present invention can also be used to prime synthesis of nucleic acid, for purpose of either analysis or isolation, using mRNA, cDNA, or genomic DNA as template.

For use as primers, at least 17 contiguous nucleotides of the isolated nucleic acids of the present invention will be used. Often, at least 18, 19, or 20 contiguous nucleotides of the nucleic acids of the present invention will be used, and on occasion at least 20, 22, 24, or 25 contiguous nucleotides of the nucleic acids of the present invention will be used, and even 30 nucleotides or more of the nucleic acids of the present invention can be used to prime specific synthesis.

The nucleic acid primers of the present invention can be used, for example, to prime first strand cDNA synthesis on an mRNA template.

Such primer extension can be done directly to analyze the message. Alternatively, synthesis on an mRNA template can be done to produce first strand cDNA. The first strand cDNA can thereafter be used, *inter alia,* directly as a single-stranded probe, as above-described, as a template for sequencing - permitting identification of alterations, including deletions, insertions, and substitutions, both normal allelic variants and mutations associated with abnormal phenotypes- or as a template, either for second strand cDNA synthesis *(e.g.,* as an antecedent to insertion into a cloning or expression vector), or for amplification.

The nucleic acid primers of the present invention can also be used, for example, to prime single base extension (SBE) for SNP detection (see, *e.g.,* U.S. Pat. No. 6,004,744, the disclosure of which is incorporated herein by reference in its entirety).

As another example, the nucleic acid primers of the present invention can be used to prime amplification of ZZAP1 nucleic acids, using transcript-derived or genomic DNA as template.

Primer-directed amplification methods are now well-established in the art. Methods for performing the polymerase chain reaction (PCR) are compiled, *inter alia,* in McPherson, PCR (Basics: From Background to Bench), Springer Verlag (2000) (ISBN: 0387916008); Innis *et al.* (eds.), PCR Applications: Protocols for Functional Genomics, Academic Press (1999) (ISBN: 0123721857); Gelfand *et al.* (eds.), PCR Strategies, Academic Press (1998) (ISBN: 0123721822); Newton *et al.,* PCR, Springer-Verlag New York (1997) (ISBN: 0387915060); Burke (ed.), PCR: Essential Techniques, John Wiley & Son Ltd (1996) (ISBN: 047195697X); White (ed.), PCR Cloning Protocols: From Molecular Cloning to Genetic Engineering, Vol. 67, Humana Press (1996) (ISBN: 0896033430); McPherson et al. (eds.), PCR 2: A Practical Approach, Oxford University Press, Inc. (1995) (ISBN: 0199634254), the disclosures of which are incorporated herein by reference in their entireties. Methods for performing RT-PCR are collected, *e.g.,* in Siebert *et al.* (eds.), Gene Cloning and Analysis by RT-PCR, Eaton Publishing Company/Bio Techniques Books Division, 1998 (ISBN: 1881299147); Siebert (ed.), PCR Technique:RT-PCR, Eaton Publishing Company/BioTechniques Books (1995) (ISBN:1881299139), the disclosure of which is incorporated herein by reference in its entirety.

Isothermal amplification approaches, such as rolling circle amplification, are also now well-described. *See, e.g.,* Schweitzer *et al*., *Curr*. *Opin*. *Biotechnol*. 12(1):21-7 (2001); U.S. Patent Nos. 6,235,502, 6,221,603, 6,210,884, 6,183,960, 5,854,033, 5,714,320, 5,648,245, and international patent publications WO 97/19193 and WO 00/15779, the disclosures of which are incorporated herein by reference in their entireties. Rolling circle amplification can be combined with other techniques to facilitate SNP detection. See, *e.g.,* Lizardi *et al*., *Nature Genet*. 19(3):225-32 (1998).

As further described below, nucleic acids of the present invention, inserted into vectors that flank the nucleic acid insert with a phage promoter, such as T7, T3, or SP6 promoter, can be used to drive *in vitro* expression of RNA complementary to either strand of the nucleic acid of the present invention. The RNA can be used, *inter alia,* as a single-stranded probe, in cDNA-mRNA subtraction, or for *in vitro* translation.

As will be further discussed herein below, nucleic acids of the present invention that encode ZZAP1 protein or portions thereof can be used, *inter alia,* to express the ZZAP1 proteins or protein fragments, either alone, or as part of fusion proteins.

Expression can be from genomic nucleic acids of the present invention, or from transcript-derived nucleic acids of the present invention.

Where protein expression is effected from genomic DNA, expression will typically be effected in eukaryotic, typically mammalian, cells capable of splicing introns from the initial RNA transcript. Expression can be driven from episomal vectors, such as EBV-based vectors, or can be effected from genomic DNA integrated into a host cell chromosome. As will be more fully described below, where expression is from transcript-derived (or otherwise intron-less) nucleic acids of the present invention, expression can be effected in wide variety of prokaryotic or eukaryotic cells.

Expressed *in vitro,* the protein, protein fragment, or protein fusion can thereafter be isolated, to be used, *inter alia*, as a standard in immunoassays specific for the proteins, or protein isoforms, of the present invention; to be used as a therapeutic agent, *e.g.,* to be administered as passive replacement therapy in individuals deficient in the proteins of the present invention, or to be administered as a vaccine; to be used for *in vitro* production of specific antibody, the antibody thereafter to be used, *e.g.,* as an analytical reagent for detection and quantitation of the proteins of the present invention or to be used as an immunotherapeutic agent.

The isolated nucleic acids of the present invention can also be used to drive *in vivo* expression of the proteins of the present invention. *In vivo* expression can be driven from a vector - typically a viral vector, often a vector based upon a replication incompetent retrovirus, an adenovirus, or an adeno-associated virus (AAV) - for purpose of gene therapy. *In vivo* expression can also be driven from signals endogenous to the nucleic acid or from a vector, often a plasmid vector, such as pVAX1 (Invitrogen, Carlsbad CA, USA), for purpose of "naked" nucleic acid vaccination, as further described in U.S. Pat. Nos. 5,589,466; 5,679,647; 5,804,566; 5,830,877; 5,843,913; 5,880,104; 5,958,891; 5,985,847; 6,017,897; 6,110,898; 6,204,250, the disclosures of which are incorporated herein by reference in their entireties.

The nucleic acids of the present invention can also be used for antisense inhibition of transcription or translation. *See* Phillips (ed.), Antisense Technology, Part B, Methods in Enzymology Vol. 314, Academic Press, Inc. (1999) (ISBN: 012182215X); Phillips (ed.), Antisense Technology, Part A, Methods in Enzymology Vol. 313, Academic Press, Inc. (1999) (ISBN: 0121822141); Hartmann *et al.* (eds.), Manual of Antisense Methodology (Perspectives in Antisense Science), Kluwer Law International (1999) (ISBN:079238539X); Stein *et al.* (eds.), Applied Antisense Oligonucleotide Technology, Wiley-Liss (cover (1998) (ISBN: 0471172790); Agrawal *et al.* (eds.), Antisense Research and Application, Springer-Verlag New York, Inc. (1998) (ISBN: 3540638334); Lichtenstein *et al.* (eds.), Antisense Technology: A Practical Approach, Vol. 185, Oxford University Press, INC. (1998) (ISBN: 0199635838); Gibson (ed.), Antisense and Ribozyme Methodology: Laboratory Companion, Chapman & Hall (1997) (ISBN: 3826100794); Chadwick *et al.* (eds.), Oligonucleotides as Therapeutic Agents - Symposium No. 209, John Wiley & Son Ltd (1997) (ISBN: 0471972797), the disclosures of which are incorporated herein by reference in their entireties.

Nucleic acids of the present invention, particularly cDNAs of the present invention, that encode full-length human ZZAP1 protein isoforms, have additional, well-recognized, immediate, real world utility as commercial products of manufacture suitable for sale.

For example, Invitrogen Corp. (Carlsbad, CA, USA), through its Research Genetics subsidiary, sells full length human cDNAs cloned into one of a selection of expression vectors as GeneStorm® expression-ready clones; utility is specific for the gene, since each gene is capable of being ordered separately and has a distinct catalogue number, and utility is substantial, each clone selling for $650.00 US. Similarly, Incyte Genomics (Palo Alto, CA, USA) sells clones from public and proprietary sources in multi-well plates or individual tubes.

Nucleic acids of the present invention that include genomic regions encoding the human ZZAP1 protein, or portions thereof, have yet further utilities.

For example, genomic nucleic acids of the present invention can be used as amplification substrates, e.g. for preparation of genome-derived single exon probes of the present invention, as described above and in copending and commonly-owned U.S. patent application nos. 09/864,761, filed May 23, 2001, 09/774,203, filed January 29, 2001, and 09/632,366, filed August 3, 2000, the disclosures of which are incorporated herein by reference in their entireties.

As another example, genomic nucleic acids of the present invention can be integrated non-homologously into the genome of somatic cells, *e.g.* CHO cells, COS cells, or 293 cells, with or without amplification of the insertional locus, in order, *e.g.,* to create stable cell lines capable of producing the proteins of the present invention.

As another example, more fully described herein below, genomic nucleic acids of the present invention can be integrated nonhomologously into embryonic stem (ES) cells to create transgenic non-human animals capable of producing the proteins of the present invention.

Genomic nucleic acids of the present invention can also be used to target homologous recombination to the human ZZAP1 locus. *See, e.g.,* U.S. Patent Nos. 6,187,305; 6,204,061; 5,631,153; 5,627,059; 5,487,992; 5,464,764; 5,614,396; 5,527,695 and 6,063,630; and Kmiec *et al.* (eds.), Gene Targeting Protocols, Vol. 133, Humana Press (2000) (ISBN: 0896033600); Joyner (ed.), Gene Targeting: A Practical Approach, Oxford University Press, Inc. (2000) (ISBN: 0199637938); Sedivy *et al.,* Gene Targeting, Oxford University Press (1998) (ISBN: 071677013X); Tymms *et al.* (eds.), Gene Knockout Protocols, Humana Press (2000) (ISBN: 0896035727); Mak *et al.* (eds.), The Gene Knockout FactsBook, Vol. 2, Academic Press, Inc. (1998) (ISBN: 0124660444); Torres *et al.,* Laboratory Protocols for Conditional Gene Targeting, Oxford University Press (1997) (ISBN: 019963677X); Vega (ed.), Gene Targeting, CRC Press, LLC (1994) (ISBN: 084938950X), the disclosures of which are incorporated herein by reference in their entireties.

Where the genomic region includes transcription regulatory elements, homologous recombination can be used to alter the expression of ZZAP1, both for purpose of *in vitro* production of ZZAP1 protein from human cells, and for purpose of gene therapy. *See, e.g.,* U.S. Pat. Nos. 5,981,214, 6,048,524; 5,272,071.

Fragments of the nucleic acids of the present invention smaller than those typically used for homologous recombination can also be used for targeted gene correction or alteration, possibly by cellular mechanisms different from those engaged during homologous recombination.

For example, partially duplexed RNA/DNA chimeras have been shown to have utility in targeted gene correction, U.S. Pat. Nos. 5,945,339, 5,888,983, 5,871,984, 5,795,972, 5,780,296, 5,760,012, 5,756,325, 5,731,181, the disclosures of which are incorporated herein by reference in their entireties. So too have small oligonucleotides fused to triplexing domains have been shown to have utility in targeted gene correction, Culver *et al.,* "Correction of chromosomal point mutations in human cells with bifunctional oligonucleotides," *Nature Biotechnol*. 17(10):989-93 (1999), as have oligonucleotides having modified terminal bases or modified terminal internucleoside bonds, Gamper *et al*., *Nucl. Acids Res.* 28(21):4332-9 (2000), the disclosures of which are incorporated herein by reference.

The isolated nucleic acids of the present invention can also be used to provide the initial substrate for recombinant engineering of ZZAP1 protein variants having desired phenotypic improvements. Such engineering includes, for example, site-directed mutagenesis, random mutagenesis with subsequent functional screening, and more elegant schemes for recombinant evolution of proteins, as are described, *inter alia,* in U.S. Pat. Nos. 6,180,406; 6,165,793; 6,117,679; and 6,096,548, the disclosures of which are incorporated herein by reference in their entireties.

Nucleic acids of the present invention can be obtained by using the labeled probes of the present invention to probe nucleic acid samples, such as genomic libraries, cDNA libraries, and mRNA samples, by standard techniques. Nucleic acids of the present invention can also be obtained by amplification, using the nucleic -acid primers of the present invention, as further demonstrated in Example 1, herein below. Nucleic acids of the present invention of fewer than about 100 nt can also be synthesized chemically, typically by solid phase synthesis using commercially available automated synthesizers.

### "Full Length" ZZAP1 Nucleic Acids

In a first series of nucleic acid embodiments, the invention provides isolated nucleic acids that encode the entirety of the ZZAP1 protein. As discussed above, the "full-length" nucleic acids of the present invention can be used, *inter alia,* to express full length ZZAP1 protein. The full-length nucleic acids can also be used as nucleic acid probes; used as probes, the isolated nucleic acids of these embodiments will hybridize to ZZAP1.

In a first such embodiment, the invention provides an isolated nucleic acid comprising (i) the nucleotide sequence of the nucleic acid of ATCC deposit PTA-3582, (ii) the nucleotide sequence of SEQ ID NO: 1, or (iii) the complement of (i) or (ii). The ATCC deposit has, and SEQ ID NO: 1 presents, the entire cDNA of ZZAP1, including the 5' untranslated (UT) region and 3' UT.

In a second embodiment, the invention provides an isolated nucleic acid comprising (i) the nucleotide sequence of SEQ ID NO: 2, (ii) a degenerate variant of the nucleotide sequence of SEQ ID NO: 2, or (iii) the complement of (i) or (ii). SEQ ID NO: 2 presents the open reading frame (ORF) from SEQ ID NO: 1.

In a third embodiment, the invention provides an isolated nucleic acid comprising (i) a nucleotide sequence that encodes a polypeptide with the amino acid sequence of SEQ ID NO: 3 or (ii) the complement of a nucleotide sequence that encodes a polypeptide with the amino acid sequence of SEQ ID NO: 3. SEQ ID NO: 3 provides the amino acid sequence of ZZAP1.

In a fourth embodiment, the invention provides an isolated nucleic acid having a nucleotide sequence that (i) encodes a polypeptide having the sequence of SEQ ID NO: 3, (ii) encodes a polypeptide having the sequence of SEQ ID NO: 3 with conservative amino acid substitutions, or (iii) that is the complement of (i) or (ii), where SEQ ID NO: 3 provides the amino acid sequence of ZZAP1.

### Selected Partial Nucleic Acids

In a second series of nucleic acid embodiments, the invention provides isolated nucleic acids that encode select portions of ZZAP1. As will be further discussed herein below, these "partial" nucleic acids can be used, *inter alia,* to express specific portions of the ZZAP1. These "partial" nucleic acids can also be used, *inter alia,* as nucleic probes.

In a first such embodiment, the invention provides an isolated nucleic acid comprising (i) the nucleotide sequence of SEQ ID NO: 4, (ii) a degenerate variant of SEQ ID NO: 4, or (iii) the complement of (i) or (ii), wherein the isolated nucleic acid is no more than about 100 kb in length, typically no more than about 75 kb in length, more typically no more than about 50 kb length. SEQ ID NO: 4 encodes a novel portion of ZZAP1. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 5 or (ii) the complement of a nucleotide sequence that encodes SEQ ID NO: 5, wherein the isolated nucleic acid is no more than about 100 kb in length, typically no more than about 75 kb in length, frequently no more than about 50 kb in length. SEQ ID NO: 5 is the amino acid sequence encoded by SEQ ID NO: 4. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 5, (ii) a nucleotide sequence that encodes SEQ ID NO: 5 with conservative substititions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising (i) the nucleotide sequence of SEQ ID NO: 6, (ii) a degenerate variant of SEQ ID NO: 6, or (iii) the complement of (i) or (ii), wherein the isolated nucleic acid is no more than about 100 kb in length, typically no more than about 75 kb in length, more typically no more than about 50 kb length. SEQ ID NO: 6 encodes a novel portion of ZZAP1. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 7 or (ii) the complement of a nucleotide sequence that encodes SEQ ID NO: 7, wherein the isolated nucleic acid is no more than about 100 kb in length, typically no more than about 75 kb in length, frequently no more than about 50 kb in length. SEQ ID NO: 7 is the amino acid sequence encoded by SEQ ID NO: 6. Often, the isolated nucleic acids of this embodiment are no more than about 25 kb in length, often no more than about 15 kb in length, and frequently no more than about 10 kb in length.

In another embodiment, the invention provides an isolated nucleic acid comprising (i) a nucleotide sequence that encodes SEQ ID NO: 7, (ii) a nucleotide sequence that encodes SEQ ID NO: 7 with conservative substititions, or (iii) the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Cross-Hybridizing Nucleic Acids

In another series of nucleic acid embodiments, the invention provides isolated nucleic acids that hybridize to various of the ZZAP1 nucleic acids of the present invention. These cross-hybridizing nucleic acids can be used, *inter alia,* as probes for, and to drive expression of, proteins that are related to ZZAP1 of the present invention as further isoforms, homologues, paralogues, or orthologues.

In a first such embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 4 or the complement of SEQ ID NO: 4, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 5, (ii) encodes a polypeptide having the sequence of SEQ ID NO: 5 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In another such embodiment, the invention provides an isolated nucleic acid comprising a sequence that hybridizes under high stringency conditions to a probe the nucleotide sequence of which consists of at least 17 nt, 18, 19, 20, 21, 22, 23, 24, 25, 30, 40, or 50 nt of SEQ ID NO: 6 or the complement of SEQ ID NO: 6, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a first such embodiment, the invention provides an isolated nucleic acid comprising a sequence that hybridizes under moderate stringency conditions to a probe the nucleotide sequence of which consists of at least 17 nt, 18, 19, 20, 21, 22, 23, 24, 25, 30, 40, or 50 nt of SEQ ID NO: 6 or the complement of SEQ ID NO: 6, wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising a sequence that hybridizes under high stringency conditions to a hybridization probe the nucleotide sequence of which (i) encodes a polypeptide having the sequence of SEQ ID NO: 7, (ii) encodes a polypeptide having the sequence of SEQ ID NO: 7 with conservative amino acid substitutions, or (iii) is the complement of (i) or (ii), wherein the isolated nucleic acid is no more than about 100 kb in length, typically no more than about 75 kb in length, and often no more than about 50 kb in length. Often, the isolated nucleic acids of this embodiment are no more than about 25 kb in length, often no more than about 15 kb in length, and frequently no more than about 10 kb in length.

### Particularly Useful Nucleic Acids

Particularly useful among the above-described nucleic acids are those that are expressed, or the complement of which are expressed, in fetal liver, placenta, brain, adult liver, lung, testis, skeletal muscle, heart and bone marrow.

Also particularly useful among the above-described nucleic acids are those that encode, or the complement of which encode, a polypeptide having protein-protein interaction activity.

Other particularly useful embodiments of the nucleic acids above-described are those that encode, or the complement of which encode, a polypeptide having V-ATPase domains.

### Nucleic Acid Fragments

In another series of nucleic acid embodiments, the invention provides fragments of various of the isolated nucleic acids of the present invention which prove useful, *inter alia,* as nucleic acid probes, as amplification primers, and to direct expression or synthesis of epitopic or immunogenic protein fragments.

In a first such embodiment, the invention provides an isolated nucleic acid comprising at least 17 nucleotides, 18 nucleotides, 20 nucleotides, 24 nucleotides, or 25 nucleotides of (i) SEQ ID NO: 4, (ii) a degenerate variant of SEQ ID NO: 4, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 5, or (ii) a nucleotide sequence that encodes a peptide of at least 15 contiguous amino acids of SEQ ID NO: 5, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 5 with conservative amino acid substitutions, (ii) a polypeptide having the sequence of at least 15 contiguous amino acids of SEQ ID NO: 5 with conservative amino acid substitutions, (iii) a polypeptide having the sequence of at least 8 contiguous amino acids of SEQ ID NO: 5 with moderately conservative substitutions, (iv) a polypeptide having the sequence of at last 15 congiuous amino acids of SEQ ID NO: 5 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In a further embodiment, the invention provides an isolated nucleic acid comprising at least 17 nucleotides, 18 nucleotides, 20 nucleotides, 24 nucleotides, or 25 nucleotides of (i) SEQ ID NO: 6, (ii) a degenerate variant of SEQ ID NO: 6, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising (i) a nucleotide sequence that encodes a peptide of at least 8 contiguous amino acids of SEQ ID NO: 7, or (ii) a nucleotide sequence that encodes a peptide of at least 15 contiguous amino acids of SEQ ID NO: 7, or (iii) the complement of (i) or (ii), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

The invention also provides an isolated nucleic acid comprising a nucleotide sequence that encodes (i) a polypeptide having the sequence of at least 8 contiguous amino acids of SEQ ID NO: 7 with conservative amino acid substitutions, (ii) a polypeptide having the sequence of at least 15 contiguous amino acids of SEQ ID NO: 7 with conservative amino acid substitutions, (iii) a polypeptide having the sequence of at least 8 contiguous amino acids of SEQ ID NO: 7 with moderately conservative substitutions, (iv) a polypeptide having the sequence of at last 15 congiuous amino acids of SEQ ID NO: 7 with moderately conservative substitutions, or (v) the complement of any of (i) - (iv), wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### Single Exon Probes

The invention further provides genome-derived single exon probes having portions of no more than one exon of the ZZAP1 gene. As further described in commonly owned and copending U.S. patent application serial no. 09/632,366, filed August 3, 2000 ("Methods and Apparatus for High Throughput Detection and Characterization of alternatively Spliced Genes"), the disclosure of which is incorporated herein by reference in its entirety, such single exon probes have particular utility in identifying and characterizing splice variants. In particular, such single exon probes are useful for identifying and discriminating the expression of distinct isoforms of ZZAP1.

In a first embodiment, the invention provides an isolated nucleic acid comprising a nucleotide sequence of no more than one portion of SEQ ID NOs: 8-23 or the complement of SEQ ID NOs: 8-23, wherein the portion comprises at least 17 contiguous nucleotides, 18 contiguous nucleotides, 20 contiguous nucleotides, 24 contiguous nucleotides, 25 contiguous nucleotides, or 50 contiguous nucleotides of any one of SEQ ID NOs: 8-23, or their complement. In a further embodiment, the exonic portion comprises the entirety of the referenced SEQ ID NO: or its complement.

In other embodiments, the invention provides isolated single exon probes having the nucleotide sequence of any one of SEQ ID NOs: 24-39.

### Transcription Control Nucleic Acids

In another aspect, the present invention provides genome-derived isolated nucleic acids that include nucleic acid sequence elements that control transcription of the ZZAP1 gene. These nucleic acids can be used, *inter alia,* to drive expression of heterologous coding regions in recombinant constructs, thus conferring upon such heterologous coding regions the expression pattern of the native ZZAP1 gene. These nucleic acids can also be used, conversely, to target heterologous transcription control elements to the ZZAP1 genomic locus, altering the expression pattern of the ZZAP1 gene itself.

In a first such embodiment, the invention provides an isolated nucleic acid comprising the nucleotide sequence of SEQ ID NO: 40 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

In another embodiment, the invention provides an isolated nucleic acid comprising at least 17, 18, 20, 24, or 25 nucleotides of the sequence of SEQ ID NO: 40 or its complement, wherein the isolated nucleic acid is no more than about 100 kb in length, typically no more than about 75 kb in length, more typically no more than about 50 kb in length. Often, the isolated nucleic acids of this embodiment are no more than about 25 kb in length, often no more than about 15 kb in length, and frequently no more than about 10 kb in length.

### VECTORS AND HOST CELLS

In another aspect, the present invention provides vectors that comprise one or more of the isolated nucleic acids of the present invention, and host cells in which such vectors have been introduced.

The vectors can be used, *inter alia,* for propagating the nucleic acids of the present invention in host cells (cloning vectors), for shuttling the nucleic acids of the present invention between host cells derived from disparate organisms (shuttle vectors), for inserting the nucleic acids of the present invention into host cell chromosomes (insertion vectors), for expressing sense or antisense RNA transcripts of the nucleic acids of the present invention *in vitro* or within a host cell, and for expressing polypeptides encoded by the nucleic acids of the present invention, alone or as fusions to heterologous polypeptides. Vectors of the present invention will often be suitable for several such uses.

Vectors are by now well-known in the art, and are described, *inter alia,* in Jones *et al.* (eds.), Vectors: Cloning Applications : Essential Techniques (Essential Techniques Series), John Wiley & Son Ltd 1998 (ISBN: 047196266X); Jones *et al.* (eds.), Vectors: Expression Systems : Essential Techniques (Essential Techniques Series), John Wiley & Son Ltd, 1998 (ISBN:0471962678); Gacesa *et al.,* Vectors: Essential Data, John Wiley & Sons, 1995 (ISBN: 0471948411); Cid-Arregui (eds.), Viral Vectors: Basic Science and Gene Therapy, Eaton Publishing Co., 2000 (ISBN: 188129935X); Sambrook *et al.,* Molecular Cloning: A Laboratory Manual (3^{rd} ed.), Cold Spring Harbor Laboratory Press, 2001 (ISBN: 0879695773); Ausubel *et al.* (eds.), Short Protocols in Molecular Biology: A Compendium of Methods from Current Protocols in Molecular Biology (4^{th} ed.), John Wiley & Sons, 1999 (ISBN: 047132938X), the disclosures of which are incorporated herein by reference in their entireties. Furthermore, an enormous variety of vectors are available commercially. Use of existing vectors and modifications thereof being well within the skill in the art, only basic features need be described here.

Typically, vectors are derived from virus, plasmid, prokaryotic or eukaryotic chromosomal elements, or some combination thereof, and include at least one origin of replication, at least one site for insertion of heterologous nucleic acid, typically in the form of a polylinker with multiple, tightly clustered, single cutting restriction sites, and at least one selectable marker, although some integrative vectors will lack an origin that is functional in the host to be chromosomally modified, and some vectors will lack selectable markers. Vectors of the present invention will further include at least one nucleic acid of the present invention inserted into the vector in at least one location.

Where present, the origin of replication and selectable markers are chosen based upon the desired host cell or host cells; the host cells, in turn, are selected based upon the desired application.

For example, prokaryotic cells, typically E. *coli,* are typically chosen for cloning. In such case, vector replication is predicated on the replication strategies of coliform-infecting phage - such as phage lambda, M13, T7, T3 and P1 - or on the replication origin of autonomously replicating episomes, notably the ColE1 plasmid and later derivatives, including pBR322 and the pUC series plasmids. Where *E. coli* is used as host, selectable markers are, analogously, chosen for selectivity in gram negative bacteria: *e.g.,* typical markers confer resistance to antibiotics, such as ampicillin, tetracycline, chloramphenicol, kanamycin, streptomycin, zeocin; auxotrophic markers can also be used.

As another example, yeast cells, typically *S. cerevisiae,* are chosen, *inter alia,* for eukaryotic genetic studies, due to the ease of targeting genetic changes by homologous recombination and to the ready ability to complement genetic defects using recombinantly expressed proteins, for identification of interacting protein components, *e.g.* through use of a two-hybrid system, and for protein expression. Vectors of the present invention for use in yeast will typically, but not invariably, contain an origin of replication suitable for use in yeast and a selectable marker that is functional in yeast.

Integrative YIp vectors do not replicate autonomously, but integrate, typically in single copy, into the yeast genome at low frequencies and thus replicate as part of the host cell chromosome; these vectors lack an origin of replication that is functional in yeast, although they typically have at least one origin of replication suitable for propagation of the vector in bacterial cells. YEp vectors, in contrast, replicate episomally and autonomously due to presence of the yeast 2 micron plasmid origin (2 µm ori). The YCp yeast centromere plasmid vectors are autonomously replicating vectors containing centromere sequences, CEN, and autonomously replicating sequences, ARS; the ARS sequences are believed to correspond to the natural replication origins of yeast chromosomes. YACs are based on yeast linear plasmids, denoted YLp, containing homologous or heterologous DNA sequences that function as telomeres (TEL) *in vivo*, as well as containing yeast ARS (origins of replication) and CEN (centromeres) segments.

Selectable markers in yeast vectors include a variety of auxotrophic markers, the most common of which are (in *Saccharomyces cerevisiae)* URA3, HIS3, LEU2, TRP1 and LYS2, which complement specific auxotrophic mutations, such as ura3-52, his3-D1, leu2-D1, trp1-D1 and lys2-201. The URA3 and LYS2 yeast genes further permit negative selection based on specific inhibitors, 5-fluoro-orotic acid (FOA) and α-aminoadipic acid (αAA), respectively, that prevent growth of the prototrophic strains but allows growth of the ura3 and lys2 mutants, respectively. Other selectable markers confer resistance to, *e*.*g*., zeocin.

As yet another example, insect cells are often chosen for high efficiency protein expression. Where the host cells are from *Spodoptera frugiperda - e.g.,* Sf9 and Sf21 cell lines, and expresSF^{TM} cells (Protein Sciences Corp., Meriden, CT, USA) - the vector replicative strategy is typically based upon the baculovirus life cycle. Typically, baculovirus transfer vectors are used to replace the wild-type AcMNPV polyhedrin gene with a heterologous gene of interest. Sequences that flank the polyhedrin gene in the wild-type genome are positioned 5' and 3' of the expression cassette on the transfer vectors. Following cotransfection with AcMNPV DNA, a homologous recombination event occurs between these sequences resulting in a recombinant virus carrying the gene of interest and the polyhedrin or p10 promoter. Selection can be based upon visual screening for lacZ fusion activity.

As yet another example, mammalian cells are often chosen for expression of proteins intended as pharmaceutical agents, and are also chosen as host cells for screening of potential agonist and antagonists of a protein or a physiological pathway.

Where mammalian cells are chosen as host cells, vectors intended for autonomous extrachromosomal replication will typically include a viral origin, such as the SV40 origin (for replication in cell lines expressing the large T-antigen, such as COS1 and COS7 cells), the papillomavirus origin, or the EBV origin for long term episomal replication (for use, *e.g.,* in 293-EBNA cells, which constitutively express the EBV EBNA-1 gene product and adenovirus E1A). Vectors intended for integration, and thus replication as part of the mammalian chromosome, can, but need not, include an origin of replication functional in mammalian cells, such as the SV40 origin. Vectors based upon viruses, such as adenovirus, adeno-associated virus, vaccinia virus, and various mammalian retroviruses, will typically replicate according to the viral replicative strategy.

Selectable markers for use in mammalian cells include resistance to neomycin (G418), blasticidin, hygromycin and to zeocin, and selection based upon the purine salvage pathway using HAT medium.

Plant cells can also be used for expression, with the vector replicon typically derived from a plant virus *(e.g.,* cauliflower mosaic virus, CaMV; tobacco mosaic virus, TMV) and selectable markers chosen for suitability in plants.

For propagation of nucleic acids of the present invention that are larger than can readily be accomodated in vectors derived from plasmids or virus, the invention further provides artificial chromosomes - BACs, YACs, PACs and HACs - that comprise ZZAP1 nucleic acids, often genomic nucleic acids.

The BAC system is based on the well-characterized *E. coli* F-factor, a low copy plasmid that exists in a supercoiled circular form in host cells. The structural features of the F-factor allow stable maintenance of individual human DNA clones as well as easy manipulation of the cloned DNA. *See* Shizuya *et al*., *Keio J*. *Med.* 50(1):26-30 (2001); Shizuya *et al*., *Proc*. *Natl*. *Acad*. *Sci*. *USA* 89(18):8794-7 (1992).

YACs are based on yeast linear plasmids, denoted YLp, containing homologous or heterologous DNA sequences that function as telomeres (TEL) *in vivo*, as well as containing yeast ARS (origins of replication) and CEN (centromeres) segments.

HACs are human artifical chromosomes. Kuroiwa *et al., Nature Biotechnol*. 18(10):1086-90 (2000); Henning *et al*., *Proc. Natl*. *Acad*. *Sci*. *USA* 96(2):592-7 (1999); Harrington *et al., Nature Genet.* 15(4):345-55 (1997). In one version, long synthetic arrays of alpha satellite DNA are combined with telomeric DNA and genomic DNA to generate linear microchromosomes that are mitotically and cytogenetically stable in the absence of selection.

PACs are P1-derived artificial chromosomes. Sternberg, *Proc*. *Natl*. *Acad*. *Sci*. *USA* 87(1):103-7 (1990); Sternberg *et al., New Biol.* 2(2):151-62 (1990); Pierce *et al*., *Proc*. *Natl Acad. Sci*. *USA* 89(6):2056-60 (1992).

Vectors of the present invention will also often include elements that permit *in vitro* transcription of RNA from the inserted heterologous nucleic acid. Such vectors typically include a phage promoter, such as that from T7, T3, or SP6, flanking the nucleic acid insert. Often two different such promoters flank the inserted nucleic acid, permitting separate *in vitro* production of both sense and antisense strands.

Expression vectors of the present invention - that is, those vectors that will drive expression of polypeptides from the inserted heterologous nucleic acid - will often include a variety of other genetic elements operatively linked to the protein-encoding heterologous nucleic acid insert, typically genetic elements that drive transcription, such as promoters and enhancer elements, those that facilitate RNA processing, such as transcription termination and/or polyadenylation signals, and those that facilitate translation, such as ribosomal consensus sequences.

For example, vectors for expressing proteins of the present invention in prokaryotic cells, typically *E. coli*, will include a promoter, often a phage promoter, such as phage lambda pL promoter, the trc promoter, a hybrid derived from the trp and lac promoters, the bacteriophage T7 promoter (in *E*. *coli* cells engineered to express the T7 polymerase), or the araBAD operon. Often, such prokaryotic expression vectors will further include transcription terminators, such as the aspA terminator, and elements that facilitate translation, such as a consensus ribosome binding site and translation termination codon, Schomer *al*., *Proc*. *et Natl*. *Acad*. *Sci*. *USA* 83:8506-8510 (1986).

As another example, vectors for expressing proteins of the present invention in yeast cells, typically *S. cerevisiae,* will include a yeast promoter, such as the CYC1 promoter, the GAL1 promoter, ADH1 promoter, or the GPD promoter, and will typically have elements that facilitate transcription termination, such as the transcription termination signals from the CYC1 or ADH1 gene.

As another example, vectors for expressing proteins of the present invention in mammalian cells will include a promoter active in mammalian cells. Such promoters are often drawn from mammalian viruses - such as the enhancer-promoter sequences from the immediate early gene of the human cytomegalovirus (CMV), the enhancer-promoter sequences from the Rous sarcoma virus long terminal repeat (RSV LTR), and the enhancer-promoter from SV40. Often, expression is enhanced by incorporation of polyadenylation sites, such as the late SV40 polyadenylation site and the polyadenylation signal and transcription termination sequences from the bovine growth hormone (BGH) gene, and ribosome binding sites. Furthermore, vectors can include introns, such as intron II of rabbit β-globin gene and the SV40 splice elements.

Vector-drive protein expression can be constitutive or inducible.

Inducible vectors include either naturally inducible promoters, such as the trc promoter, which is regulated by the lac operon, and the pL promoter, which is regulated by tryptophan, the MMTV-LTR promoter, which is inducible by dexamethasone, or can contain synthetic promoters and/or additional elements that confer inducible control on adjacent promoters. Examples of inducible synthetic promoters are the hybrid Plac/ara-1 promoter and the PLtetO-1 promoter. The PltetO-1 promoter takes advantage of the high expression levels from the PL promoter of phage lambda, but replaces the lambda repressor sites with two copies of operator 2 of the Tn10 tetracycline resistance operon, causing this promoter to be tightly repressed by the Tet repressor protein and induced in response to tetracycline (Tc) and Tc derivatives such as anhydrotetracycline.

As another example of inducible elements, hormone response elements, such as the glucocorticoid response element (GRE) and the estrogen response element (ERE), can confer hormone inducibility where vectors are used for expression in cells having the respective hormone receptors. To reduce background levels of expression, elements responsive to ecdysone, an insect hormone, can be used instead, with coexpression of the ecdysone receptor.

Expression vectors can be designed to fuse the expressed polypeptide to small protein tags that facilitate purification and/or visualization.

For example, proteins of the present invention can be expressed with a polyhistidine tag that facilitates purification of the fusion protein by immobilized metal affinity chromatography, for example using NiNTA resin (Qiagen Inc., Valencia, CA, USA) or TALON^{TM} resin (cobalt immobilized affinity chromatography medium, Clontech Labs, Palo Alto, CA, USA). As another example, the fusion protein can include a chitin-binding tag and self-excising intein, permitting chitin-based purification with self-removal of the fused tag (IMPACT^{TM} system, New England Biolabs, Inc., Beverley, MA, USA). Alternatively, the fusion protein can include a calmodulin-binding peptide tag, permitting purification by calmodulin affinity resin (Stratagene, La Jolla, CA, USA), or a specifically excisable fragment of the biotin carboxylase carrier protein, permitting purification of *in vivo* biotinylated protein using an avidin resin and subsequent tag removal (Promega, Madison, WI, USA). As another useful alternative, the proteins of the present invention can be expressed as a fusion to glutathione-S-transferase, the affinity and specificity of binding to glutathione permitting purification using glutathione affinity resins, such as Glutathione-Superflow Resin (Clontech Laboratories, Palo Alto, CA, USA), with subsequent elution with free glutathione.

Other tags include, for example, the Xpress epitope, detectable by anti-Xpress antibody (Invitrogen, Carlsbad, CA, USA), a myc tag, detectable by anti-myc tag antibody, the V5 epitope, detectable by anti-V5 antibody (Invitrogen, Carlsbad, CA, USA), FLAG® epitope, detectable by anti-FLAG® antibody (Stratagene, La Jolla, CA, USA), and the HA epitope.

For secretion of expressed proteins, vectors can include appropriate sequences that encode secretion signals, such as leader peptides. For example, the pSecTag2 vectors (Invitrogen, Carlsbad, CA, USA) are 5.2 kb mammalian expression vectors that carry the secretion signal from the V-J2-C region of the mouse Ig kappa-chain for efficient secretion of recombinant proteins from a variety of mammalian cell lines.

Expression vectors can also be designed to fuse proteins encoded by the heterologous nucleic acid insert to polypeptides larger than purification and/or identification tags. Useful protein fusions include those that permit display of the encoded protein on the surface of a phage or cell, fusions to intrinsically fluorescent proteins, such as those that have a green fluorescent protein (GFP)-like chromophore, fusions to the IgG Fc region, and fusions for use in two hybrid systems.

Vectors for phage display fuse the encoded polypeptide to, *e.g.,* the gene III protein (pIII) or gene VIII protein (pVIII) for display on the surface of filamentous phage, such as M13. *See* Barbas *et al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, San Diego: Academic Press, Inc., 1996; Abelson *et al.* (eds.), Combinatorial Chemistry, Methods in Enzymology vol. 267, Academic Press (May 1996).

Vectors for yeast display, *e.g.* the pYD1 yeast display vector (Invitrogen, Carlsbad, CA, USA), use the α-agglutinin yeast adhesion receptor to display recombinant protein on the surface of *S. cerevisiae.* Vectors for mammalian display, *e.g.,* the pDisplay™ vector (Invitrogen, Carlsbad, CA, USA), target recombinant proteins using an N-terminal cell surface targeting signal and a C-terminal transmembrane anchoring domain of platelet derived growth factor receptor.

A wide variety of vectors now exist that fuse proteins encoded by heterologous nucleic acids to the chromophore of the substrate-independent, intrinsically fluorescent green fluorescent protein from *Aequorea victoria* ("GFP") and its variants. These proteins are intrinsically fluorescent: the GFP-like chromophore is entirely encoded by its amino acid sequence and can fluoresce without requirement for cofactor or substrate.

Structurally, the GFP-like chromophore comprises an 11-stranded β-barrel (β-can) with a central α-helix, the central α-helix having a conjugated π-resonance system that includes two aromatic ring systems and the bridge between them. The π-resonance system is created by autocatalytic cyclization among amino acids; cyclization proceeds through an imidazolinone intermediate, with subsequent dehydrogenation by molecular oxygen at the Cα-Cβ bond of a participating tyrosine.

The GFP-like chromophore can be selected from GFP-like chromophores found in naturally occurring proteins, such as *A. victoria* GFP (GenBank accession number AAA27721), *Renilla reniformis* GFP, FP583 (GenBank accession no. AF168419) (DsRed), FP593 (AF272711), FP483 (AF168420), FP484 (AF168424), FP595 (AF246709), FP486 (AF168421), FP538 (AF168423), and FP506 (AF168422), and need include only so much of the native protein as is needed to retain the chromophore's intrinsic fluorescence. Methods for determining the minimal domain required for fluorescence are known in the art. Li *et al.*,"Deletions of the *Aequorea victoria* Green Fluorescent Protein Define the Minimal Domain Required for Fluorescence," *J. Biol*. *Chem.* 272:28545-28549 (1997).

Alternatively, the GFP-like chromophore can be selected from GFP-like chromophores modified from those found in nature. Typically, such modifications are made to improve recombinant production in heterologous expression systems (with or without change in protein sequence), to alter the excitation and/or emission spectra of the native protein, to facilitate purification, to facilitate or as a consequence of cloning, or are a fortuitous consequence of research investigation.

The methods for engineering such modified GFP-like chromophores and testing them for fluorescence activity, both alone and as part of protein fusions, are well-known in the art. Early results of these efforts are reviewed in Heim *et al*., *Curr*. *Biol.* 6:178-182 (1996), incorporated herein by reference in its entirety; a more recent review, with tabulation of useful mutations, is found in Palm *et al.,* "Spectral Variants of Green Fluorescent Protein," in Green Fluorescent Proteins, Conn (ed.), *Methods Enzymol*. vol. 302, pp. 378 - 394 (1999), incorporated herein by reference in its entirety. A variety of such modified chromophores are now commercially available and can readily be used in the fusion proteins of the present invention.

For example, EGFP ("enhanced GFP"), Cormack *et al., Gene* 173:33-38 (1996); U.S. Pat. Nos. 6,090,919 and 5,804,387, is a red-shifted, human codon-optimized variant of GFP that has been engineered for brighter fluorescence, higher expression in mammalian cells, and for an excitation spectrum optimized for use in flow cytometers. EGFP can usefully contribute a GFP-like chromophore to the fusion proteins of the present invention. A variety of EGFP vectors, both plasmid and viral, are available commercially (Clontech Labs, Palo Alto, CA, USA), including vectors for bacterial expression, vectors for N-terminal protein fusion expression, vectors for expression of C-terminal protein fusions, and for bicistronic expression.

Toward the other end of the emission spectrum, EBFP ("enhanced blue fluorescent protein") and BFP2 contain four amino acid substitutions that shift the emission from green to blue, enhance the brightness of fluorescence and improve solubility of the protein, Heim *et al*., *Curr*. *Biol*. 6:178-182 (1996); Cormack *et al*., *Gene* 173:33-38 (1996). EBFP is optimized for expression in mammalian cells whereas BFP2, which retains the original jellyfish codons, can be expressed in bacteria; as is further discussed below, the host cell of production does not affect the utility of the resulting fusion protein. The GFP-like chromophores from EBFP and BFP2 can usefully be included in the fusion proteins of the present invention, and vectors containing these blue-shifted variants are available from Clontech Labs (Palo Alto, CA, USA).

Analogously, EYFP ("enhanced yellow fluorescent protein"), also available from Clontech Labs, contains four amino acid substitutions, different from EBFP, Ormö *et al., Science* 273:1392-1395 (1996), that shift the emission from green to yellowish-green. Citrine, an improved yellow fluorescent protein mutant, is described in Heikal *et al*., *Proc*. *Natl*. *Acad*. *Sci*. *USA* 97:11996-12001 (2000). ECFP ("enhanced cyan fluorescent protein") (Clontech Labs, Palo Alto, CA, USA) contains six amino acid substitutions, one of which shifts the emission spectrum from green to cyan. Heim *et al*., *Curr*. *Biol.* 6:178-182 (1996); Miyawaki *et al*., *Nature* 388:882-887 (1997). The GFP-like chromophore of each of these GFP variants can usefully be included in the fusion proteins of the present invention.

The GFP-like chromophore can also be drawn from other modified GFPs, including those described in U.S. Pat. Nos. 6,124,128; 6,096,865; 6,090,919; 6,066,476; 6,054,321; 6,027,881; 5,968,750; 5,874,304; 5,804,387; 5,777,079; 5,741,668; and 5,625,048, the disclosures of which are incorporated herein by reference in their entireties. See also Conn (ed.), Green Fluorescent Protein, *Methods in Enzymol*. Vol. 302, pp 378-394 (1999), incorporated herein by reference in its entirety. A variety of such modified chromophores are now commercially available and can readily be used in the fusion proteins of the present invention.

Fusions to the IgG Fc region increase serum half life of protein pharmaceutical products through interaction with the FcRn receptor (also denominated the FcRp receptor and the Brambell receptor, FcRb), further described in international patent application nos. WO 97/43316, WO 97/34631, WO 96/32478, WO 96/18412.

For long-term, high-yield recombinant production of the proteins, protein fusions, and protein fragments of the present invention, stable expression is particularly useful.

Stable expression is readily achieved by integration into the host cell genome of vectors having selectable markers, followed by selection for integrants.

For example, the pUB6/V5-His A, B, and C vectors (Invitrogen, Carlsbad, CA, USA) are designed for high-level stable expression of heterologous proteins in a wide range of mammalian tissue types and cell lines. pUB6/V5-His uses the promoter/enhancer sequence from the human ubiquitin C gene to drive expression of recombinant proteins: expression levels in 293, CHO, and NIH3T3 cells are comparable to levels from the CMV and human EF-1a promoters. The bsd gene permits rapid selection of stably transfected mammalian cells with the potent antibiotic blasticidin.

Replication incompetent retroviral vectors, typically derived from Moloney murine leukemia virus, prove particularly useful for creating stable transfectants having integrated provirus. The highly efficient transduction machinery of retroviruses, coupled with the availability of a variety of packaging cell lines - such as RetroPack™ PT 67, EcoPack2™ -293, AmphoPack-293, GP2-293 cell lines (all available from Clontech Laboratories, Palo Alto, CA, USA) - allow a wide host range to be infected with high efficiency; varying the multiplicity of infection readily adjusts the copy number of the integrated provirus. Retroviral vectors are available with a variety of selectable markers, such as resistance to neomycin, hygromycin, and puromycin, permitting ready selection of stable integrants.

The present invention further includes host cells comprising the vectors of the present invention, either present episomally within the cell or integrated, in whole or in part, into the host cell chromosome.

Among other considerations, some of which are described above, a host cell strain may be chosen for its ability to process the expressed protein in the desired fashion. Such post-translational modifications of the polypeptide include, but are not limited to, acetylation, carboxylation, glycosylation, phosphorylation, lipidation, and acylation, and it is an aspect of the present invention to provide ZZAP1 proteins with such post-translational modifications.

As noted earlier, host cells can be prokaryotic or eukaryotic. Representative examples of appropriate host cells include, but are not limited to, bacterial cells, such as *E*. *coli*, *Caulobacter crescentus*, *Streptomyces* species, and *Salmonella typhimurium*; yeast cells, such as *Saccharomyces cerevisiae*, *Schizosaccharomyces pombe*, *Pichia pastoris*, *Pichia methanolica*; insect cell lines, such as those from *Spodoptera frugiperda - e.g.,* Sf9 and Sf21 cell lines, and expresSF™ cells (Protein Sciences Corp., Meriden, CT, *USA) - Drosophila* S2 cells, and *Trichoplusia ni* High Five® Cells (Invitrogen, Carlsbad, CA, USA); and mammalian cells. Typical mammalian cells include COS1 and COS7 cells, chinese hamster ovary (CHO) cells, NIH 3T3 cells, 293 cells, HEPG2 cells, HeLa cells, L cells, murine ES cell lines (e.g., from strains 129/SV, C57/BL6, DBA-1, 129/SVJ), K562, Jurkat cells, and BW5147. Other mammalian cell lines are well known and readily available from the American Type Culture Collection (ATCC) (Manassas, VA, USA) and the National Institute of General medical Sciences (NIGMS) Human Genetic Cell Repository at the Coriell Cell Repositories (Camden, NJ, USA).

Methods for introducing the vectors and nucleic acids of the present invention into the host cells are well known in the art; the choice of technique will depend primarily upon the specific vector to be introduced and the host cell chosen.

For example, phage lambda vectors will typically be packaged using a packaging extract *(e.g.,* Gigapack® packaging extract, Stratagene, La Jolla, CA, USA), and the packaged virus used to infect *E. coli*. Plasmid vectors will typically be introduced into chemically competent or electrocompetent bacterial cells.

*E*. *coli* cells can be rendered chemically competent by treatment, *e.g.,* with CaCl₂, or a solution of Mg²⁺, Mn²⁺, Ca²⁺, Rb⁺ or K⁺, dimethyl sulfoxide, dithiothreitol, and hexamine cobalt (III), Hanahan, *J. Mol. Biol.* 166(4):557-80 (1983), and vectors introduced by heat shock. A wide variety of chemically competent strains are also available commercially *(e.g.,* Epicurian Coli® XL10-Gold® Ultracompetent Cells (Stratagene, La Jolla, CA, USA); DH5α competent cells (Clontech Laboratories, Palo Alto, CA, USA); TOP10 Chemically Competent E. coli Kit (Invitrogen, Carlsbad, CA, USA)).

Bacterial cells can be rendered electrocompetent - that is, competent to take up exogenous DNA by electroporation - by various pre-pulse treatments; vectors are introduced by electroporation followed by subsequent outgrowth in selected media. An extensive series of protocols is provided online in Electroprotocols (BioRad, Richmond, CA, USA) (http://www.bio-rad.com/LifeScience/pdf/New_Gene_Pulser.pdf).

Vectors can be introduced into yeast cells by spheroplasting, treatment with lithium salts, electroporation, or protoplast fusion.

Spheroplasts are prepared by the action of hydrolytic enzymes - a snail-gut extract, usually denoted Glusulase, or Zymolyase, an enzyme from *Arthrobacter luteus -* to remove portions of the cell wall in the presence of osmotic stabilizers, typically 1 M sorbitol. DNA is added to the spheroplasts, and the mixture is co-precipitated with a solution of polyethylene glycol (PEG) and Ca²⁺. Subsequently, the cells are resuspended in a solution of sorbitol, mixed with molten agar and then layered on the surface of a selective plate containing sorbitol. For lithium-mediated transformation, yeast cells are treated with lithium acetate, which apparently permeabilizes the cell wall, DNA is added and the cells are co-precipitated with PEG. The cells are exposed to a brief heat shock, washed free of PEG and lithium acetate, and subsequently spread on plates containing ordinary selective medium. Increased frequencies of transformation are obtained by using specially-prepared single-stranded carrier DNA and certain organic solvents. Schiestl *et al*., *Curr. Genet*. 16(5-6):339-46 (1989). For electroporation, freshly-grown yeast cultures are typically washed, suspended in an osmotic protectant, such as sorbitol, mixed with DNA, and the cell suspension pulsed in an electroporation device. Subsequently, the cells are spread on the surface of plates containing selective media. Becker et *al., Methods Enzymol*. 194:182-7 (1991). The efficiency of transformation by electroporation can be increased over 100-fold by using PEG, single-stranded carrier DNA and cells that are in late log-phase of growth. Larger constructs, such as YACs, can be introduced by protoplast fusion.

Mammalian and insect cells can be directly infected by packaged viral vectors, or transfected by chemical or electrical means.

For chemical transfection, DNA can be coprecipitated with CaPO₄ or introduced using liposomal and nonliposomal lipid-based agents. Commercial kits are available for CaPO₄ transfection (CalPhos™ Mammalian Transfection Kit, Clontech Laboratories, Palo Alto, CA, USA), and lipid-mediated transfection can be practiced using commercial reagents, such as LIPOFECTAMINE™ 2000, LIPOFECTAMINE™ Reagent, CELLFECTIN® Reagent, and LIPOFECTIN® Reagent (Invitrogen, Carlsbad, CA, USA), DOTAP Liposomal Transfection Reagent, FuGENE 6, X-tremeGENE Q2, DOSPER, (Roche Molecular Biochemicals, Indianapolis, IN USA), Effectene™, PolyFect®, Superfect® (Qiagen, Inc., Valencia, CA, USA). Protocols for electroporating mammalian cells can be found online in Electroprotocols (Bio-Rad, Richmond, CA, USA) (http://www.bio-rad.com/LifeScience/pdf/New_Gene_Pulser.pdf). *See also,* Norton *et al.* (eds.), Gene Transfer Methods: Introducing DNA into Living Cells and Organisms, BioTechniques Books, Eaton Publishing Co. (2000) (ISBN 1-881299-34-1), incorporated herein by reference in its entirety.

Other transfection techniques include transfection by particle embardment. *See*, *e.g.,* Cheng *et al*., *Proc. Natl*. *Acad. Sci*. *USA* 90(10):4455-9 (1993); Yang *et al., Proc. Natl*. *Acad. Sci*. *USA* 87(24):9568-72 (1990).

### PROTEINS

In another aspect, the present invention provides ZZAP1 proteins, various fragments thereof suitable for use as antigens *(e.g.,* for epitope mapping) and for use as immunogens (e.g., for raising antibodies or as vaccines), fusions of ZZAP1 polypeptides and fragments to heterologous polypeptides, and conjugates of the proteins, fragments, and fusions of the present invention to other moieties (*e.g.*, to carrier proteins, to fluorophores).

FIG. 3 presents the predicted amino acid sequences encoded by the ZZAP1 cDNA clone. The amino acid sequence is further presented, respectively, in SEQ ID NO: 3.

Unless otherwise indicated, amino acid sequences of the proteins of the present invention were determined as a predicted translation from a nucleic acid sequence. Accordingly, any amino acid sequence presented herein may contain errors due to errors in the nucleic acid sequence, as described in detail above. Furthermore, single nucleotide polymorphisms (SNPs) occur frequently in eukaryotic genomes - more than 1.4 million SNPs have already identified in the human genome, International Human Genome Sequencing Consortium, *Nature* 409:860 - 921 (2001) - and the sequence determined from one individual of a species may differ from other allelic forms present within the population. Small deletions and insertions can often be found that do not alter the function of the protein.

Accordingly, it is an aspect of the present invention to provide proteins not only identical in sequence to those described with particularity herein, but also to provide isolated proteins at least about 65% identical in sequence to those described with particularity herein, typically at least about 70%, 75%, 80%, 85%, or 90% identical in sequence to those described with particularity herein, usefully at least about 91%, 92%, 93%, 94%, or 95% identical in sequence to those described with particularity herein, usefully at least about 96%, 97%, 98%, or 99% identical in sequence to those described with particularity herein, and, most conservatively, at least about 99.5%, 99.6%, 99.7%, 99.8% and 99.9% identical in sequence to those described with particularity herein. These sequence variants can be naturally occurring or can result from human intervention by way of random or directed mutagenesis.

For purposes herein, percent identity of two amino acid sequences is determined using the procedure of Tatiana *et al.,* "Blast 2 sequences - a new tool for comparing protein and nucleotide sequences", *FEMS Microbiol Lett.* 174:247-250 (1999), which procedure is effectuated by the computer program BLAST 2 SEQUENCES, available online at
http://www.ncbi.nlm.nih.gov/blast/b12seq/b12.html,
To assess percent identity of amino acid sequences, the BLASTP module of BLAST 2 SEQUENCES is used with default values of (i) BLOSUM62 matrix, Henikoff *et al*., *Proc*. *Natl*. *Acad*. *Sci USA* 89(22):10915-9 (1992); (ii) open gap 11 and extension gap 1 penalties; and (iii) gap x_dropoff 50 expect 10 word size 3 filter, and both sequences are entered in their entireties.

As is well known, amino acid substitutions occur frequently among natural allelic variants, with conservative substitutions often occasioning only de *minimis* change in protein function.

Accordingly, it is an aspect of the present invention to provide proteins not only identical in sequence to those described with particularity herein, but also to provide isolated proteins having the sequence of ZZAP1 proteins, or portions thereof, with conservative amino acid substitutions. It is a further aspect to provide isolated proteins having the sequence of ZZAP1 proteins, and portions thereof, with moderately conservative amino acid substitutions. These conservatively-substituted and moderately conservatively-substituted variants can be naturally occurring or can result from human intervention.

Although there are a variety of metrics for calling conservative amino acid substitutions, based primarily on either observed changes among evolutionarily related proteins or on predicted chemical similarity, for purposes herein a conservative replacement is any change having a positive value in the PAM250 log-likelihood matrix reproduced herein below *(see* Gonnet *et al., Science* 256(5062):1443-5 (1992)): 
|  A | R | N | D | C | Q | E | G | H | I | L | K | M | F | P | S | T | W | Y | V
A | 2 | -1 | 0 | 0 | 0 | 0 | 0 | 0 | -1 | -1 | -1 | 0 | -1 | -2 | 0 | 1 | 1 | -4 | -2 | 0
R | -1 | 5 | 0 | 0 | -2 | 2 | 0 | -1 | 1 | -2 | -2 | 3 | -2 | -3 | -1 | 0 | 0 | -2 | -2 | -2
N | 0 | 0 | 4 | 2 | -2 | 1 | 1 | 0 | 1 | -3 | -3 | 1 | -2 | -3 | -1 | 1 | 0 | -4 | -1 | -2
D | 0 | 0 | 2 | 5 | -3 | 1 | 3 | 0 | 0 | -4 | -4 | 0 | -3 | -4 | -1 | 0 | 0 | -5 | -3 | -3
C | 0 | -2 | -2 | -3 | 12 | -2 | -3 | -2 | -1 | -1 | -2 | -3 | -1 | -1 | -3 | 0 | 0 | -1 | 0 | 0
Q | 0 | 2 | 1 | 1 | -2 | 3 | 2 | -1 | 1 | -2 | -2 | 2 | -1 | -3 | 0 | 0 | 0 | -3 | -2 | -2
E | 0 | 0 | 1 | 3 | -3 | 2 | 4 | -1 | 0 | -3 | -3 | 1 | -2 | -4 | 0 | 0 | 0 | -4 | -3 | -2
G | 0 | -1 | 0 | 0 | -2 | -1 | -1 | 7 | -1 | -4 | -4 | -1 | -4 | -5 | -2 | 0 | -1 | -4 | -4 | -3
H | -1 | 1 | 1 | 0 | -1 | 1 | 0 | -1 | 6 | -2 | -2 | 1 | -1 | 0 | -1 | 0 | 0 | -1 | 2 | -2
I | -1 | -2 | -3 | -4 | -1 | -2 | -3 | -4 | -2 | 4 | 3 | -2 | 2 | 1 | -3 | -2 | -1 | -2 | -1 | 3
L | -1 | -2 | -3 | -4 | -2 | -2 | -3 | -4 | -2 | 3 | 4 | -2 | 3 | 2 | -2 | -2 | -1 | -1 | 0 | 2
K | 0 | 3 | 1 | 0 | -3 | 2 | 1 | -1 | 1 | -2 | -2 | 3 | -1 | -3 | -1 | 0 | 0 | -4 | -2 | -2
M | -1 | -2 | -2 | -3 | -1 | -1 | -2 | -4 | -1 | 2 | 3 | -1 | 4 | 2 | -2 | -1 | -1 | -1 | 0 | 2
F | -2 | -3 | -3 | -4 | -1 | -3 | -4 | -5 | 0 | 1 | 2 | -3 | 2 | 7 | -4 | -3 | -2 | 4 | 5 | 0
P | 0 | -1 | -1 | -1 | -3 | 0 | 0 | -2 | -1 | -3 | -2 | -1 | -2 | -4 | 8 | 0 | 0 | -5 | -3 | -2
S | 1 | 0 | 1 | 0 | 0 | 0 | 0 | 0 | 0 | -2 | -2 | 0 | -1 | -3 | 0 | 2 | 2 | -3 | -2 | -1
T | 1 | 0 | 0 | 0 | 0 | 0 | 0 | -1 | 0 | -1 | -1 | 0 | -1 | -2 | 0 | 2 | 2 | -4 | -2 | 0
W | -4 | -2 | -4 | -5 | -1 | -3 | -4 | -4 | -1 | -2 | -1 | -4 | -1 | 4 | -5 | -3 | -4 | 14 | 4 | -3
Y | -2 | -2 | -1 | -3 | 0 | -2 | -3 | -4 | 2 | -1 | 0 | -2 | 0 | 5 | -3 | -2 | -2 | 4 | 8 | -1
V | 0 | -2 | -2 | -3 | 0 | -2 | -2 | -3 | -2 | 3 | 2 | -2 | 2 | 0 | -2 | -1 | 0 | -3 | -1 | 3
 For purposes herein, a "moderately conservative" replacement is any change having a nonnegative value in the PAM250 log-likelihood matrix reproduced herein above.

As is also well known in the art, relatedness of proteins can also be characterized using a functional test, the ability of the encoding nucleic acids to base-pair to one another at defined hybridization stringencies.

It is, therefore, another aspect of the invention to provide isolated proteins not only identical in sequence to those described with particularity herein, but also to provide isolated proteins ("hybridization related proteins") that are encoded by nucleic acids that hybridize under high stringency conditions (as defined herein above) to all or to a portion of various of the isolated nucleic acids of the present invention ("reference nucleic acids"). It is a further aspect of the invention to provide isolated proteins ("hybridization related proteins") that are encoded by nucleic acids that hybridize under moderate stringency conditions (as defined herein above) to all or to a portion of various of the isolated nucleic acids of the present invention ("reference nucleic acids").

The hybridization related proteins can be alternative isoforms, homologues, paralogues, and orthologues of the ZZAP1 protein of the present invention. Particularly useful orthologues are those from other primate species, such as chimpanzee, rhesus macaque monkey, baboon, orangutan, and gorilla, from rodents, such as rats, mice, guinea pigs; from lagomorphs, such as rabbits, and from domestic livestock, such as cow, pig, sheep, horse, and goat.

Relatedness of proteins can also be characterized using a second functional test, the ability of a first protein competitively to inhibit the binding of a second protein to an antibody.

It is, therefore, another aspect of the present invention to provide isolated proteins not only identical in sequence to those described with particularity herein, but also to provide isolated proteins ("cross-reactive proteins") that competitively inhibit the binding of antibodies to all or to a portion of various of the isolated ZZAP1 proteins of the present invention ("reference proteins"). Such competitive inhibition can readily be determined using immunoassays well known in the art.

Among the proteins of the present invention that differ in amino acid sequence from those described with particularity herein - including those that have deletions and insertions causing up to 10% non-identity, those having conservative or moderately conservative substitutions, hybridization related proteins, and cross-reactive proteins - those that substantially retain one or more ZZAP1 activities are particularly useful. As described above, those activities include ATP hydrolysis and protein-protein interactions.

Residues that are tolerant of change while retaining function can be identified by altering the protein at known residues using methods known in the art, such as alanine scanning mutagenesis, Cunningham *et al*., *Science* 244(4908):1081-5 (1989); transposon linker scanning mutagenesis, Chen *et al*., *Gene* 263(1-2):39-48 (2001); combinations of homolog- and alanine-scanning mutagenesis, Jin *et al*., J. Mol. Biol. 226(3):851-65 (1992); combinatorial alanine scanning, Weiss *et al.,* Proc. Natl. Acad. Sci USA 97(16):8950-4 (2000), followed by functional assay. Transposon linker scanning kits are available commercially (New England Biolabs, Beverly, MA, USA, catalog. no. E7-102S; EZ::TN™ In-Frame Linker Insertion Kit, catalogue no. EZI04KN, Epicentre Technologies Corporation, Madison, WI, USA).

As further described below, the isolated proteins of the present invention can readily be used as specific immunogens to raise antibodies that specifically recognize ZZAP1 proteins, their isoforms, homologues, paralogues, and/or orthologues. The antibodies, in turn, can be used, *inter alia,* specifically to assay for the ZZAP1 proteins of the present invention - *e.g.* by ELISA for detection of protein fluid samples, such as serum, by immunohistochemistry or laser scanning cytometry, for detection of protein in tissue samples, or by flow cytometry, for detection of intracellular protein in cell suspensions - for specific antibody-mediated isolation and/or purification of ZZAP1 proteins, as for example by immunoprecipitation, and for use as specific agonists or antagonists of ZZAP1 action.

The isolated proteins of the present invention are also immediately available for use as specific standards in assays used to determine the concentration and/or amount specifically of the ZZAP1 proteins of the present invention. As is well known, ELISA kits for detection and quantitation of protein analytes typically include isolated and purified protein of known concentration for use as a measurement standard (*e.g*., the human interferon-γ OptEIA kit, catalog no. 555142, Pharmingen, San Diego, CA, USA includes human recombinant gamma interferon, baculovirus produced).

The isolated proteins of the present invention are also immediately available for use as specific biomolecule capture probes for surface-enhanced laser desorption ionization (SELDI) detection of protein-protein interactions, WO 98/59362; WO 98/59360; WO 98/59361; and Merchant *et al*., *Electrophoresis* 21(6):1164-77 (2000), the disclosures of which are incorporated herein by reference in their entireties. Analogously, the isolated proteins of the present invention are also immediately available for use as specific biomolecule capture probes on BIACORE surface plasmon resonance probes. . *See* Weinberger *et al*., *Pharmacogenomics* 1(4):395-416 (2000); Malmqvist, *Biochem*. *Soc*. *Trans.* 27(2):335-40 (1999).

The isolated proteins of the present invention are also useful as a therapeutic supplement in patients having a specific deficiency in ZZAP1 production.

In another aspect, the invention also provides fragments of various of the proteins of the present invention. The protein fragments are useful, *inter alia,* as antigenic and immunogenic fragments of ZZAP1.

By "fragments" of a protein is here intended isolated proteins (equally, polypeptides, peptides, oligopeptides), however obtained, that have an amino acid sequence identical to a portion of the reference amino acid sequence, which portion is at least 6 amino acids and less than the entirety of the reference nucleic acid. As so defined, "fragments" need not be obtained by physical fragmentation of the reference protein, although such provenance is not thereby precluded.

Fragments of at least 6 contiguous amino acids are useful in mapping B cell and T cell epitopes of the reference protein. *See, e.g.,* Geysen *et al.,* "Use of peptide synthesis to probe viral antigens for epitopes to a resolution of a single amino acid," *Proc*. *Natl*. *Acad*. *Sci. USA* 81:3998-4002 (1984) and U.S. Pat. Nos. 4,708,871 and 5,595,915, the disclosures of which are incorporated herein by reference in their entireties. Because the fragment need not itself be immunogenic, part of an immunodominant epitope, nor even recognized by native antibody, to be useful in such epitope mapping, all fragments of at least 6 amino acids of the proteins of the present invention have utility in such a study.

Fragments of at least 8 contiguous amino acids, often at least 15 contiguous amino acids, have utility as immunogens for raising antibodies that recognize the proteins of the present invention. *See, e.g.,* Lerner, "Tapping the immunological repertoire to produce antibodies of predetermined specificity," Nature 299:592-596 (1982); Shinnick *et al.,* "Synthetic peptide immunogens as vaccines," *Annu*. *Rev*. *Microbiol*. 37:425-46 (1983); Sutcliffe *et al.,* "Antibodies that react with predetermined sites on proteins," *Science* 219:660-6 (1983), the disclosures of which are incorporated herein by reference in their entireties. As further described in the above-cited references, virtually all 8-mers, conjugated to a carrier, such as a protein, prove immunogenic - that is, prove capable of eliciting antibody for the conjugated peptide; accordingly, all fragments of at least 8 amino acids of the proteins of the present invention have utility as immunogens.

Fragments of at least 8, 9, 10 or 12 contiguous amino acids are also useful as competitive inhibitors of binding of the entire protein, or a portion thereof, to antibodies (as in epitope mapping), and to natural binding partners, such as subunits in a multimeric complex or to receptors or ligands of the subject protein; this competitive inhibition permits identification and separation of molecules that bind specifically to the protein of interest, U.S. Pat. Nos. 5,539,084 and 5,783,674, incorporated herein by reference in their entireties.

The protein, or protein fragment, of the present invention is thus at least 6 amino acids in length, typically at least 8, 9, 10 or 12 amino acids in length, and often at least 15 amino acids in length. Often, the protein or the present invention, or fragment thereof, is at least 20 amino acids in length, even 25 amino acids, 30 amino acids, 35 amino acids, or 50 amino acids or more in length. Of course, larger fragments having at least 75 amino acids, 100 amino acids, or even 150 amino acids are also useful, and at times preferred.

The present invention further provides fusions of each of the proteins and protein fragments of the present invention to heterologous polypeptides.

By fusion is here intended that the protein or protein fragment of the present invention is linearly contiguous to the heterologous polypeptide in a peptide-bonded polymer of amino acids or amino acid analogues; by "heterologous polypeptide" is here intended a polypeptide that does not naturally occur in contiguity with the protein or protein fragment of the present invention. As so defined, the fusion can consist entirely of a plurality of fragments of the ZZAP1 protein in altered arrangement; in such case, any of the ZZAP1 fragments can be considered heterologous to the other ZZAP1 fragments in the fusion protein. More typically, however, the heterologous polypeptide is not drawn from the ZZAP1 protein itself.

The fusion proteins of the present invention will include at least one fragment of the protein of the present invention, which fragment is at least 6, typically at least 8, often at least 15, and usefully at least 16, 17, 18, 19, or 20 amino acids long. The fragment of the protein of the present to be included in the fusion can usefully be at least 25 amino acids long, at least 50 amino acids long, and can be at least 75, 100, or even 150 amino acids long. Fusions that include the entirety of the proteins of the present invention have particular utility.

The heterologous polypeptide included within the fusion protein of the present invention is at least 6 amino acids in length, often at least 8 amino acids in length, and usefully at least 15, 20, and 25 amino acids in length. Fusions that include larger polypeptides, such as the IgG Fc region, and even entire proteins (such as GFP chromophore-containing proteins), have particular utility.

As described above in the description of vectors and expression vectors of the present invention, which discussion is incorporated herein by reference in its entirety, heterologous polypeptides to be included in the fusion proteins of the present invention can usefully include those designed to facilitate purification and/or visualization of recombinantly-expressed proteins. Although purification tags can also be incorporated into fusions that are chemically synthesized, chemical synthesis typically provides sufficient purity that further purification by HPLC suffices; however, visualization tags as above described retain their utility even when the protein is produced by chemical synthesis, and when so included render the fusion proteins of the present invention useful as directly detectable markers of ZZAP1 presence.

As also discussed above, heterologous polypeptides to be included in the fusion proteins of the present invention can usefully include those that facilitate secretion of recombinantly expressed proteins - into the periplasmic space or extracellular milieu for prokaryotic hosts, into the culture medium for eukaryotic cells - through incorporation of secretion signals and/or leader sequences.

Other useful protein fusions of the present invention include those that permit use of the protein of the present invention as bait in a yeast two-hybrid system. See Bartel *et al.* (eds.), The Yeast Two-Hybrid System, Oxford University Press (1997) (ISBN: 0195109384); Zhu *et al.,* Yeast Hybrid Technologies, Eaton Publishing, (2000) (ISBN 1-881299-15-5); Fields *et al., Trends Genet.* 10(8):286-92 (1994); Mendelsohn *et al*., *Curr*. *Opin*. *Biotechnol*. 5(5):482-6 (1994); Luban *et al*., *Curr*. *Opin.* *Biotechnol*. 6(1):59-64 (1995); Allen *et* al., Trends Biochem. Sci. 20(12):511-6 (1995); Drees, *Curr. Opin*. *Chem. Biol.* 3(1):64-70 (1999); Topcu *et al*., *Pharm. Res.* 17(9):1049-55 (2000); Fashena et al., Gene 250(1-2):1-14 (2000), the disclosures of which are incorporated herein by reference in their entireties. Typically, such fusion is to either *E. coli* LexA or yeast GAL4 DNA binding domains. Related bait plasmids are available that express the bait fused to a nuclear localization signal.

Other useful protein fusions include those that permit display of the encoded protein on the surface of a phage or cell, fusions to intrinsically fluorescent proteins, such as green fluorescent protein (GFP), and fusions to the IgG Fc region, as described above, which discussion is incorporated here by reference in its entirety.

The proteins and protein fragments of the present invention can also usefully be fused to protein toxins, such as Pseudomonas exotoxin A, diphtheria toxin, shiga toxin A, anthrax toxin lethal factor, ricin, in order to effect ablation of cells that bind or take up the proteins of the present invention.

The isolated proteins, protein fragments, and protein fusions of the present invention can be composed of natural amino acids linked by native peptide bonds, or can contain any or all of nonnatural amino acid analogues, nonnative bonds, and post-synthetic (post translational) modifications, either throughout the length of the protein or localized to one or more portions thereof.

As is well known in the art, when the isolated protein is used, e.g., for epitope mapping, the range of such nonnatural analogues, nonnative inter-residue bonds, or post-synthesis modifications will be limited to those that permit binding of the peptide to antibodies. When used as an immunogen for the preparation of antibodies in a non-human host, such as a mouse, the range of such nonnatural analogues, nonnative inter-residue bonds, or post-synthesis modifications will be limited to those that do not interfere with the immunogenicity of the protein. When the isolated protein is used as a therapeutic agent, such as a vaccine or for replacement therapy, the range of such changes will be limited to those that do not confer toxicity upon the isolated protein.

Non-natural amino acids can be incorporated during solid phase chemical synthesis or by recombinant techniques, although the former is typically more common.

Solid phase chemical synthesis of peptides is well established in the art. Procedures are described, inter alia, in Chan *et al.* (eds.), Fmoc Solid Phase Peptide Synthesis: A Practical Approach (Practical Approach Series), Oxford Univ. Press (March 2000) (ISBN: 0199637245); Jones, Amino Acid and Peptide Synthesis (Oxford Chemistry Primers, No 7), Oxford Univ. Press (August 1992) (ISBN: 0198556683); and Bodanszky, Principles of Peptide Synthesis (Springer Laboratory), Springer Verlag (December 1993) (ISBN: 0387564314), the disclosures of which are incorporated herein by reference in their entireties.

For example, D-enantiomers of natural amino acids can readily be incorporated during chemical peptide synthesis: peptides assembled from D-amino acids are more resistant to proteolytic attack; incorporation of D-enantiomers can also be used to confer specific three dimensional conformations on the peptide. Other amino acid analogues commonly added during chemical synthesis include ornithine, norleucine, phosphorylated amino acids (typically phosphoserine, phosphothreonine, phosphotyrosine), L-malonyltyrosine, a non-hydrolyzable analog of phosphotyrosine (Kole *et al*., *Biochem*. *Biophys*. *Res*. *Com*. 209:817-821 (1995)), and various halogenated phenylalanine derivatives.

Amino acid analogues having detectable labels are also usefully incorporated during synthesis to provide a labeled polypeptide.

Biotin, for example (indirectly detectable through interaction with avidin, streptavidin, neutravidin, captavidin, or anti-biotin antibody), can be added using biotinoyl-- (9-fluorenylmethoxycarbonyl)-L-lysine (FMOC biocytin) (Molecular Probes, Eugene, OR, USA). (Biotin can also be added enzymatically by incorporation into a fusion protein of a *E. coli* BirA substrate peptide.)

The FMOC and tBOC derivatives of dabcyl-L-lysine (Molecular Probes, Inc., Eugene, OR, USA) can be used to incorporate the dabcyl chromophore at selected sites in the peptide sequence during synthesis. The aminonaphthalene derivative EDANS, the most common fluorophore for pairing with the dabcyl quencher in fluorescence resonance energy transfer (FRET) systems, can be introduced during automated synthesis of peptides by using EDANS--FMOC-L-glutamic acid or the corresponding tBOC derivative (both from Molecular Probes, Inc., Eugene, OR, USA). Tetramethylrhodamine fluorophores can be incorporated during automated FMOC synthesis of peptides using (FMOC)--TMR-L-lysine (Molecular Probes, Inc. Eugene, OR, USA).

Other useful amino acid analogues that can be incorporated during chemical synthesis include aspartic acid, glutamic acid, lysine, and tyrosine analogues having allyl side-chain protection (Applied Biosystems, Inc., Foster City, CA, USA); the allyl side chain permits synthesis of cyclic, branched-chain, sulfonated, glycosylated, and phosphorylated peptides.

A large number of other FMOC-protected non-natural amino acid analogues capable of incorporation during chemical synthesis are available commercially, including, *e.g.*, Fmoc-2-aminobicyclo[2.2.1]heptane-2-carboxylic acid, Fmoc-3-endo-aminobicyclo[2.2.1]heptane-2-endo-carboxylic acid, Fmoc-3-exo-aminobicyclo[2.2.1]heptane-2-exo-carboxylic acid, Fmoc-3-endo-amino-bicyclo[2.2.1]hept-5-ene-2-endo-carboxylic acid, Fmoc-3-exo-amino-bicyclo[2.2.1]hept-5-ene-2-exo-carboxylic acid, Fmoc-cis-2-amino-1-cyclohexanecarboxylic acid, Fmoc-trans-2-amino-1-cyclohexanecarboxylic acid, Fmoc-1-amino-1-cyclopentanecarboxylic acid, Fmoc-cis-2-amino-1-cyclopentanecarboxylic acid, Fmoc-1-amino-1-cyclopropanecarboxylic acid, Fmoc-D-2-amino-4-(ethylthio)butyric acid, Fmoc-L-2-amino-4-(ethylthio)butyric acid, Fmoc-L-buthionine, Fmoc-S-methyl-L-Cysteine, Fmoc-2-aminobenzoic acid (anthranillic acid), Fmoc-3-aminobenzoic acid, Fmoc-4-aminobenzoic acid, Fmoc-2-aminobenzophenone-2'-carboxylic acid, Fmoc-N-(4-aminobenzoyl)-b-alanine, Fmoc-2-amino-4,5-dimethoxybenzoic acid, Fmoc-4-aminohippuric acid, Fmoc-2-amino-3-hydroxybenzoic acid, Fmoc-2-amino-5-hydroxybenzoic acid, Fmoc-3-amino-4-hydroxybenzoic acid, Fmoc-4-amino-3-hydroxybenzoic acid, Fmoc-4-amino-2-hydroxybenzoic acid, Fmoc-5-amino-2-hydroxybenzoic acid, Fmoc-2-amino-3-methoxybenzoic acid, Fmoc-4-amino-3-methoxybenzoic acid, Fmoc-2-amino-3-methylbenzoic acid, Fmoc-2-amino-5-methylbenzoic acid, Fmoc-2-amino-6-methylbenzoic acid, Fmoc-3-amino-2-methylbenzoic acid, Fmoc-3-amino-4-methylbenzoic acid, Fmoc-4-amino-3-methylbenzoic acid, Fmoc-3-amino-2-naphtoic acid, Fmoc-D,L-3-amino-3-phenylpropionic acid, Fmoc-L-Methyldopa, Fmoc-2-amino-4,6-dimethyl-3-pyridinecarboxylic acid, Fmoc-D,L-?-amino-2-thiophenacetic acid, Fmoc-4-(carboxymethyl)piperazine, Fmoc-4-carboxypiperazine, Fmoc-4-(carboxymethyl)homopiperazine, Fmoc-4-phenyl-4-piperidinecarboxylic acid, Fmoc-L-1,2,3,4-tetrahydronorharman-3-carboxylic acid, Fmoc-L-thiazolidine-4-carboxylic acid, all available from The Peptide Laboratory (Richmond, CA, USA).

Non-natural residues can also be added biosynthetically by engineering a suppressor tRNA, typically one that recognizes the UAG stop codon, by chemical aminoacylation with the desired unnatural amino acid and. Conventional site-directed mutagenesis is used to introduce the chosen stop codon UAG at the site of interest in the protein gene. When the acylated suppressor tRNA and the mutant gene are combined in an *in vitro* transcription/translation system, the unnatural amino acid is incorporated in response to the UAG codon to give a protein containing that amino acid at the specified position. Liu *et al*., *Proc*. *Natl Acad*. *Sci*. *USA* 96(9):4780-5 (1999); Wang *et al., Science* 292(5516):498-500 (2001).

The isolated proteins, protein fragments and fusion proteins of the present invention can also include nonnative inter-residue bonds, including bonds that lead to circular and branched forms.

The isolated proteins and protein fragments of the present invention can also include post-translational and post-synthetic modifications, either throughout the length of the protein or localized to one or more portions thereof.

For example, when produced by recombinant expression in eukaryotic cells, the isolated proteins, fragments, and fusion proteins of the present invention will typically include N-linked and/or O-linked glycosylation, the pattern of which will reflect both the availability of glycosylation sites on the protein sequence and the identity of the host cell. Further modification of glycosylation pattern can be performed enzymatically.

As another example, recombinant polypeptides of the invention may also include an initial modified methionine residue, in some cases resulting from host-mediated processes.

When the proteins, protein fragments, and protein fusions of the present invention are produced by chemical synthesis, post-synthetic modification can be performed before deprotection and cleavage from the resin or after deprotection and cleavage. Modification before deprotection and cleavage of the synthesized protein often allows greater control, e.g. by allowing targeting of the modifying moiety to the N-terminus of a resin-bound synthetic peptide.

Useful post-synthetic (and post-translational) modifications include conjugation to detectable labels, such as fluorophores.

A wide variety of amine-reactive and thiol-reactive fluorophore derivatives have been synthesized that react under nondenaturing conditions with N-terminal amino groups and epsilon amino groups of lysine residues, on the one hand, and with free thiol groups of cysteine residues, on the other.

Kits are available commercially that permit conjugation of proteins to a variety of amine-reactive or thiol-reactive fluorophores: Molecular Probes, Inc. (Eugene, OR, USA), *e.g.,* offers kits for conjugating proteins to Alexa Fluor 350, Alexa Fluor 430, Fluorescein-EX, Alexa Fluor 488, Oregon Green 488, Alexa Fluor 532, Alexa Fluor 546, Alexa Fluor 546, Alexa Fluor 568, Alexa Fluor 594, and Texas Red-X.

A wide variety of other amine-reactive and thiol-reactive fluorophores are available commercially (Molecular Probes, Inc., Eugene, OR, USA), including Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA).

The polypeptides of the present invention can also be conjugated to fluorophores, other proteins, and other macromolecules, using bifunctional linking reagents.

Common homobifunctional reagents include, *e.g.,* APG, AEDP, BASED, BMB, BMDB, BMH, BMOE, BM[PEO]3, BM[PEO]4, BS3, BSOCOES, DFDNB, DMA, DMP, DMS, DPDPB, DSG, DSP (Lomant's Reagent), DSS, DST, DTBP, DTME, DTSSP, EGS, HBVS, Sulfo-BSOCOES, Sulfo-DST, Sulfo-EGS (all available from Pierce, Rockford, IL, USA); common heterobifunctional cross-linkers include ABH, AMAS, ANB-NOS, APDP, ASBA, BMPA, BMPH, BMPS, EDC, EMCA, EMCH, EMCS, KMUA, KMUH, GMBS, LC-SMCC, LC-SPDP, MBS, M2C2H, MPBH, MSA, NHS-ASA, PDPH, PMPI, SADP, SAED, SAND, SANPAH, SASD, SATP, SBAP, SFAD, SIA, SIAB, SMCC, SMPB, SMPH, SMPT, SPDP, Sulfo-EMCS, Sulfo-GMBS, Sulfo-HSAB, Sulfo-KMUS, Sulfo-LC-SPDP, Sulfo-MBS, Sulfo-NHS-LC-ASA, Sulfo-SADP, Sulfo-SANPAH, Sulfo-SIAB, Sulfo-SMCC, Sulfo-SMPB, Sulfo-LC-SMPT, SVSB, TFCS (all available Pierce, Rockford, IL, USA).

The proteins, protein fragments, and protein fusions of the present invention can be conjugated, using such cross-linking reagents, to fluorophores that are not amine- or thiol-reactive.

Other labels that usefully can be conjugated to the proteins, protein fragments, and fusion proteins of the present invention include radioactive labels, echosonographic contrast reagents, and MRI contrast agents.

The proteins, protein fragments, and protein fusions of the present invention can also usefully be conjugated using cross-linking agents to carrier proteins, such as KLH, bovine thyroglobulin, and even bovine serum albumin (BSA), to increase immunogenicity for raising anti-ZZAP1 antibodies.

The proteins, protein fragments, and protein fusions of the present invention can also usefully be conjugated to polyethylene glycol (PEG); PEGylation increases the serum half life of proteins administered intravenously for replacement therapy. Delgado *et al*., *Crit. Rev*. *Ther*. *Drug Carrier Syst.* 9(3-4):249-304 (1992); Scott *et al*., *Curr*. *Pharm*. *Des.* 4(6):423-38 (1998); DeSantis *et al*., *Curr*. *Opin*. *Biotechnol*. 10(4):324-30 (1999), incorporated herein by reference in their entireties. PEG monomers can be attached to the protein directly or through a linker, with PEGylation using PEG monomers activated with tresyl chloride (2,2,2-trifluoroethanesulphonyl chloride) permitting direct attachment under mild conditions.

The isolated proteins of the present invention, including fusions thereof, can be produced by recombinant expression, typically using the expression vectors of the present invention as above-described or, if fewer than about 100 amino acids, by chemical synthesis (typically, solid phase synthesis), and, on occasion, by *in vitro* translation.

Production of the isolated proteins of the present invention can optionally be followed by purification. Purification of recombinantly expressed proteins is now well within the skill in the art. *See, e.g.,* Thorner *et al.* (eds.), Applications of Chimeric Genes and Hybrid Proteins, Part A: Gene Expression and Protein Purification (Methods in Enzymology, Volume 326), Academic Press (2000), (ISBN: 0121822273); Harbin (ed.), Cloning, Gene Expression and Protein Purification : Experimental Procedures and Process Rationale, Oxford Univ. Press (2001) (ISBN: 0195132947); Marshak *et al.,* Strategies for Protein Purification and Characterization: A Laboratory Course Manual, Cold Spring Harbor Laboratory Press (1996) (ISBN: 0-87969-385-1); and Roe (ed.), Protein Purification Applications, Oxford University Press (2001), the disclosures of which are incorporated herein by reference in their entireties, and thus need not be detailed here.

Briefly, however, if purification tags have been fused through use of an expression vector that appends such tag, purification can be effected, at least in part, by means appropriate to the tag, such as use of immobilized metal affinity chromatography for polyhistidine tags. Other techniques common in the art include ammonium sulfate fractionation, immunoprecipitation, fast protein liquid chromatography (FPLC), high performance liquid chromatography (HPLC), and preparative gel electrophoresis.

Purification of chemically-synthesized peptides can readily be effected, *e.g.*, by HPLC.

Accordingly, it is an aspect of the present invention to provide the isolated proteins of the present invention in pure or substantially pure form.

A purified protein of the present invention is an isolated protein, as above described, that is present at a concentration of at least 95%, as measured on a weight basis (w/w) with respect to total protein in a composition. Such purities can often be obtained during chemical synthesis without further purification, as, *e.g.,* by HPLC. Purified proteins of the present invention can be present at a concentration (measured on a weight basis with respect to total protein in a composition) of 96%, 97%, 98%, and even 99%. The proteins of the present invention can even be present at levels of 99.5%, 99.6%, and even 99.7%, 99.8%, or even 99.9% following purification, as by HPLC.

Although high levels of purity are particularly useful when the isolated proteins of the present invention are used as therapeutic agents - such as vaccines, or for replacement therapy - the isolated proteins of the present invention are also useful at lower purity. For example, partially purified proteins of the present invention can be used as immunogens to raise antibodies in laboratory animals.

Thus, in another aspect, the present invention provides the isolated proteins of the present invention in substantially purified form. A "substantially purified protein" of the present invention is an isolated protein, as above described, present at a concentration of at least 70%, measured on a weight basis with respect to total protein in a composition. Usefully, the substantially purified protein is present at a concentration, measured on a weight basis with respect to total protein in a composition, of at least 75%, 80%, or even at least 85%, 90%, 91%, 92%, 93%, 94%, 94.5% or even at least 94.9%.

In preferred embodiments, the purified and substantially purified proteins of the present invention are in compositions that lack detectable ampholytes, acrylamide monomers, bis-acrylamide monomers, and polyacrylamide.

The proteins, fragments, and fusions of the present invention can usefully be attached to a substrate. The substrate can porous or solid, planar or non-planar; the bond can be covalent or noncovalent.

For example, the proteins, fragments, and fusions of the present invention can usefully be bound to a porous substrate, commonly a membrane, typically comprising nitrocellulose, polyvinylidene fluoride (PVDF), or cationically derivatized, hydrophilic PVDF; so bound, the proteins, fragments, and fusions of the present invention can be used to detect and quantify antibodies, *e.g.* in serum, that bind specifically to the immobilized protein of the present invention.

As another example, the proteins, fragments, and fusions of the present invention can usefully be bound to a substantially nonporous substrate, such as plastic, to detect and quantify antibodies, *e.g.* in serum, that bind specifically to the immobilized protein of the present invention. Such plastics include polymethylacrylic, polyethylene, polypropylene, polyacrylate, polymethylmethacrylate, polyvinylchloride, polytetrafluoroethylene, polystyrene, polycarbonate, polyacetal, polysulfone, celluloseacetate, cellulosenitrate, nitrocellulose, or mixtures thereof; when the assay is performed in standard microtiter dish, the plastic is typically polystyrene.

The proteins, fragments, and fusions of the present invention can also be attached to a substrate suitable for use as a surface enhanced laser desorption ionization source; so attached, the protein, fragment, or fusion of the present invention is useful for binding and then detecting secondary proteins that bind with sufficient affinity or avidity to the surface-bound protein to indicate biologic interaction therebetween. The proteins, fragments, and fusions of the present invention can also be attached to a substrate suitable for use in surface plasmon resonance detection; so attached, the protein, fragment, or fusion of the present invention is useful for binding and then detecting secondary proteins that bind with sufficient affinity or avidity to the surface-bound protein to indicate biological interaction therebetween.

### ZZAP1 Proteins

In a first series of protein embodiments, the invention provides an isolated ZZAP1 polypeptide having an amino acid sequence encoded by the cDNA in ATCC Deposit No. PTA-3582, or the amino acid sequence in SEQ ID NO: 3, which are full length ZZAP1 proteins. When used as immunogens, the full length proteins of the present invention can be used, *inter alia,* to elicit antibodies that bind to a variety of epitopes of the ZZAP1 protein.

The invention further provides fragments of the above-described polypeptides, particularly fragments having at least 6 amino acids, typically at least 8 amino acids, often at least 15 amino acids, and even the entirety of the sequence given in SEQ ID NO: 3.

The invention further provides fragments of at least 6 amino acids, typically at least 8 amino acids, often at least 15 amino acids, and even the entirety of the sequence given in SEQ ID NO: 5.

The invention further provides fragments of at least 6 amino acids, typically at least 8 amino acids, often at least 15 amino acids, and even the entirety of the sequence given in SEQ ID NO: 7.

As described above, the invention further provides proteins that differ in sequence from those described with particularity in the above-referenced SEQ ID NOs., whether by way of insertion or deletion, by way of conservative or moderately conservative substitutions, as hybridization related proteins, or as cross-hybridizing proteins, with those that substantially retain a ZZAP1 activity particularly useful.

The invention further provides fusions of the proteins and protein fragments herein described to heterologous polypeptides.

### ANTIBODIES AND ANTIBODY-PRODUCING CELLS

In another aspect, the invention provides antibodies, including fragments and derivatives thereof, that bind specifically to ZZAP1 proteins and protein fragments of the present invention or to one or more of the proteins and protein fragments encoded by the isolated ZZAP1 nucleic acids of the present invention. The antibodies of the present invention can be specific for all of linear epitopes, discontinuous epitopes, or conformational epitopes of such proteins or protein fragments, either as present on the protein in its native conformation or, in some cases, as present on the proteins as denatured, as, *e.g.*, by solubilization in SDS.

In other embodiments, the invention provides antibodies, including fragments and derivatives thereof, the binding of which can be competitively inhibited by one or more of the ZZAP1 proteins and protein fragments of the present invention, or by one or more of the proteins and protein fragments encoded by the isolated ZZAP1 nucleic acids of the present invention.

As used herein, the term "antibody" refers to a polypeptide, at least a portion of which is encoded by at least one immunoglobulin gene, which can bind specifically to a first molecular species, and to fragments or derivatives thereof that remain capable of such specific binding.

By "bind specifically" and "specific binding" is here intended the ability of the antibody to bind to a first molecular species in preference to binding to other molecular species with which the antibody and first molecular species are admixed. An antibody is said specifically to "recognize" a first molecular species when it can bind specifically to that first molecular species.

As is well known in the art, the degree to which an antibody can discriminate as among molecular species in a mixture will depend, in part, upon the conformational relatedness of the species in the mixture; typically, the antibodies of the present invention will discriminate over adventitious binding to non-ZZAP1 proteins by at least two-fold, more typically by at least 5-fold, typically by more than 10-fold, 25-fold, 50-fold, 75-fold, and often by more than 100-fold, and on occasion by more than 500-fold or 1000-fold. When used to detect the proteins or protein fragments of the present invention, the antibody of the present invention is sufficiently specific when it can be used to determine the presence of the protein of the present invention in samples derived from human adult liver, brain, fetal liver, lung, placenta, testis, skeletal muscle, heart and bone marrow.

Typically, the affinity or avidity of an antibody (or antibody multimer, as in the case of an IgM pentamer) of the present invention for a protein or protein fragment of the present invention will be at least about 1 x 10⁻⁶ molar (M), typically at least about 5 x 10⁻⁷ M, usefully at least about 1 x 10⁻⁷ M, with affinities and avidities of at least 1 x 10⁻⁸ M, 5 x 10⁻⁹ M, and 1 x 10⁻¹⁰ M proving especially useful.

The antibodies of the present invention can be naturally-occurring forms, such as IgG, IgM, IgD, IgE, and IgA, from any mammalian species.

Human antibodies can, but will infrequently, be drawn directly from human donors or human cells. In such case, antibodies to the proteins of the present invention will typically have resulted from fortuitous immunization, such as autoimmune immunization, with the protein or protein fragments of the present invention. Such antibodies will typically, but will not invariably, be polyclonal.

Human antibodies are more frequently obtained using transgenic animals that express human immunoglobulin genes, which transgenic animals can be affirmatively immunized with the protein immunogen of the present invention. Human Ig-transgenic mice capable of producing human antibodies and methods of producing human antibodies therefrom upon specific immunization are described, *inter alia,* in U.S. Patent Nos. 6,162,963; 6,150,584; 6,114,598; 6,075,181; 5,939,598; 5,877,397; 5,874,299; 5,814,318; 5,789,650; 5,770,429; 5,661,016; 5,633,425; 5,625,126; 5,569,825; 5,545,807; 5,545,806, and 5,591,669, the disclosures of which are incorporated herein by reference in their entireties. Such antibodies are typically monoclonal, and are typically produced using techniques developed for production of murine antibodies.

Human antibodies are particularly useful, and often preferred, when the antibodies of the present invention are to be administered to human beings as *in vivo* diagnostic or therapeutic agents, since recipient immune response to the administered antibody will often be substantially less than that occasioned by administration of an antibody derived from another species, such as mouse.

IgG, IgM, IgD, IgE and IgA antibodies of the present invention are also usefully obtained from other mammalian species, including rodents - typically mouse, but also rat, guinea pig, and hamster - lagomorphs, typically rabbits, and also larger mammals, such as sheep, goats, cows, and horses. In such cases, as with the transgenic human-antibody-producing non-human mammals, fortuitous immunization is not required, and the non-human mammal is typically affirmatively immunized, according to standard immunization protocols, with the protein or protein fragment of the present invention.

As discussed above, virtually all fragments of 8 or more contiguous amino acids of the proteins of the present invention can be used effectively as immunogens when conjugated to a carrier, typically a protein such as bovine thyroglobulin, keyhole limpet hemocyanin, or bovine serum albumin, conveniently using a bifunctional linker such as those described elsewhere above, which discussion is incorporated by reference here.

Immunogenicity can also be conferred by fusion of the proteins and protein fragments of the present invention to other moieties.

For example, peptides of the present invention can be produced by solid phase synthesis on a branched polylysine core matrix; these multiple antigenic peptides (MAPs) provide high purity, increased avidity, accurate chemical definition and improved safety in vaccine development. Tam *et al.,* Proc. Natl. Acad. Sci. USA 85:5409-5413 (1988); Posnett et al., *J. Biol*. *Chem*. 263, 1719-1725 (1988).

Protocols for immunizing non-human mammals are well-established in the art, Harlow *et al*. (eds.), Antibodies: A Laboratory Manual, Cold Spring Harbor Laboratory (1998) (ISBN: 0879693142); Coligan *et al.* (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907), the disclosures of which are incorporated herein by reference, and often include multiple immunizations, either with or without adjuvants such as Freund's complete adjuvant and Freund's incomplete adjuvant.

Antibodies from nonhuman mammals can be polyclonal or monoclonal, with polyclonal antibodies having certain advantages in immunohistochemical detection of the proteins of the present invention and monoclonal antibodies having advantages in identifying and distinguishing particular epitopes of the proteins of the present invention.

Following immunization, the antibodies of the present invention can be produced using any art-accepted technique. Such techniques are well known in the art, Coligan *et al*. (eds.), Current Protocols in Immunology, John Wiley & Sons, Inc. (2001) (ISBN: 0-471-52276-7); Zola, Monoclonal Antibodies : Preparation and Use of Monoclonal Antibodies and Engineered Antibody Derivatives (Basics: From Background to Bench), Springer Verlag (2000) (ISBN: 0387915907); Howard *et al.* (eds.), Basic Methods in Antibody Production and Characterization, CRC Press (2000) (ISBN: 0849394457); Harlow *et al*. (eds.), Antibodies: A Laboratory Manual, Cold Spring Harbor Laboratory (1998) (ISBN: 0879693142); Davis (ed.), Monoclonal Antibody Protocols, Vol. 45, Humana Press (1995) (ISBN: 0896033082); Delves (ed.), Antibody Production: Essential Techniques, John Wiley & Son Ltd (1997) (ISBN: 0471970107); Kenney, Antibody Solution: An Antibody Methods Manual, Chapman & Hall (1997) (ISBN: 0412141914), incorporated herein by reference in their entireties, and thus need not be detailed here.

Briefly, however, such techniques include, *inter alia,* production of monoclonal antibodies by hybridomas and expression of antibodies or fragments or derivatives thereof from host cells engineered to express immunoglobulin genes or fragments thereof. These two methods of production are not mutually exclusive: genes encoding antibodies specific for the proteins or protein fragments of the present invention can be cloned from hybridomas and thereafter expressed in other host cells. Nor need the two necessarily be performed together: *e.g.,* genes encoding antibodies specific for the proteins and protein fragments of the present invention can be cloned directly from B cells known to be specific for the desired protein, as further described in U.S. Pat. No. 5,627,052, the disclosure of which is incorporated herein by reference in its entirety, or from antibody-displaying phage.

Recombinant expression in host cells is particularly useful when fragments or derivatives of the antibodies of the present invention are desired.

Host cells for recombinant antibody production - either whole antibodies, antibody fragments, or antibody derivatives - can be prokaryotic or eukaryotic.

Prokaryotic hosts are particularly useful for producing phage displayed antibodies of the present invention.

The technology of phage-displayed antibodies, in which antibody variable region fragments are fused, for example, to the gene III protein (pIII) or gene VIII protein (pVIII) for display on the surface of filamentous phage, such as M13, is by now well-established, Sidhu, *Curr*. *Opin*. *Biotechnol*. 11(6):610-6 (2000); Griffiths *et al*., *Curr*. *Opin*. *Biotechnol*. 9(1):102-8 (1998); Hoogenboom *et al*., *Immunotechnology*, 4(1):1-20 (1998); Rader *et al*., *Current Opinion in Biotechnology* 8:503-508 (1997); Aujame *et al*., *Human Antibodies* 8:155-168 (1997); Hoogenboom, *Trends in Biotechnol*. 15:62-70 (1997); de Kruif *et al.,* 17:453-455 (1996); Barbas *et al*., *Trends in Biotechnol*. 14:230-234 (1996); Winter *et al*., *Ann*. *Rev*. *Immunol*. 433-455 (1994), and techniques and protocols required to generate, propagate, screen (pan), and use the antibody fragments from such libraries have recently been compiled, Barbas *et al.,* Phage Display: A Laboratory Manual, Cold Spring Harbor Laboratory Press (2001) (ISBN 0-87969-546-3); Kay *et al.* (eds.), Phage Display of Peptides and Proteins: A Laboratory Manual, Academic Press, Inc. (1996); Abelson *et al.* (eds.), Combinatorial Chemistry, Methods in Enzymology vol. 267, Academic Press (May 1996), the disclosures of which are incorporated herein by reference in their entireties.

Typically, phage-displayed antibody fragments are scFv fragments or Fab fragments; when desired, full length antibodies can be produced by cloning the variable regions from the displaying phage into a complete antibody and expressing the full length antibody in a further prokaryotic or a eukaryotic host cell.

Eukaryotic cells are also useful for expression of the antibodies, antibody fragments, and antibody derivatives of the present invention.

For example, antibody fragments of the present invention can be produced in *Pichia pastoris*, Takahashi *et al., Biosci*. *Biotechnol. Biochem*. 64(10):2138-44 (2000); Freyre *et al.,* J. Biotechnol. 76(2-3):157-63 (2000); Fischer *et al., Biotechnol*. *Appl*. *Biochem*. 30 (Pt 2):117-20 (1999); Pennell *et al*., *Res*. *Immunol*. 149(6):599-603 (1998); Eldin *et al*., *J. Immunol*. *Methods.* 201(1):67-75 (1997); and in *Saccharomyces cerevisiae,* Frenken *et al*., *Res*. *Immunol*. 149(6):589-99 (1998); Shusta et al., *Nature Biotechnol*. 16(8):773-7 (1998), the disclosures of which are incorporated herein by reference in their entireties.

Antibodies, including antibody fragments and derivatives, of the present invention can also be produced in insect cells, Li *et al*., *Protein Expr*. *Purif.* 21(1):121-8 (2001); Ailor *et al*., *Biotechnol*. *Bioeng.* 58(2-3):196-203 (1998); Hsu *et al*., *Biotechnol. Prog.* 13(1):96-104 (1997); Edelman *et al., Immunology* 91(1):13-9 (1997); and Nesbit *et al*., *J*. *Immunol*. *Methods.* 151(1-2):201-8 (1992), the disclosures of which are incorporated herein by reference in their entireties.

Antibodies and fragments and derivatives thereof of the present invention can also be produced in plant cells, Giddings *et al*., *Nature Biotechnol*. 18(11):1151-5 (2000); Gavilondo *et al*., *Biotechniques* 29(1):128-38 (2000); Fischer *et al*., *J*. *Biol. Regul*. *Homeost*. *Agents* 14(2):83-92 (2000); Fischer *et al., Biotechnol*. *Appl*. *Biochem*. 30 (Pt 2):113-6 (1999); Fischer *et al*., *Biol*. *Chem.* 380(7-8):825-39 (1999); Russell, *Curr*. *Top.* *Microbiol. Immunol*. 240:119-38 (1999); and Ma *et al*., *Plant Physiol*. 109(2):341-6 (1995), the disclosures of which are incorporated herein by reference in their entireties.

Mammalian cells useful for recombinant expression of antibodies, antibody fragments, and antibody derivatives of the present invention include CHO cells, COS cells, 293 cells, and myeloma cells.

Verma *et al*., *J*. *Immunol. Methods* 216(1-2):165-81 (1998), review and compare bacterial, yeast, insect and mammalian expression systems for expression of antibodies.

Antibodies of the present invention can also be prepared by cell free translation, as further described in Merk *et al.,* J. Biochem. (Tokyo). 125(2):328-33 (1999) and Ryabova *et al*., *Nature Biotechnol*. 15(1):79-84 (1997), and in the milk of transgenic animals, as further described in Pollock *et al*., *J. Immunol*. *Methods* 231(1-2):147-57 (1999), the disclosures of which are incorporated herein by reference in their entireties.

The invention further provides antibody fragments that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful fragments are Fab, Fab', Fv, F(ab)'₂, and single chain Fv (scFv) fragments. Other useful fragments are described in Hudson, *Curr*. *Opin*. *Biotechnol*. 9(4):395-402 (1998).

It is also an aspect of the present invention to provide antibody derivatives that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

Among such useful derivatives are chimeric, primatized, and humanized antibodies; such derivatives are less immunogenic in human beings, and thus more suitable for in vivo administration, than are unmodified antibodies from non-human mammalian species.

Chimeric antibodies typically include heavy and/or light chain variable regions (including both CDR and framework residues) of immunoglobulins of one species, typically mouse, fused to constant regions of another species, typically human. *See, e.g.,* U.S. Pat. No. 5,807,715; Morrison *et al*., *Proc*. *Natl*. *Acad. Sci* USA.81(21):6851-5 (1984); Sharon *et al*., *Nature* 309(5966):364-7 (1984); Takeda *et al*., *Nature* 314 (6010) :452-4 (1985), the disclosures of which are incorporated herein by reference in their entireties. Primatized and humanized antibodies typically include heavy and/or light chain CDRs from a murine antibody grafted into a non-human primate or human antibody V region framework, usually further comprising a human constant region, Riechmann *et al*., *Nature* 332(6162):323-7 (1988); Co *et al*., *Nature* 351(6326):501-2 (1991); U.S. Pat. Nos. 6,054,297; 5,821,337; 5,770,196; 5,766,886; 5,821,123; 5,869,619; 6,180,377; 6,013,256; 5,693,761; and 6,180,370, the disclosures of which are incorporated herein by reference in their entireties.

Other useful antibody derivatives of the invention include heteromeric antibody complexes and antibody fusions, such as diabodies (bispecific antibodies), single-chain diabodies, and intrabodies.

The antibodies of the present invention, including fragments and derivatives thereof, can usefully be labeled. It is, therefore, another aspect of the present invention to provide labeled antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

The choice of label depends, in part, upon the desired use.

For example, when the antibodies of the present invention are used for immunohistochemical staining of tissue samples, the label can usefully be an enzyme that catalyzes production and local deposition of a detectable product.

Enzymes typically conjugated to antibodies to permit their immunohistochemical visualization are well known, and include alkaline phosphatase, β-galactosidase, glucose oxidase, horseradish peroxidase (HRP), and urease. Typical substrates for production and deposition of visually detectable products include o-nitrophenyl-beta-D-galactopyranoside (ONPG); o-phenylenediamine dihydrochloride (OPD); p-nitrophenyl phosphate (PNPP); p-nitrophenyl-beta-D-galactopryanoside (PNPG); 3',3'-diaminobenzidine (DAB); 3-amino-9-ethylcarbazole (AEC); 4-chloro-1-naphthol (CN); 5-bromo-4-chloro-3-indolyl-phosphate (BCIP); ABTS®; BluoGal; iodonitrotetrazolium (INT); nitroblue tetrazolium chloride (NBT); phenazine methosulfate (PMS); phenolphthalein monophosphate (PMP); tetramethyl benzidine (TMB); tetranitroblue tetrazolium (TNBT); X-Gal; X-Gluc; and X-Glucoside.

Other substrates can be used to produce products for local deposition that are luminescent. For example, in the presence of hydrogen peroxide (H₂O₂), horseradish peroxidase (HRP) can catalyze the oxidation of cyclic diacylhydrazides, such as luminol. Immediately following the oxidation, the luminol is in an excited state (intermediate reaction product), which decays to the ground state by emitting light. Strong enhancement of the light emission is produced by enhancers, such as phenolic compounds. Advantages include high sensitivity, high resolution, and rapid detection without radioactivity and requiring only small amounts of antibody. *See, e.g.,* Thorpe *et al*., *Methods Enzymol*. 133:331-53 (1986); Kricka *et al*., *J*. *Immunoassay* 17(1):67-83 (1996); and Lundqvist *et al*., *J*. *Biolumin*. *Chemilumin*. 10(6):353-9 (1995), the disclosures of which are incorporated herein by reference in their entireties. Kits for such enhanced chemiluminescent detection (ECL) are available commercially.

The antibodies can also be labeled using colloidal gold.

As another example, when the antibodies of the present invention are used, *e.g.,* for flow cytometric detection, for scanning laser cytometric detection, or for fluorescent immunoassay, they can usefully be labeled with fluorophores.

There are a wide variety of fluorophore labels that can usefully be attached to the antibodies of the present invention.

For flow cytometric applications, both for extracellular detection and for intracellular detection, common useful fluorophores can be fluorescein isothiocyanate (FITC), allophycocyanin (APC), R-phycoerythrin (PE), peridinin chlorophyll protein (PerCP), Texas Red, Cy3, Cy5, fluorescence resonance energy tandem fluorophores such as PerCP-Cy5.5, PE-Cy5, PE-Cy5.5, PE-Cy7, PE-Texas Red, and APC-Cy7.

Other fluorophores include, *inter alia,* Alexa Fluor® 350, Alexa Fluor® 488, Alexa Fluor® 532, Alexa Fluor® 546, Alexa Fluor® 568, Alexa Fluor® 594, Alexa Fluor® 647 (monoclonal antibody labeling kits available from Molecular Probes, Inc., Eugene, OR, USA), BODIPY dyes, such as BODIPY 493/503, BODIPY FL, BODIPY R6G, BODIPY 530/550, BODIPY TMR, BODIPY 558/568, BODIPY 558/568, BODIPY 564/570, BODIPY 576/589, BODIPY 581/591, BODIPY TR, BODIPY 630/650, BODIPY 650/665, Cascade Blue, Cascade Yellow, Dansyl, lissamine rhodamine B, Marina Blue, Oregon Green 488, Oregon Green 514, Pacific Blue, rhodamine 6G, rhodamine green, rhodamine red, tetramethylrhodamine, Texas Red (available from Molecular Probes, Inc., Eugene, OR, USA), and Cy2, Cy3, Cy3.5, Cy5, Cy5.5, Cy7, all of which are also useful for fluorescently labeling the antibodies of the present invention.

For secondary detection using labeled avidin, streptavidin, captavidin or neutravidin, the antibodies of the present invention can usefully be labeled with biotin.

When the antibodies of the present invention are used, *e*.*g*., for western blotting applications, they can usefully be labeled with radioisotopes, such as ³³P, ³²P, ³⁵S, ³H, and ¹²⁵I.

As another example, when the antibodies of the present invention are used for radioimmunotherapy, the label can usefully be ²²⁸Th, ²²⁷Ac, ²²⁵Ac , ²²³Ra, ²¹³Bi, ²¹²Pb, ²¹²Bi, ²¹¹At, ²⁰³Pb, ¹⁹⁴Os, ¹⁸⁸Re, ¹⁸⁶Re, ¹⁵³Sm, ¹⁴⁹Tb, ¹³¹I, ¹²⁵I, ¹¹¹In, ¹⁰⁵Rh, ^{99m}Tc, ⁹⁷Ru, ⁹⁰Y, ⁹⁰Sr, ⁸⁸Y, ⁷²Se, ⁶⁷Cu, or ⁴⁷Sc.

As another example, when the antibodies of the present invention are to be used for *in vivo* diagnostic use, they can be rendered detectable by conjugation to MRI contrast agents, such as gadolinium diethylenetriaminepentaacetic acid (DTPA), Lauffer *et al., Radiology* 207(2):529-38 (1998), or by radioisotopic labeling

As would be understood, use of the labels described above is not restricted to the application as for which they were mentioned.

The antibodies of the present invention, including fragments and derivatives thereof, can also be conjugated to toxins, in order to target the toxin's ablative action to cells that display and/or express the proteins of the present invention. Commonly, the antibody in such immunotoxins is conjugated to Pseudomonas exotoxin A, diphtheria toxin, shiga toxin A, anthrax toxin lethal factor, or ricin. *See* Hall (ed.), Immunotoxin Methods and Protocols (Methods in Molecular Biology, Vol 166), Humana Press (2000) (ISBN:0896037754); and Frankel *et al.* (eds.), Clinical Applications of Immunotoxins, Springer-Verlag New York, Incorporated (1998) (ISBN:3540640975), the disclosures of which are incorporated herein by reference in their entireties, for review.

The antibodies of the present invention can usefully be attached to a substrate, and it is, therefore, another aspect of the invention to provide antibodies that bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention, attached to a substrate.

Substrates can be porous or nonporous, planar or nonplanar.

For example, the antibodies of the present invention can usefully be conjugated to filtration media, such as NHS-activated Sepharose or CNBr-activated Sepharose for purposes of immunoaffinity chromatography.

For example, the antibodies of the present invention can usefully be attached to paramagnetic microspheres, typically by biotin-streptavidin interaction, which microsphere can then be used for isolation of cells that express or display the proteins of the present invention. As another example, the antibodies of the present invention can usefully be attached to the surface of a microtiter plate for ELISA.

As noted above, the antibodies of the present invention can be produced in prokaryotic and eukaryotic cells. It is, therefore, another aspect of the present invention to provide cells that express the antibodies of the present invention, including hybridoma cells, B cells, plasma cells, and host cells recombinantly modified to express the antibodies of the present invention.

In yet a further aspect, the present invention provides aptamers evolved to bind specifically to one or more of the proteins and protein fragments of the present invention, to one or more of the proteins and protein fragments encoded by the isolated nucleic acids of the present invention, or the binding of which can be competitively inhibited by one or more of the proteins and protein fragments of the present invention or one or more of the proteins and protein fragments encoded by the isolated nucleic acids of the present invention.

### ZZAP1 Antibodies

In a first series of antibody embodiments, the invention provides antibodies, both polyclonal and monoclonal, and fragments and derivatives thereof, that bind specifically to a polypeptide having an amino acid sequence encoded by the cDNA in ATCC Deposit No. PTA-3582, or that have the amino acid sequence in SEQ ID NO: 3, which are full length ZZAP1 proteins.

In a second series of antibody embodiments, the invention provides antibodies, both polyclonal and monoclonal, and fragments and derivatives thereof, that bind specifically to a polypeptide having an amino acid sequence in SEQ ID NO: 5, which is a novel portion of the ZZAP1 protein.

In another series of antibody embodiments, the invention provides antibodies, both polyclonal and monoclonal, and fragments and derivatives thereof, that bind specifically to a polypeptide having an amino acid sequence in SEQ ID NO: 7, which is a novel portion of the ZZAP1 protein.

Such antibodies are useful in *in vitro* immunoassays, such as ELISA, western blot or immunohistochemical assay of disease tissue or cells. Such antibodies are also useful in isolating and purifying ZZAP1 proteins, including related cross-reactive proteins, by immunoprecipitation, immunoaffinity chromatography, or magnetic bead-mediated purification.

In another series of antibody embodiments, the invention provides antibodies, both polyclonal and monoclonal, and fragments and derivatives thereof, the specific binding of which can be competitively inhibited by the isolated proteins and polypeptides of the present invention.

In other embodiments, the invention further provides the above-described antibodies detectably labeled, and in yet other embodiments, provides the above-described antibodies attached to a substrate.

### PHARMACEUTICAL COMPOSITIONS

ZZAP1 has important functions as a molecule with protein domains which may hydrolyze ATP and participate in protein-protein interactions; defects in ZZAP1 expression, activity, distribution, localization, and/or solubility are a cause of human disease, which disease can manifest as a disorder of placenta, fetal liver, brain, adult liver, lung, testis, skeletal muscle, heart or bone marrow function.

Accordingly, pharmaceutical compositions comprising nucleic acids, proteins, and antibodies of the present invention, as well as mimetics, agonists, antagonists, or inhibitors of ZZAP1 activity, can be administered as therapeutics for treatment of ZZAP1 defects.

Thus, in another aspect, the invention provides pharmaceutical compositions comprising the nucleic acids, nucleic acid fragments, proteins, protein fusions, protein fragments, antibodies, antibody derivatives, antibody fragments, mimetics, agonists, antagonists, and inhibitors of the present invention.

Such a composition typically contains from about 0.1 to 90% by weight of a therapeutic agent of the invention formulated in and/or with a pharmaceutically acceptable carrier or excipient.

Pharmaceutical formulation is a well-established art, and is further described in Gennaro (ed.), Remington: The Science and Practice of Pharmacy, 20^{th} ed., Lippincott, Williams & Wilkins (2000) (ISBN: 0683306472); Ansel *et al.,* Pharmaceutical Dosage Forms and Drug Delivery Systems, 7^{th} ed., Lippincott Williams & Wilkins Publishers (1999) (ISBN: 0683305727); and Kibbe (ed.), Handbook of Pharmaceutical Excipients American Pharmaceutical Association, 3^{rd} ed. (2000) (ISBN: 091733096X), the disclosures of which are incorporated herein by reference in their entireties, and thus need not be described in detail herein.

Briefly, however, formulation of the pharmaceutical compositions of the present invention will depend upon the route chosen for administration. The pharmaceutical compositions utilized in this invention can be administered by various routes including both enteral and parenteral routes, including oral, intravenous, intramuscular, subcutaneous, inhalation, topical, sublingual, rectal, intra-arterial, intramedullary, intrathecal, intraventricular, transmucosal, transdermal, intranasal, intraperitoneal, intrapulmonary, and intrauterine.

Oral dosage forms can be formulated as tablets, pills, dragees, capsules, liquids, gels, syrups, slurries, suspensions, and the like, for ingestion by the patient.

Solid formulations of the compositions for oral administration can contain suitable carriers or excipients, such as carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, sodium carboxymethylcellulose, or microcrystalline cellulose; gums including arabic and tragacanth; proteins such as gelatin and collagen; inorganics, such as kaolin, calcium carbonate, dicalcium phosphate, sodium chloride; and other agents such as acacia and alginic acid.

Agents that facilitate disintegration and/or solubilization can be added, such as the cross-linked polyvinyl pyrrolidone, agar, alginic acid, or a salt thereof, such as sodium alginate, microcrystalline cellulose, corn starch, sodium starch glycolate, and alginic acid.

Tablet binders that can be used include acacia, methylcellulose, sodium carboxymethylcellulose, polyvinylpyrrolidone (Povidone™), hydroxypropyl methylcellulose, sucrose, starch and ethylcellulose.

Lubricants that can be used include magnesium stearates, stearic acid, silicone fluid, talc, waxes, oils, and colloidal silica.

Fillers, agents that facilitate disintegration and/or solubilization, tablet binders and lubricants, including the aforementioned, can be used singly or in combination.

Solid oral dosage forms need not be uniform throughout.

For example, dragee cores can be used in conjunction with suitable coatings, such as concentrated sugar solutions, which can also contain gum arabic, talc, polyvinylpyrrolidone, carbopol gel, polyethylene glycol, and/or titanium dioxide, lacquer solutions, and suitable organic solvents or solvent mixtures.

Oral dosage forms of the present invention include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds can be dissolved or suspended in suitable liquids, such as fatty oils, liquid, or liquid polyethylene glycol with or without stabilizers.

Additionally, dyestuffs or pigments can be added to the tablets or dragee coatings for product identification or to characterize the quantity of active compound, *i*.*e*., dosage.

Liquid formulations of the pharmaceutical compositions for oral (enteral) administration are prepared in water or other aqueous vehicles and can contain various suspending agents such as methylcellulose, alginates, tragacanth, pectin, kelgin, carrageenan, acacia, polyvinylpyrrolidone, and polyvinyl alcohol. The liquid formulations can also include solutions, emulsions, syrups and elixirs containing, together with the active compound(s), wetting agents, sweeteners, and coloring and flavoring agents.

The pharmaceutical compositions of the present invention can also be formulated for parenteral administration.

For intravenous injection, water soluble versions of the compounds of the present invention are formulated in, or if provided as a lyophilate, mixed with, a physiologically acceptable fluid vehicle, such as 5% dextrose ("D5"), physiologically buffered saline, 0.9% saline, Hanks' solution, or Ringer's solution.

Intramuscular preparations, *e.g.* a sterile formulation of a suitable soluble salt form of the compounds of the present invention, can be dissolved and administered in a pharmaceutical excipient such as Water-for-Injection, 0.9% saline, or 5% glucose solution. Alternatively, a suitable insoluble form of the compound can be prepared and administered as a suspension in an aqueous base or a pharmaceutically acceptable oil base, such as an ester of a long chain fatty acid (*e.g.*, ethyl oleate), fatty oils such as sesame oil, triglycerides, or liposomes.

Parenteral formulations of the compositions can contain various carriers such as vegetable oils, dimethylacetamide, dimethylformamide, ethyl lactate, ethyl carbonate, isopropyl myristate, ethanol, polyols (glycerol, propylene glycol, liquid polyethylene glycol, and the like).

Aqueous injection suspensions can also contain substances that increase the viscosity of the suspension, such as sodium carboxymethyl cellulose, sorbitol, or dextran. Non-lipid polycationic amino polymers can also be used for delivery.

Optionally, the suspension can also contain suitable stabilizers or agents that increase the solubility of the compounds to allow for the preparation of highly concentrated solutions.

Pharmaceutical compositions of the present invention can also be formulated to permit injectable, long-term, deposition.

The pharmaceutical compositions of the present invention can be administered topically.

A topical semi-solid ointment formulation typically contains a concentration of the active ingredient from about 1 to 20%, *e.g.*, 5 to 10%, in a carrier such as a pharmaceutical cream base. Various formulations for topical use include drops, tinctures, lotions, creams, solutions, and ointments containing the active ingredient and various supports and vehicles. In other transdermal formulations, typically in patch-delivered formulations, the pharmaceutically active compound is formulated with one or more skin penetrants, such as 2-N-methyl-pyrrolidone (NMP) or Azone.

Inhalation formulations can also readily be formulated.

For inhalation, various powder and liquid formulations can be prepared.

The pharmaceutically active compound in the pharmaceutical compositions of the present inention can be provided as the salt of a variety of acids, including but not limited to hydrochloric, sulfuric, acetic, lactic, tartaric, malic, and succinic acid. Salts tend to be more soluble in aqueous or other protonic solvents than are the corresponding free base forms.

After pharmaceutical compositions have been prepared, they are packaged in an appropriate container and labeled for treatment of an indicated condition.

The active compound will be present in an amount effective to achieve the intended purpose. The determination of an effective dose is well within the capability of those skilled in the art.

A "therapeutically effective dose" refers to that amount of active ingredient - for example ZZAP1 protein, fusion protein, or fragments thereof, antibodies specific for ZZAP1, agonists, antagonists or inhibitors of ZZAP1 - which ameliorates the signs or symptoms of the disease or prevents progression thereof; as would be understood in the medical arts, cure, although desired, is not required.

The therapeutically effective dose of the pharmaceutical agents of the present invention can be estimated initially by *in vitro* tests, such as cell culture assays, followed by assay in model animals, usually mice, rats, rabbits, dogs, or pigs. The animal model can also be used to determine an initial useful concentration range and route of administration.

For example, the ED50 (the dose therapeutically effective in 50% of the population) and LD50 (the dose lethal to 50% of the population) can be determined in one or more cell culture of animal model systems. The dose ratio of toxic to therapeutic effects is the therapeutic index, which can be expressed as LD50/ED50. Pharmaceutical compositions that exhibit large therapeutic indices are particularly useful.

The data obtained from cell culture assays and animal studies is used in formulating an initial dosage range for human use, and preferably provides a range of circulating concentrations that includes the ED50 with little or no toxicity. After administration, or between successive administrations, the circulating concentration of active agent varies within this range depending upon pharmacokinetic factors well known in the art, such as the dosage form employed, sensitivity of the patient, and the route of administration.

The exact dosage will be determined by the practitioner, in light of factors specific to the subject requiring treatment. Factors that can be taken into account by the practitioner include the severity of the disease state, general health of the subject, age, weight, gender of the subject, diet, time and frequency of administration, drug combination(s), reaction sensitivities, and tolerance/response to therapy. Long-acting pharmaceutical compositions can be administered every 3 to 4 days, every week, or once every two weeks depending on half-life and clearance rate of the particular formulation.

Normal dosage amounts may vary from 0.1 to 100,000 micrograms, up to a total dose of about 1 g, depending upon the route of administration. Where the therapeutic agent is a protein or antibody of the present invention, the therapeutic protein or antibody agent typically is administered at a daily dosage of 0.01 mg to 30 mg/kg of body weight of the patient (e.g., 1mg/kg to 5 mg/kg). The pharmaceutical formulation can be administered in multiple doses per day, if desired, to achieve the total desired daily dose.

Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

Conventional methods, known to those of ordinary skill in the art of medicine, can be used to administer the pharmaceutical formulation(s) of the present invention to the patient. The pharmaceutical compositions of the present invention can be administered alone, or in combination with other therapeutic agents or interventions.

### THERAPEUTIC METHODS

The present invention further provides methods of treating subjects having defects in ZZAP1 - *e.g.*, in expression, activity, distribution, localization, and/or solubility of ZZAP1 - which can manifest as a disorder of fetal liver, placenta, brain, adult liver, lung, testis, skeletal muscle, heart or bone marrow function. As used herein, "treating" includes all medically-acceptable types of therapeutic intervention, including palliation and prophylaxis (prevention) of disease.

In one embodiment of the therapeutic methods of the present invention, a therapeutically effective amount of a pharmaceutical composition comprising ZZAP1 protein, fusion, fragment or derivative thereof is administered to a subject with a clinically-significant ZZAP1 defect.

Protein compositions are administered, for example, to complement a deficiency in native ZZAP1. In other embodiments, protein compositions are administered as a vaccine to elicit a humoral and/or cellular immune response to ZZAP1. The immune response can be used to modulate activity of ZZAP1 or, depending on the immunogen, to immunize against aberrant or aberrantly expressed forms, such as mutant or inappropriately expressed isoforms. In yet other embodiments, protein fusions having a toxic moiety are administered to ablate cells that aberrantly accumulate ZZAP1.

In another embodiment of the therapeutic methods of the present invention, a therapeutically effective amount of a pharmaceutical composition comprising nucleic acid of the present invention is administered. The nucleic acid can be delivered in a vector that drives expression of ZZAP1 protein, fusion, or fragment thereof, or without such vector.

Nucleic acid compositions that can drive expression of ZZAP1 are administered, for example, to complement a deficiency in native ZZAP1, or as DNA vaccines. Expression vectors derived from virus, replication deficient retroviruses, adenovirus, adeno-associated (AAV) virus, herpes virus, or vaccinia virus can be used - *see, e.g.,* Cid-Arregui (ed.), Viral Vectors: Basic Science and Gene Therapy, Eaton Publishing Co., 2000 (ISBN: 188129935X) - as can plasmids .

Antisense nucleic acid compositions, or vectors that drive expression of ZZAP1 antisense nucleic acids, are administered to downregulate transcription and/or translation of ZZAP1 in circumstances in which excessive production, or production of aberrant protein, is the pathophysiologic basis of disease.

Antisense compositions useful in therapy can have sequence that is complementary to coding or to noncoding regions of the ZZAP1 gene. For example, oligonucleotides derived from the transcription initiation site, *e.g.,* between positions -10 and +10 from the start site, are particularly useful.

Catalytic antisense compositions, such as ribozymes, that are capable of sequence-specific hybridization to ZZAP1 transcripts, are also useful in therapy. *See, e.g.,* Phylactou, *Adv*. *Drug Deliv*. *Rev.* 44(2-3):97-108 (2000); Phylactou *et al., Hum*. *Mol*. *Genet.* 7(10):1649-53 (1998); Rossi, *Ciba Found*. *Symp.* 209:195-204 (1997); and Sigurdsson *et al*., *Trends Biotechnol*. 13(8):286-9 (1995), the disclosures of which are incorporated herein by reference in their entireties.

Other nucleic acids useful in the therapeutic methods of the present invention are those that are capable of triplex helix formation in or near the ZZAP1 genomic locus. Such triplexing oligonucleotides are able to inhibit transcription, Intody *et al.,* Nucleic Acids Res. 28(21):4283-90 (2000); McGuffie *et al*., *Cancer Res.* 60(14):3790-9 (2000), the disclosures of which are incorporated herein by reference, and pharmaceutical compositions comprising such triplex forming oligos (TFOs) are administered in circumstances in which excessive production, or production of aberrant protein, is a pathophysiologic basis of disease.

In another embodiment of the therapeutic methods of the present invention, a therapeutically effective amount of a pharmaceutical composition comprising an antibody (including fragment or derivative thereof) of the present invention is administered. As is well known, antibody compositions are administered, for example, to antagonize activity of ZZAP1, or to target therapeutic agents to sites of ZZAP1 presence and/or accumulation.

In another embodiment of the therapeutic methods of the present invention, a pharmaceutical composition comprising a non-antibody antagonist of ZZAP1 is administered. Antagonists of ZZAP1 can be produced using methods generally known in the art. In particular, purified ZZAP1 can be used to screen libraries of pharmaceutical agents, often combinatorial libraries of small molecules, to identify those that specifically bind and antagonize at least one activity of ZZAP1.

In other embodiments a pharmaceutical composition comprising an agonist of ZZAP1 is administered. Agonists can be identified using methods analogous to those used to identify antagonists.

In still other therapeutic methods of the present invention, pharmaceutical compositions comprising host cells that express ZZAP1, fusions, or fragments thereof can be administered. In such cases, the cells are typically autologous, so as to circumvent xenogeneic or allotypic rejection, and are administered to complement defects in ZZAP1 production or activity.

In other embodiments, pharmaceutical compositions comprising the ZZAP1 proteins, nucleic acids, antibodies, antagonists, and agonists of the present invention can be administered in combination with other appropriate therapeutic agents. Selection of the appropriate agents for use in combination therapy can be made by one of ordinary skill in the art according to conventional pharmaceutical principles. The combination of therapeutic agents or approaches can act additively or synergistically to effect the treatment or prevention of the various disorders described above, providing greater therapeutic efficacy and/or permitting use of the pharmaceutical compositions of the present invention using lower dosages, reducing the potential for adverse side effects.

### TRANSGENIC ANIMALS AND CELLS

In another aspect, the invention provides transgenic cells and non-human organisms comprising ZZAP1 isoform nucleic acids, and transgenic cells and non-human organisms with targeted disruption of the endogenous orthologue of the human ZZAP1 gene.

The cells can be embryonic stem cells or somatic cells. The transgenic non-human organisms can be chimeric, nonchimeric heterozygotes, and nonchimeric homozygotes.

### DIAGNOSTIC METHODS

The nucleic acids of the present invention can be used as nucleic acid probes to assess the levels of ZZAP1 mRNA in disease tissues and cells, and antibodies of the present invention can be used to assess the expression levels of ZZAP1 proteins in disease tissues and cells to diagnose cancer or other diseases (such as osteosclerosis or AIDS).

The following examples are offered for purpose of illustration, not limitation.

### EXAMPLE 1

### Identification and Characterization of cDNAs Encoding ZZAP1 Proteins

Predicating our gene discovery efforts on use of genome-derived single exon probes and hybridization to genome-derived single exon microarrays - an approach that we have previously demonstrated will readily identify novel genes that have proven refractory to mRNA-based identification efforts - we identified an exon in raw human genomic sequence that is particularly expressed in human fetal liver, placenta, brain, liver, lung, testis, skeletal muscle, heart and bone marrow.

Briefly, bioinformatic algorithms were applied to human genomic sequence data to identify putative exons. Each of the predicted exons was amplified from genomic DNA, typically centering the putative coding sequence within a larger amplicon that included flanking noncoding sequence. These genome-derived single exon probes were arrayed on a support and expression of the bioinformatically predicted exons assessed through a series of simultaneous two-color hybridizations to the genome-derived single exon microarrays.

The approach and procedures are further described in detail in Penn *et al.,* "Mining the Human Genome using Microarrays of Open Reading Frames," *Nature Genetics* 26:315-318 (2000); commonly owned and copending U.S. patent application nos. 09/864,761, filed May 23, 2001, 09/774,203, filed January 29, 2001, and 09/632,366, filed August 3, 2000, the disclosures of which are incorporated herein by reference in their entireties.

Using a graphical display particularly designed to facilitate computerized query of the resulting exon-specific expression data, as further described in commonly owned and copending U.S. patent application no. 09/864,761, filed May 23, 2001, 09/774,203, filed January 29, 2001 and 09/632,366, filed August 3, 2000, the disclosures of which are incorporated herein by reference in their entireties, 2 exons were identified that are expressed in all the human tissues tested; subsequent analysis revealed that the 2 exons belong to the same gene.

Table 1 summarizes the microarray expression data obtained using genome-derived single exon probes corresponding to exons 9 and 12. Each probe was completely sequenced on both strands prior to its use on a genome-derived single exon microarray; sequencing confirmed the exact chemical structure of each probe. An added benefit of sequencing is that it placed us in possession of a set of single base-incremented fragments of the sequenced nucleic acid, starting from the sequencing primer's 3' OH. (Since the single exon probes were first obtained by PCR amplification from genomic DNA, we were of course additionally in possession of an even larger set of single base incremented fragments of each of the single exon probes, each fragment corresponding to an extension product from one of the two amplification primers.)

Signals and expression ratios are normalized values measured and calculated as further described in commonly owned and copending U.S. patent application nos. 09/864,761, filed May 23, 2001, 09/774,203, filed January 29, 2001, 09/632,366, filed August 3, 2000, and U.S. provisional patent application no. 60/207,456, filed May 26, 2000, the disclosures of which are incorporated herein by reference in their entireties.

**Table 1**

| Expression Analysis Genome-Derived Single Exon Microarray | | | | |
|---|---|---|---|---|
| | Amplicon 4631 (exon 9) | | Amplicon 13258 (exon 12) | |
| | Signal | Expression ratio | Signal | Expression ratio |
| ADULT LIVER | 1.00 | 2.20 | | |
| BRAIN | 0.69 | 1.67 | | |
| BT474 | 0.56 | -1.38 | | |
| FETAL LIVER | 0.79 | 1.44 | 0.85 | 1.67 |
| HELA | 0.69 | 1.71 | | |
| LUNG | 1.00 | 2.28 | | |
| PLACENTA | 0.82 | 1.26 | 0.4 | 1.09 |

As shown in Table 1, using genome-derived single expn microarray analysis, significant expression of exons 9 and 12 were seen only in adult liver, brain, fetal liver, lung, placenta, and two cell lines, hela and bt474. Adult liver, brain, fetal liver, lung, placenta, and two cell lines, hela and bt474-specific expression was further confirmed by northern blot analysis (see below).

Marathon-Ready™ adult testis cDNA (Clontech Laboratories, Palo Alto, CA, USA) was used as a substrate for standard RACE (rapid amplification of cDNA ends) to obtain cDNA clones that correspond to the ZZAP1 gene, span 1849 bp and appear to contain the entire coding region of the gene to which the exons contribute; for reasons described below, we termed this cDNA ZZAP1. Marathon-Ready™ cDNAs are adaptor-ligated double stranded cDNAs suitable for 3' and 5' RACE. Chenchik *et al., BioTechniques* 21:526-532 (1996); Chenchik et *al*., *CLONTECHniques* X(1):5-8 (January 1995). RACE techniques are described, *inter alia*, in the Marathon-Ready™ cDNA User Manual (Clontech Labs., Palo Alto, CA), Ausubel *et al.* (eds.), Short Protocols in Molecular Biology : A Compendium of Methods from Current Protocols in Molecular Biology, 4^{th} edition (April 1999), John Wiley & Sons (ISBN: 047132938X) and Sambrook *et al.* (eds.), Molecular Cloning: A Laboratory Manual (3rd ed.), Cold Spring Harbor Laboratory Press (2000) (ISBN: 0879695773), the disclosures of which are incorporated herein by reference in their entireties.

The ZZAP1 cDNA was sequenced on both strands using a MegaBace™ sequencer (Amersham Biosciences, Sunnyvale, CA, USA). Sequencing both strands provided us with the exact chemical structure of the cDNA, which is shown in FIG. 3 and further presented in the SEQUENCE LISTING as SEQ ID NO: 1, and placed us in actual physical possession of the entire set of single-base incremented fragments of the sequenced clone, starting at the 5' and 3' termini.

ZZAP1 cDNA was deposited at the American Type Culture Collection on August 2, 2001 and accorded accession number PTA-3582.

As shown in FIG. 3, the ZZAP1 cDNA spans 1849 nucleotides and contains an open reading frame from nucleotide 23 through and including nt 1795 (inclusive of termination codon), predicting a protein of 590 amino acids with a (post-translationally unmodified) molecular weight of 70.3 kD. The clone appears full length, with the reading frame opening starting with a methionine and terminating with a stop codon.

BLAST query of genomic sequence identified four BACS (GenBank accession numbers AC015846.6, AC006440.9, AC007867.3, AC004805.1), as shown in FIG 2, spanning 188.8kb, 170kb, 200kb, and 184.3kb, respectively, that constitute the minimum set of clones encompassing the cDNA sequence. Based upon the known origin of the BACs, the ZZAP1 gene can be mapped to human chromosome 17q23.3

Comparison of the cDNA and genomic sequences identified 16 exons. Exon organization is listed in Table 2.

**Table 2**

| **ZZAP1 Exon Structure** | | | |
|---|---|---|---|
| **Exon no.** | **cDNA range** | **genomic range** | **BAC accession** |
| 1 | 1-145 | 129609-129753 | AC015846.6 |
| 2 | 146-300 | 77685-77531 | |
| 3 | 301-430 | 76914-76785 | |
| 4 | 431-523 | 76067-75975 | |
| 5 | 524-583 | 75222-75163 | |
| 6 | 584-663 | 53431-53352 | |
| 7 | 664-799 | 9260-9125 | |
| 8 | 800-907 | 8467-8574 | AC006440.9 |
| 9 | 908-1090 | 33822-34004 | |
| 10 | 1091-1321 | 84122-84352 | |
| 11 | 1322-1384 | 116978-117040 | AC007867.3 |
| 12 | 1385-1534 | 124487-124636 | |
| 13 | 1535-1624 | 133155-133066 | AC004805.1 |
| 14 | 1625-1726 | 85002-84901 | |
| 15 | 1727-1791 | 81125-81061 | |
| 16 | 1792-1849 | 79911-79854 | |

FIG. 2 schematizes the exon organization of the ZZAP1 clone.

At the top is shown the four bacterial artificial chromosomes (BACs), with GenBank accession numbers, that span the ZZAP1 locus. The genome-derived single-exon probes first used to demonstrate expression from this locus are shown below the BACs and labeled "500".

As shown in FIG. 2, ZZAP1 is comprised of exons 1-16 and encodes a protein of 590 amino acids, with a predicted molecular weight, prior to any post-translational modification, of 70.4kD.

As further discussed in the examples herein, expression of ZZAP1 was assessed using hybridization to genome-derived single exon microarrays and RT-PCR. Microarray analysis of the exons nine and twelve showed expression in adult liver, brain, fetal liver, lung, and placenta as well as cell lines, bt474 and hela. RT-PCR confirmed microarray data, and further provided expression data for testis, skeletal muscle, heart, and bone marrow.

The sequence of the ZZAP1 cDNA was used as a BLAST query into the GenBank nr and dbEst databases. The nr database includes all non-redundant GenBank coding sequence translations, sequences derived from the 3-dimensional structures in the Brookhaven Protein Data Bank (PDB), sequences from SwissProt, sequences from the protein information resource (PIR), and sequences from protein research foundation (PRF). The dbEst (database of expressed sequence tags) includes ESTs, short, single pass read cDNA (mRNA) sequences, and cDNA sequences from differential display experiments and RACE experiments.

BLAST search identified multiple human and mouse ESTs, one from chicken (AL584942.1) and one from cow (AW657062.1) as having sequence closely related to ZZAP1.

Globally, the human ZZAP1 proteins resembles a putative macaque transcript (GenBank accession: BAB21900.1) with 93 % amino acid identity and 93 % amino acid similarity over the entire open reading frame. ZZAP1 also resembles a putative mouse gene (GenBank accession: AAH05723.1) with 90 % amino acid identity and 94 % amino acid similarity over 183 amino acids.

Motif searches using Pfam (http://pfam.wustl.edu), SMART (http://smart.embl- heidelberg.de), and PROSITE pattern and profile databases (http://www.expasy.ch/prosite), identified several known domains shared with other V-ATPase domain containing proteins. The gene was named ZZAP1 because it contains two bZIP transcription factor motifs and is similarity to V-ATPases.

FIG. 1 shows the domain structure of ZZAP1 and the putative macaque homologue.

As schematized in FIG. 1, the newly isolated gene product ZZAP1 shares certain protein domains and an overall structural organization with the putative macaque transcript (GenBank accession number BAB21900). The shared structural features strongly imply that human CFP1 plays a role similar to that of other V-ATPase containing proteins in having protein-protein interaction activity, aberrant expression of some of these proteins leads to cancer, osteosclerosis or AIDS.

Like other proteins with ATP hydrolysis activity, ZZAP1 has two V-ATPase domains occuring at amino acids 2-222 and again at 271-544 (http://pfam.wustl.edu/). These domains bear particular similarity to the membrane-spanning a-subunit of V-ATPases. V-ATPase domains are involved in hydrolyzing ATP to ADP and an inorganic phosphate, and operating as ATP-dependent proton pumps. These domains are present in a number of different proteins and are involved in the acidification of intra- and extra-cellular compartments.

In common with the putative macaque transcript, ZZAP1 also has a leucine zipper motif at amino acids 558-579. Leucine zippers are found in a number of transcription regulatory proteins and consist of a periodic repetition of leucine residues at every seventh turn forming an alpha-helical conformation able to interact with other polypeptides to facilitate dimerization. As a result, it is believed that leucine zipper motifs play a key role in eukaryotic gene regulation through its role in protein-protein interaction activity and protein-DNA binding.

Other signatures of the newly isolated ZZAP1 proteins was identified by searching the PROSITE database (http://www.expasy.ch/tools/scnpsitl.html). These include one N-glycosylation site (512-515), two cAMP- and cGMP-dependent protein kinase phosphorylation sites (93-96 and 574-577), seven Protein kinase C phosphorylation sites (202-204, 446-448, 249-251, 412-414, 272-274, 553-555, 377-379), six Casein kinase II phosphorylation sites (83-86, 117-120, 139-142, 193-196, 272-275, and 465-468), two Tyrosine kinase phosphorylation sites (73-80, 439-445), a single N-myristoylation site (548-553), one amidation site (587-590), and a single leucine zipper pattern (558-579).

Additional signatures of the newly isolated ZZAP1 proteins were identified by searching the PFAM database (http://pfam.wostl.edu/cgi-bin/nph-hmmsearch). These include two bZIP transcription factor motifs (45-75 and 141-157), one Ribosomal protein S21 motif (224-277), one Myosin tail pattern (326-361), a single DNA gyrase/topoisomerase IV, subunit a domain (358-380), and an Hr1 repeat motif (479-562).

Possession of the genomic sequence permitted search for promoter and other control sequences for the ZZAP1 gene. A putative transcriptional control region, inclusive of promoter and downstream elements, was defined as 1 kb around the transcription start site, itself defined as the first nucleotide of the ZZAP1 cDNA clone. The region, drawn from sequence of BAC AC005020.5, has the sequence given in SEQ ID NO: 40, which lists 1000 nucleotides before the transcription start site.

We have thus identified a newly described human gene, ZZAP1, which shares certain protein domains and an overall structural organization with other V-ATPase domain containing proteins. The shared structural features imply that the ZZAP1 protein plays a role similar to other V-ATPase domain containing proteins, as having ATP hydrolysis and protein-protein interaction activity, making the ZZAP1 proteins and nucleic acids clinically useful diagnostic markers and potential therapeutic agents for cancer, osteosclerosis or AIDS.

### EXAMPLE 2

### Preparation and Labeling of Useful Fragments of ZZAP1

Useful fragments of ZZAP1 are produced by PCR, using standard techniques, or solid phase chemical synthesis using an automated nucleic acid synthesizer. Each fragment is sequenced, confirming the exact chemical structure thereof.

The exact chemical structure of preferred fragments is provided in the attached SEQUENCE LISTING, the disclosure of which is incorporated herein by reference in its entirety. The following summary identifies the fragments whose structures are more fully described in the SEQUENCE LISTING:
SEQ ID NO: 1 (nt, full length ZZAP1 cDNA)
SEQ ID NO: 2 (nt, cDNA ORF)
SEQ ID NO: 3 (aa, full length coding sequence of ZZAP1)
SEQ ID NO: 4 (nt, (nt 271 - 298) portion of ZZAP1)
SEQ ID NO: 5 (aa, (aa 84 - 92) CDS entirely within portion of ZZAP1)
SEQ ID NO: 6 (nt, (nt 794 - 860) portion of ZZAP1)
SEQ ID NO: 7 (aa, (aa 258 - 279) CDS entirely within portion of ZZAP1)
SEQ ID NO: 8 - 23 (nt, exons 1 - 16 (from genomic sequence))
SEQ ID NO: 24 - 39 (nt, 500 bp genomic amplicon centered about exons 1 - 16)
SEQ ID NO: 40 (nt, 1000 bp putative promoter)
SEQ ID NOs: 41 - 52 (nt, 17-mers scanning nt 271 - 298)
SEQ ID NOs: 53 - 56 (nt, 25-mers scanning nt 271 - 298)
SEQ ID NOs: 57 - 107 (nt, 17-mers scanning nt 794 - 860)
SEQ ID NOs: 108 - 150 (nt, 25-mers scanning nt 794 - 860)
SEQ ID NO: 151 (nt, forward primer for RT-PCR analysis of ZZAP1 expression)
SEQ ID NO: 152 (nt, reverse primer for RT-PCR analysis of ZZAP1 expression)

Upon confirmation of the exact structure, each of the above-described nucleic acids of confirmed structure is recognized to be immediately useful as a ZZAP1-specific probe.

For use as labeled nucleic acid probes, the above-described ZZAP1 nucleic acids are separately labeled by random priming. As is well known in the art of molecular biology, random priming places the investigator in possession of a near-complete set of labeled fragments of the template of varying length and varying starting nucleotide.

The labeled probes are used to identify the ZZAP1 gene on a Southern blot, and are used to measure expression of ZZAP1 mRNA on a northern blot and by RT-PCR, using standard techniques.

### Example 3

### RT-PCR analysis of expression of human ZZAP1

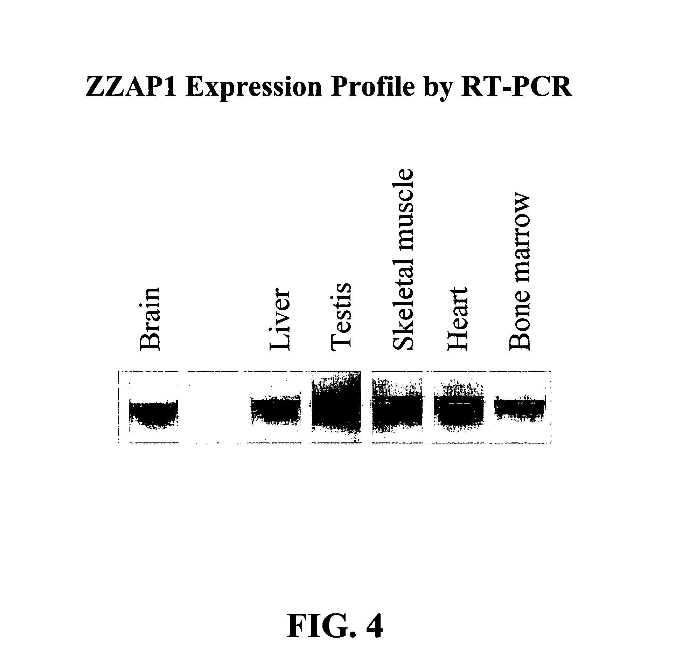

RT-PCR analysis was performed to determine the expression pattern of the human ZZAP1 gene. A forward primer (SEQ ID NO: 151; 5'-GCTTCACAATGCTGTAGCAGAAATG-3') and a reverse primer (SEQ ID NO: 152; 5'-CTGTTCCTGTGACTCCAATTCTT-3') derived from the open reading frame of ZZAP1 were used in standard RT-PCR (Sambrook et al., Molecular cloning: 3rd edition, 2001). Templates for the PCR were obtained from brain, liver, testis, skeletal muscle, heart, and bone marrow, and reactions were carried out according to the following schedule: 94°C, 20 seconds; 65°C 20 seconds; 72°C, 60 seconds, for 35 cycles. PCR products were separated on an agarose gel and visualized with a Typhoon™ fluorimager and Imagequant™ software (Amersham Biosciences, Sunnyvale, CA). RT-PCR product for ZZAP1 was found to be present most highly in liver, testis, skeletal muscle, and heart, and is also present in bone marrow and brain (FIG. 4).

### EXAMPLE 4

### Production of ZZAP1 Protein

The full length ZZAP1 cDNA clone is cloned into the mammalian expression vector pcDNA3.1/HISA (Invitrogen, Carlsbad, CA, USA), transfected into COS7 cells, transfectants selected with G418, and protein expression in transfectants confirmed by detection of the anti-Xpress™ epitope according to manufacturer's instructions. Protein is purified using immobilized metal affinity chromatography and vector-encoded protein sequence is then removed with enterokinase, per manufacturer's instructions, followed by gel filtration and/or HPLC.

Following epitope tag removal, ZZAP1 protein is present at a concentration of at least 70%, measured on a weight basis with respect to total protein (i.e., w/w), and is free of acrylamide monomers, bis acrylamide monomers, polyacrylamide and ampholytes. Further HPLC purification provides ZZAP1 protein at a concentration of at least 95%, measured on a weight basis with respect to total protein (i.e., w/w).

### EXAMPLE 5

### Production of Anti-ZZAP1 Antibody

Purified proteins prepared as in Example 3 are conjugated to carrier proteins and used to prepare murine monoclonal antibodies by standard techniques. Initial screening with the unconjugated purified proteins, followed by competitive inhibition screening using peptide fragments of the ZZAP1, identifies monoclonal antibodies with specificity for ZZAP1.

### EXAMPLE 6

### Use of ZZAP1 Probes and Antibodies for Diagnosis of Diseases

After informed consent is obtained, samples are drawn from tissues or cells and tested for ZZAP1 mRNA levels by standard techniques and tested additionally for ZZAP1 protein levels using anti-ZZAP1 antibodies in a standard ELISA.

### EXAMPLE 7

### Use of ZZAP1 Nucleic Acids, Proteins, and Antibodies in Therapy

Once over-expression of ZZAP1 is detected in patients, ZZAP1 antisense RNA or ZZAP1 specific antibody is introduced into disease cells to reduce the amount of the protein.

Once mutations of ZZAP1 has been detected in patients, normal ZZAP1 is reintroduced into the patient's disease cells by introduction of expression vectors that drive ZZAP1 expression or by introducing ZZAP1 proteins into cells. Antibodies for the mutated forms of ZZAP1 are used to block the function of the abnormal forms of the protein.

### EXAMPLE 8

### ZZAP1 Disease Associations

Diseases that map to the ZZAP1 chromosomal region are shown in Table 3.

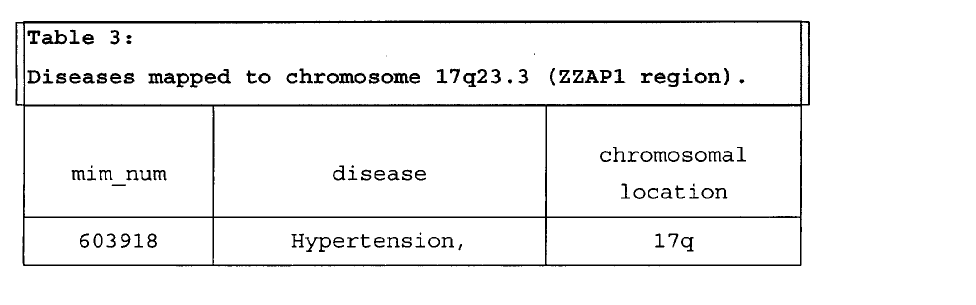

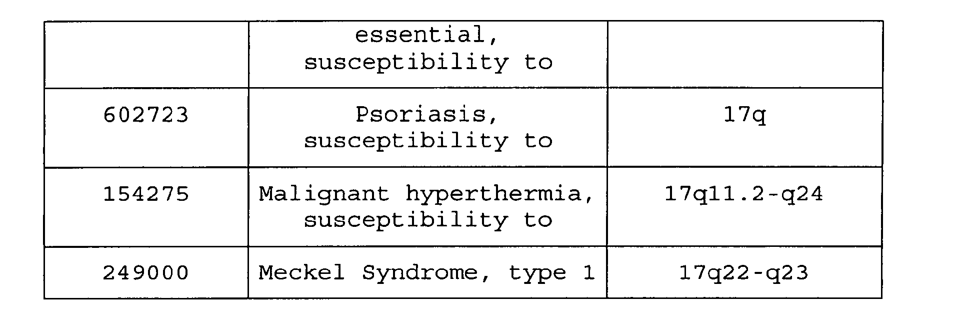

All patents, patent publications, and other published references mentioned herein are hereby incorporated by reference in their entireties as if each had been individually and specifically incorporated by reference herein. While preferred illustrative embodiments of the present invention are described, one skilled in the art will appreciate that the present invention can be practiced by other than the described embodiments, which are presented for purposes of illustration only and not by way of limitation. The present invention is limited only by the claims that follow.

## Claims

1. An isolated nucleic acid that encodes an V-ATPase domains containing protein (ZZAP1), comprising:
(a) a nucleotide sequence selected from the group consisting of:
(i) SEQ ID NO: 1;
(ii) the complement of the sequences set forth in (i);
(iii) the nucleotide sequence of SEQ ID NO: 2;
(iv) a degenerate variant of the sequences set forth in (iii);
(v) the complement of the sequences set forth in (iii) and (iv); and
(vi) the nucleotide sequence of the cDNAs having ATCC accession nos. PTA-3582; or
(b) a nucleotide sequence selected from the group consisting of:
(i) a nucleotide sequence that encodes a polypeptide having the sequence of SEQ ID NO: 3;
(ii) a nucleotide sequence that encodes a polypeptide having the sequence of SEQ ID NO: 3, with conservative amino acid substitutions; and
(iii) the complement of the sequences set forth in (i) and (ii),
wherein said isolated nucleic acid comprising a nucleotide sequence selected from group (b) is no more than about 100 kb in length.

2. The isolated nucleic acid of claim 1 wherein said nucleic acid, or the complement of said nucleic acid, encodes a polypeptide having V-ATPase domains' activity.

3. The isolated nucleic acid of either of claims 1 or 2, wherein said nucleic acid, or the complement of said nucleic acid, is expressed in adult liver, brain, fetal liver, lung, placenta, testis, skeletal muscle, heart, bone marrow, as well as cell lines bt474 and hela.

4. A nucleic acid probe, comprising:
(a) the nucleic acid of claim 1; or
(b) at least 17 contiguous nucleotides of SEQ ID NO: 4 or SEQ ID NO: 6,
wherein said probe according to (b) is no longer than about 100 kb in length.

5. The probe of claim 4, wherein said probe is detectably labeled.

6. The probe of either of claims 4 or 5, attached to a substrate.

7. A microarray, wherein at least one probe of said array is a probe according to claim 4.

8. The isolated nucleic acid molecule of any of claims 1 to 3, wherein said nucleic acid molecule is operably linked to one or more expression control elements.

9. A replicable vector comprising a nucleic acid molecule of any of claims 1 to 3 or 8.

10. A host cell transformed to contain the nucleic acid molecule of any one of claims 1 to 3 or 8 or 9, or the progeny thereof.

11. A method for producing a polypeptide, the method comprising: culturing the host cell of claim 10 under conditions in which the protein encoded by said nucleic acid molecule is expressed.

12. An isolated polypeptide produced by the method of claim 11.

13. An isolated polypeptide, comprising:
(a) an amino acid sequence selected from the group consisting of:
(i) SEQ ID NO: 3; and
(ii) the amino acid sequence of the cDNAs having ATCC accession nos. PTA-3582;
(b) an amino acid sequence having at least 65% amino acid sequence identity to that of (a) (i) or (a) (ii);
(c) an amino acid sequence according to (a)(i) or (a)(ii) in which at least 95% of deviations from the sequence of (a)(i) or (a)(ii) are conservative substitutions; or
(d) a fragment of at least 8 contiguous amino acids of any of (a) - (c) .

14. A fusion protein, said fusion protein comprising a polypeptide of claim 13 fused to a heterologous amino acid sequence.

15. An isolated antibody, or antigen-binding fragment or derivative thereof, the binding of which can be competitively inhibited by a polypeptide of claim 13.

16. A transgenic non-human animal modified to contain the nucleic acid molecule of any one of claims 1 to 3 or 8 or 9.

17. A transgenic non-human animal unable to express the endogenous orthologue of the nucleic acid molecule of any of claims 1 to 3 or 8 or 9.

18. A method of identifying agents that modulate the expression of human ZZAP1, the method comprising:
contacting a cell or tissue sample believed to express human ZZAP1 with a chemical or biological agent, and then
comparing the amount of human ZZAP1 expression in said cell or tissue sample with that of a control,
changes in the amount relative to control identifying an agent that modulates expression of human ZZAP1.

19. A method of identifying agonists and antagonists of human ZZAP1, the method comprising:
contacting a cell or tissue sample believed to express human ZZAP1 with a chemical or biological agent, and then
comparing the activity of human ZZAP1 with that of a control,
increased activity relative to a control identifying an agonist, decreased activity relative to a control identifying an antagonist.

20. A purified agonist of the polypeptide of claim 13.

21. A purified antagonist of the polypeptide of claim 13.

22. A method of identifying a specific binding partner for a polypeptide according to claim 13, the method comprising:
contacting said polypeptide to a potential binding partner; and
determining if the potential binding partner binds to said polypeptide.

23. The method of claim 22, wherein said contacting is performed *in vivo*.

24. A purified binding partner of the polypeptide of claim 13.

25. A method for detecting a target nucleic acid in a sample, said target being a nucleic acid according to any of claims 1 to 3 or 8 or 9, the method comprising:
a) hybridizing the sample with a probe comprising at least 17 contiguous nucleotides of a sequence complementary to said target nucleic acid in said sample under high stringency hybridization conditions, and
b) detecting the presence or absence, and optionally the amount, of said binding.

26. A method of diagnosing a disease caused by mutation in human ZZAP1, comprising:
detecting said mutation in a sample of nucleic acids that derives from a subject suspected to have said disease.

27. A method of diagnosing or monitoring a disease caused by altered expression of human ZZAP1, comprising:
determining the level of expression of human ZZAP1 in a sample of nucleic acids or proteins that derives from a subject suspected to have said disease,
alterations from a normal level of expression providing diagnostic and/or monitoring information.

28. A diagnostic composition comprising the nucleic acid of any of claims 1 to 3, said nucleic acid being detectably labeled.

29. The diagnostic composition of claim 28, wherein said composition is further suitable for *in vivo* administration.

30. A diagnostic composition comprising the polypeptide of claim 13, said polypeptide being detectably labeled.

31. The diagnostic composition of claim 30, wherein said composition is further suitable for *in vivo* administration.

32. A diagnostic composition comprising the antibody, or antigen-binding fragment or derivative thereof, of claim 15

33. The diagnostic composition of claim 32 wherein said antibody or antigen-binding fragment or derivative thereof is detectably labeled.

34. The diagnostic composition of claim 33, wherein said composition is further suitable for *in vivo* administration.

35. A pharmaceutical composition comprising the nucleic acid of any of claims 1 to 3 or 8 or 9 and a pharmaceutically acceptable excipient.

36. A pharmaceutical composition comprising the polypeptide of claim 13 and a pharmaceutically acceptable excipient.

37. A pharmaceutical composition comprising the antibody or antigen-binding fragment or derivative thereof of claim 15 and a pharmaceutically acceptable excipient.

38. A pharmaceutical composition comprising the agonist of claim 20 and a pharmaceutically acceptable excipient.

39. A pharmaceutical composition comprising the antagonist of claim 21 and a pharmaceutically acceptable excipient.

40. Nucleic acid of any of claims 1 to 3 or 8 or 9 for use in therapy.

41. Use of the nucleic acid of any of claims 1 to 3 or 8 or 9 for the manufacture of a medicament for the treatment or prevention of a disorder associated with decreased expression or activity of human ZZAP1.

42. Polypeptide of claim 13 for use in therapy.

43. Use of polypeptide of claim 13 for the manufacture of a medicament for the treatment or prevention of a disorder associated with decreased expression or activity of human ZZAP1.

44. Use of the agonist of claim 20 for the manufacture of a medicament for the treatment or prevention of a disorder associated with decreased expression or activity of human ZZAP1.

45. Antibody or antigen-binding fragment or derivative thereof of claim 15 for use in therapy.

46. Use of the antibody or antigen binding fragment or derivative thereof of claim 15 for the manufacture of a medicament for the treatment or prevention of a disorder associated with increased expression or activity of human ZZAP1.

47. Use of the antagonist of claim 21 for the manufacture of a medicament for the treatment or prevention of a disorder associated with increased expression or activity of human ZZAP1.

48. A method of modulating the expression of a nucleic acid according to any of claims 1 to 3 or 8 or 9 comprising; administering an effective amount of an agent which modulates the expression of a nucleic acid according to claim 1.

49. A method of modulating at least one activity of a polypeptide according to claim 13, the method comprising; administering an effective amount of an agent which modulates at least one activity of a polypeptide according to claim 13.
